# EUROPEAN PATENT SPECIFICATION

(11) **EP 1 278 535 B1**
(45) Date of publication and mention of the grant of the patent: **25.06.2008**
(21) Application number: 01938157.3
(22) Date of filing: 03.05.2001
(51) Int. Cl.: A61K 38/17, A61K 48/00, A61K 39/395, A61K 31/7088, G01N 33/50, C12Q 1/68, C07K 14/47, A61P 37/00, A61P 35/00

(54) **USES OF TGAP7 FOR THE MODULATION OF LEUCOCYTE ACTIVATION**
VERWENDUNGEN VON TGAP7 ZUR MODULATION DER AKTIVIERUNG VON LEUKOZYTEN
UTILISATIONS DE TGAP7 EN VUE DE MODULER L'ACTIVATION DE LEUCOCYTES

(30) Priority: 03.05.2000 EP 00109462
(43) Date of publication of application: 29.01.2003
(73) Proprietor: CellAct Pharma GmbH, 44227 Dortmund (DE)
(72) Inventor: UTKU, Nalàn, 10719 Berlin (DE); SCHLAWINSKY, Mirko, 10709 Berlin (DE)
(74) Representative: Von Renesse, Dorothea
(86) International application number: PCT/EP2001/005010
(87) International publication number: WO 2001/082948

(56) References cited:
- WO-A-99/11782
- Q. GAO ET AL.: "THE E6 ONCOPROTEINS OF HIGH-RISK PAPILLOMAVIRUSES BIND TO A NOVEL PUTATIVE GAP PROTEIN, E6TP1, AND TARGET IT FOR DEGRADATION." MOLECULAR AND CELLULAR BIOLOGY, vol. 19, no. 1, January 1999 (1999-01), pages 733-744, XP002188109 WASHINGTON, DC, US cited in the application
- N. UTKU ET AL.: "PREVENTION OF ACUTE ALLOGRAFT REJECTION BY ANTIBODY TARGETING OF TIRC7, A NOVEL T CELL MEMBRANE PROTEIN." IMMUNITY, vol. 9, October 1998 (1998-10), pages 509-518, XP002188110 CAMBRIDGE, MA, US cited in the application

## Description

### FIELD OF THE INVENTION

The present invention relates to pharmaceutical compositions comprising polynucleotides encoding a TGAP7 protein or a biologically active fragment thereof.
Furthermore, the present invention relates to pharmaceutical compositions comprising a nucleic acid molecule of at least 15 nucleotides in length hybridizing specifically with a polynucleotide described herein or with a complementary strand thereof. In addition, the present invention pertains to pharmaceutical compositions comprising vectors containing polynucleotides encoding a TGAP7 protein, comprising (host-)cells which comprise said polynucleotide(s) or said vectors, comprising a TGAP7 protein or biologically active fragments thereof, comprising an antibody which specifically recognizes a TGAP7 protein or a fragment thereof, or comprising an antisense construct capable of inhibiting the expression of a polynucleotide encoding a TGAP7 protein. Additonally, this invention provides diagnostic compositions and methods of diagnosing biologically conditions. Also, the invention relates to methods for identifying binding partners to a TGAP7 protein and to methods for identifying leucocyte activating or co-stimulating compounds or for identifying inhibitors of leucocyte activation and stimulation. Finally, the present invention relates to the use of the before described polynucleotide(s), vector(s), protein(s), antisense construct(s) for the preparation of compositions for diagnosing or the treatment of acute and chronic diseases, involving T-cell activation and Th1 and Th2 immune response, for the treatment of acute and chronic rejection of allo-and xeno-organ transplants and bone marrow transplantation, for the treatment of rheumatoid arthritis, lupus erythramatodes, multiple sklerosis, encephalitis, vasculitis, diabetes mellitus, pancreatitis, gastritis, thyroiditis, for the treatment of (maligne) disorders of T, B or NK cells, for the treatment of asthma, lepramatosis, Helicobacter pylori associated gastritis or for the treatment of skin tumors, adrenal tumors or lung tumors, wound healing, growth disorders, inflammatory and/or infectious diseases.

### BACKGROUND OF THE INVENTION

T-cell activation is accompanied with sequential changes in the expression of various genes over several days and involves multiple signaling pathways [1]. Stimulation of T-cells is initiated by the interaction of antigen-specific T-cell receptors (TCR) with MHC bound antigenic peptides presented on the surface of antigen presenting cells (APC), but full proliferative T-cell response requires additional costimulatory signals which are provided by the interaction of proteins expressed on the surface of T-cells and APC [2,3,4,5]. In addition, a number of cytokines as well as other proteins are known to augment T-cell activation, although many of them appear not to be essential for the basic proliferative T-cell response [3,6]. Moreover, a growing body of evidence indicates that the microtubule cytoskeleton of lymphocytes plays a major role in T-cell activation. Stimulation of T-cells was demonstrated to result in molecular rearrangement in the actin cytoskeleton leading to re-localization and concentration of signaling molecules in restricted areas of the cell membrane close to the bound APC [7,8,9].

Although considerable information on T-cell activation has been gathered in recent years, the complex molecular mechanisms of stimulation and signaling pathways are not completely understood. Since T-cell activation provides the central event in various types of inflammation as well as in autoimmune disease and graft rejection, knowledge about the distinct steps and molecules involved in the stimulation process is of considerable biomedical importance, as they might provide targets for therapeutic modulation of the immune response. Therapeutic prevention of T-cell activation in organ transplantation and autoimmune diseases presently relies on panimmunosuppressive drugs interfering with downstream intracellullar events.

Alloreactive CD4 or CD8 cells or specific alloantibodies are capable of mediating, inter alia, allograft rejection. The following immune mechanisms cause graft rejection by different mechanisms:
(a) alloactive T-cells can recruit and activate macrophages, initiating graft injury by "delayed-type" hypersensitivity response;
(b) alloactive cytotoxic T-cells are capable of directly lysing graft endothelial and parenchymal cells; and
(c) alloantibodies bind to endothelium, activate the complement system, and injure thereby graft blood vessels.

The various forms of (allograft) rejection imply a temporal sequence of events including hyperacute, acute vascular and acute cellular as well as chronic rejection.

Hyperacute rejection plays an important role in xenotransplantation and is due to natural antibodies (Ref: Milford, E., Utku A, N. Guidelines for use of immunogenetic tests in organ transplanation, Manual of Clinical Laboratory Immunology, ASM Press 1997). Hyperacute rejection is mediated by preexisting antibodies that bind to endothelium and activate complement and is characterized by rapid thrombotic occlusion of the graft vasculature. In more recent clinical experience, hyperacute rejection of allografts is usually mediated by antibodies directed against protein alloantigens, such as foreign MHC molecules, or against less well described alloantigens expressed on vascular endothelial cells. Such antibodies generally arise as a result of prior exposure to alloantigens through blood transfusion, prior transplantation, or multiple pregnancies (loc. cit.). These antibodies are often of the IgG type. By testing recipients for the presence of such reactive antibodies with the cells of potential donors, hyperacute rejection has been virtually eliminated from clinical allo-transplantation but remains a major problem in xenotransplantation.

Acute vascular rejection is mediated by IgG antibodies produced by B-cells against endothelial alloantigens and involves activation of complement. T-cells contribute to vascular injury by responding to alloantigens present on endothelial cells, leading to direct cell lysis of these cells, or the production of cytokines that recruit and activate inflammatory cells.

Acute cellular rejection is characterized by necrosis of parenchymal cells and is usually accompanied by lymphocyte and macrophage infiltrates. These infiltrating leucocytes are responsible for the lysis of the graft. Several different effector mechanisms may be involved in acute cellular rejection including CTL-mediated lysis, activated-macrophage-mediated lysis (as delayed type hypersensibility, DTH), and natural killer cells mediated lysis.

The identification of both antibody and lymphocytes as important effector mechanism in acute graft rejection suggests that this process is similar to normal antiviral immune responses. The basis of similarity probably arises from the fact that the foreign class I MHC molecules present in the graft are recognized as if they were self MHC molecules associated with endogenously synthesized foreign peptides.

Chronic rejection is characterized by fibrosis with loss of normal organ structures. The fibrosis may represent wound healing following the cellular necrosis of acute rejection or a form of DTH in which activated macrophages secrete mesenchymal cell growth factors, or alternatively chronic rejection is a response to chronic ischemia caused by injury of blood vessels. Vascular occlusion is due to proliferation of intimal smooth muscle cells, called accelerated or graft arteriosclerosis. This features of chronic inflammation is characterized by fibrosis in autoimmune diseases such as lupus erythramatodes, sklerodermia and panarteriitis nodosa. Chronic inflammatory immune response involves all parts of cellular and humoral immune system (T-, B-, NK- cells, monocytes) and is the response to the extensive production of autoantibodies and creation of immune complexes against different cellular components. This leads to multiple organ failure caused by significant tissues damage such as myositis, polyneuropathia, heart disease, vasculitis, etc.

Considering, inter alia, the above described temporal sequence events in allograft and xenograft rejection, it is desired to specifically modulate lymphocyte cell responses, i.e. to modulate T-, B-, NK-cells and/or monocyte responses during immunological processes. Furthermore, it is desired to specifically modulate immunologica processes like, inter alia, autoimmunological events.

Specific modulation of the immune response remains, therefore, a longstanding goal in immunological research.

### SUMMARY OF THE INVENTION

The present invention relates to pharmaceutical compositions comprising polynucleotides encoding an immune response modulating protein TGAP7. Furthermore, the present invention relates to pharmaceutical compositions comprising peptides and polypeptides derived therefrom as well as to pharmaceutical compositions comprising antibodies capable of inhibiting leucocyte stimulation through the immune response modulating protein TGAP7. More particularly, the present invention relates to applications in the medical field that directly arise from the polynucleotides, protein, peptides, (poly)peptides, antisense constructs and antibodies described in this invention. Additionally, the present invention relates to a novel method for testing activators and inhibitors of leucocyte proliferation, i.e. of leucocyte activation and/or stimulation. The pharmaceutical compositions, methods and uses of the invention are useful therapeutically and/or diagnostically in situations where it is desirable to modulate (antigen-specific) immune responses, e.g., inducing and maintain (antigen-specific) T-cell or B-cell non/unresponsiveness, wherein said non/unresponsiveness comprises the selective inhibition of immune cell subsets which are able of creating a response to specific antigen(s), inter alia, antigen(s) in transplanted tissue. The pharmaceutical compositions, methods and uses of the invention are furthermore useful to restore (antigen-specific) B or T-cell responsiveness. For example, it may be necessary to induce or maintain "selective immune" unresponsiveness in a subject who has received an organ or bone marrow transplant to prevent graft rejection by inhibiting stimulation through the TGAP7 protein in cells of the immune system such as T-cells, B-cells, NK-cells, monocytes and/or macrophages. In addition, T-cell unresponsiveness can be maintained by blocking TGAP7 stimulation in a subject who has an autoimmune disease to alleviate symptoms of the autoimmune disease. In these cases, a TGAP7 inhibitory agent is administered to the subject in an amount and over a period of time sufficient to maintain T-cell unresponsiveness. Alternatively, T-cell unresponsiveness can be reversed in a subject bearing a tumor to stimulate a tumor specific NK- and T-cell response or in a subject receiving a vaccine to enhance the efficacy of the vaccine. For example, it might be useful to induce or maintain the status of activation of the immune cells through vaccination with TGAP7 peptides in a subject who developed a tumor to orchestrate the enhancement of immune response in T-, B-, NK-cells and/or monocytes.

### DETAILED DESCRIPTION OF THE PRESENT INVENTION

In view of the need of therapeutic means for the diagnosis and treatment of diseases related to immune responses of the human body, the technical problem of the invention is to provide means and methods for the modulation of immune cell responses which are particularly useful in organ transplantation and autoimmune diseases.
The solution to this technical problem is achieved by providing the embodiments characterized in the claims, namely novel pharmaceutical compositions comprising an immune response modulating protein encoded by a immune response cDNA designated "TGAP7" which exhibits a central role in leucocyte activation and growth, wherein said leucocyte activation refers to the activation of T-, B-, NK-cells and/or monocytes. TGAP7 mRNA is transiently upregulated in the early phase of T-cell activation.
In a first set of experiments, the TGAP7 protein encoding cDNA has been cloned and characterized; see Example 1. Furthermore, the expression pattern of TGAP7 was investigated after allo-stimulation of human leucocytes at time points 0, 1, 24 and 72 h and results obtained with alloactivated T-cells revealed an upregulation of TGAP7 only after 24 h after immune activation of the TGAP7 gene. It is thus an excellent marker for diagnosis of the status of immune response in a subject.

An independent set of experiments performed in accordance with the present invention, surprisingly revealed that modulation of the TGAP7 expression with specific TGAP7 antisense polynucleotides efficiently leads to a significant down regulation of lymphocyte activation in response to allo- and mitogens. The latter described results obtained in accordance with the present invention provide evidence for an essential role of TGAP7 in the early events of leucocyte activation.
Thus, targeting of TGAP7 protein and its encoding gene provides a novel therapeutic approach for modulation of the immune response.
Accordingly, the invention relates to a pharmaceutical composition comprising a polynucleotide encoding a TGAP7 protein or a biologically active fragment thereof comprising a nucleic acid sequence selected from the group consisting of:
(i) DNA sequences comprising a nucleotide sequence encoding the amino acid sequence depicted in SEQ ID NO: 2 or 4;
(ii) DNA sequences comprising the nucleotide sequence depicted in SEQ ID NO: 1 or 3;
(iii) DNA sequences comprising a nucleotide sequence encoding a fragment or derivative of the protein encoded by the DNA sequence of (i) or (ii);
(iv) DNA sequences the complementary strand of which hybridizes with and which is at least 70% identical to the polynucleotide as defined in any one of (i) to (iii); and
(v) DNA sequences the nucleotide of which is degenerate to the nucleotide sequence of a DNA sequence of any one of (i) to (iv);

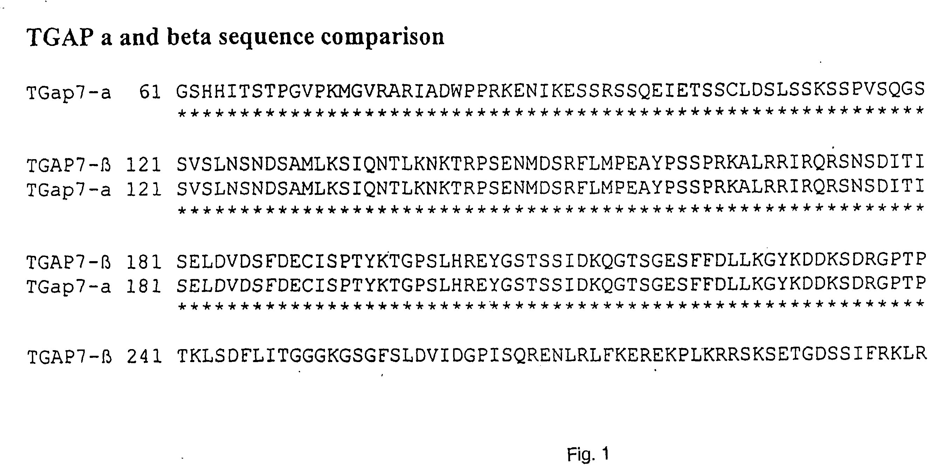

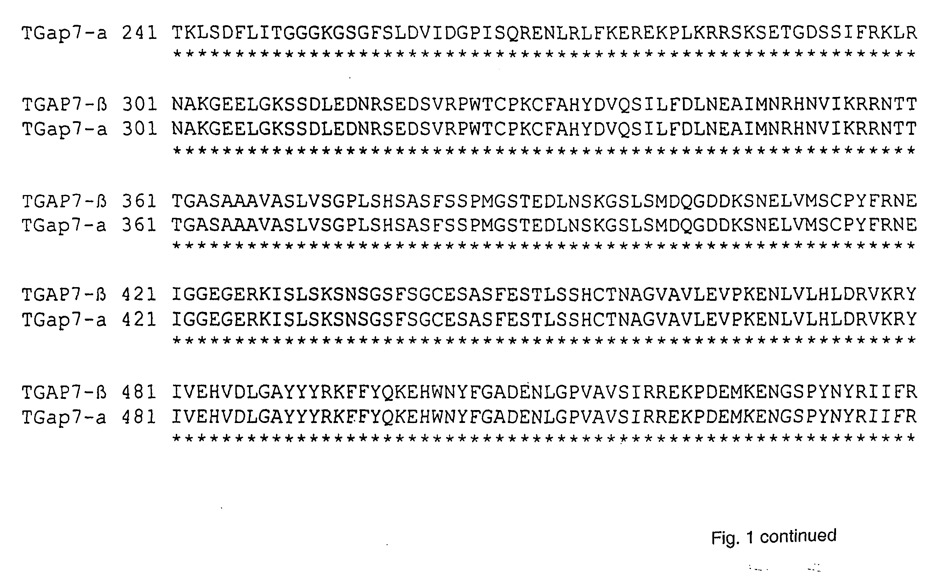

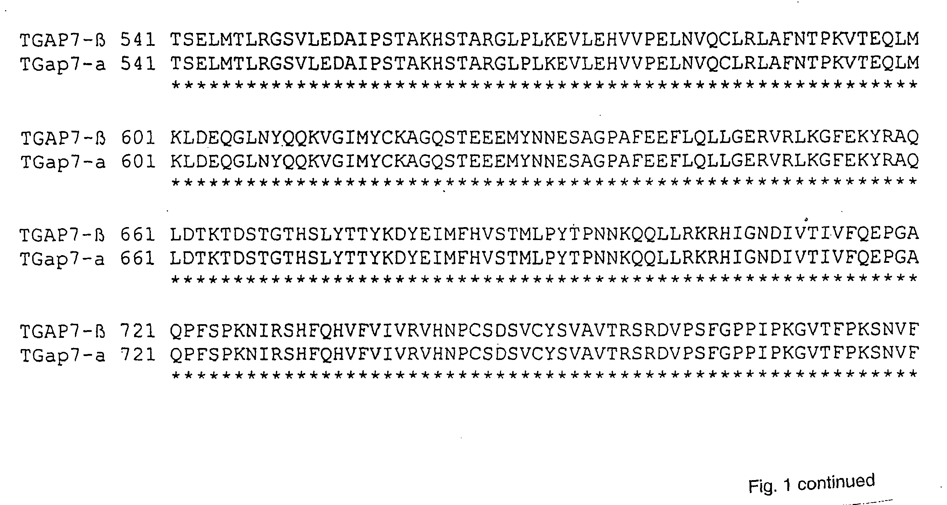

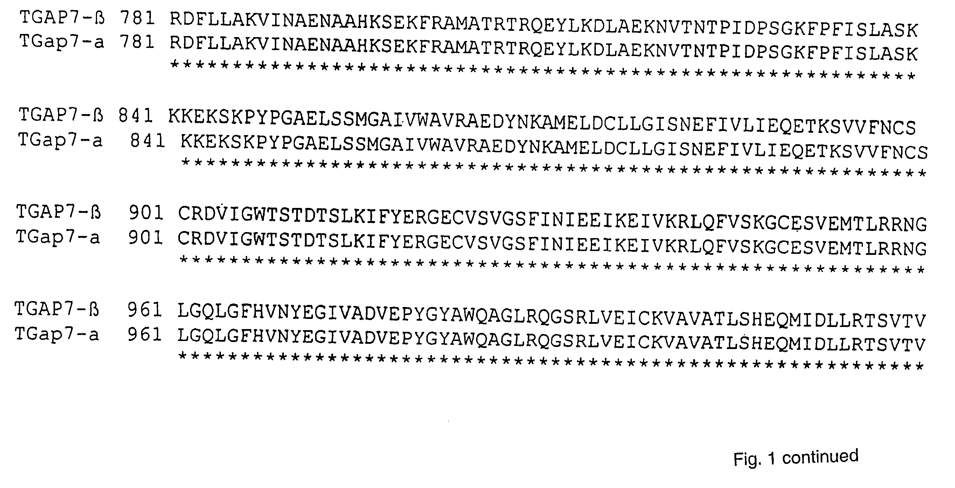

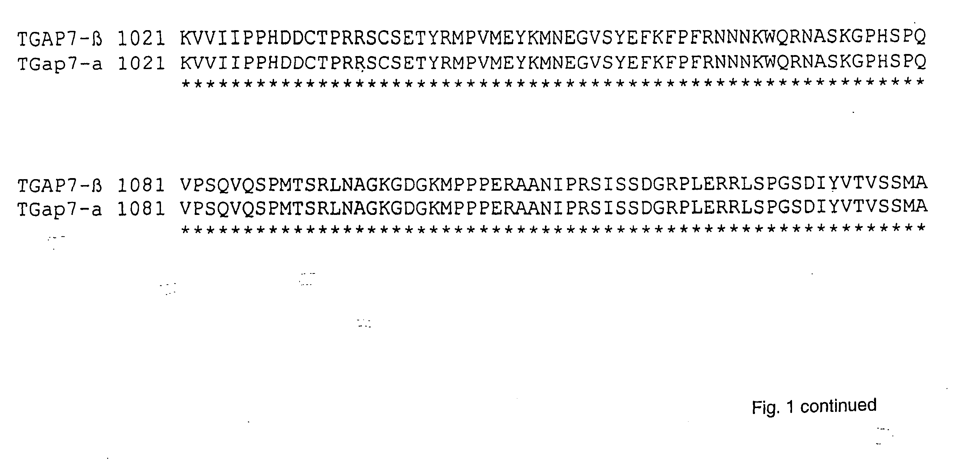

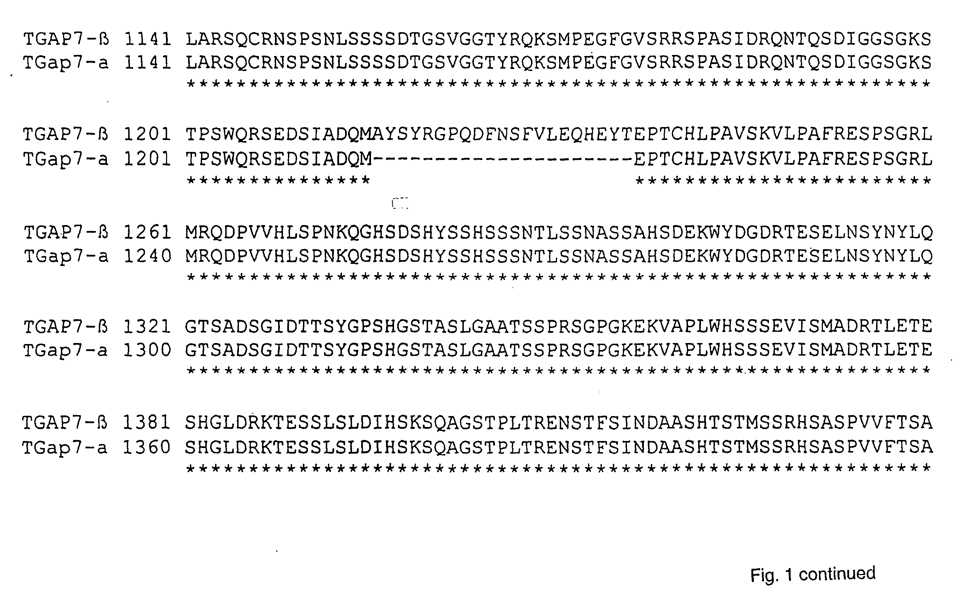

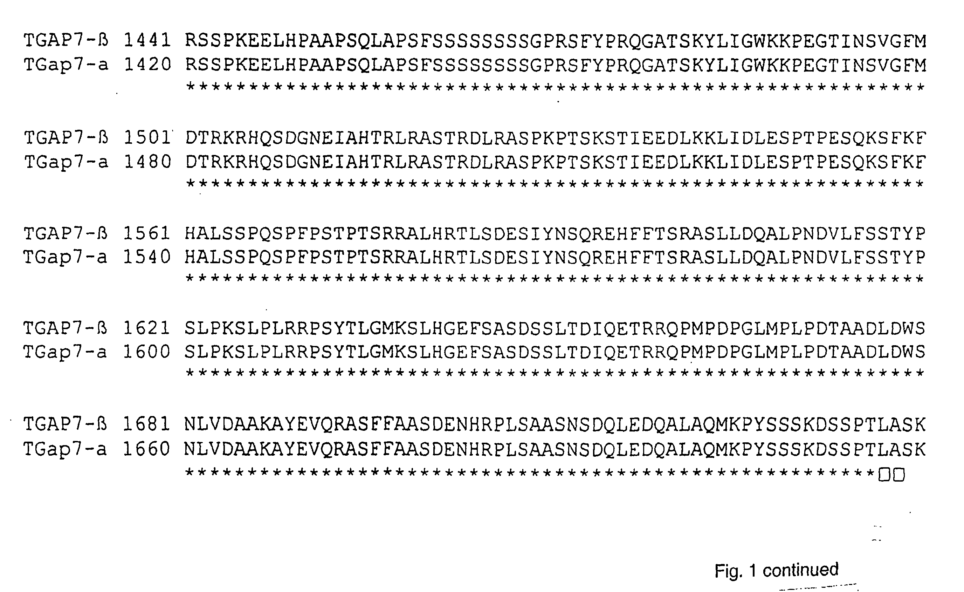

The term "TGAP7 protein", in accordance with the present invention, denotes a protein involved in the signal transduction of leucocyte activation and/or proliferation and down-regulation of which results in suppressing leucocyte, preferably T-, B-, NK-cell and/or monocyte proliferation in response to alloactivation in a mixed lymphocyte culture or in response to mitogens when exogeneously added to the culture. In accordance with this invention it has been surprisingly found that a cDNA is differentially expressed in alloactivated leucocytes, i.e. human T-cells. This differentially expressed cDNA was termed TGAP7 and it was shown that this cDNA encodes a protein sequence which is 100% identical to a putative GAP protein E6TPl (see Gao (1999), MCB 19, 733-744). In accordance with the present inventon, it has surprisingly been found that said protein/polypeptide plays an important role in the differentiation of quiescent T-cells to activate T-cells after allo-/autoantigen stimulation and/or cell activation/proliferation processes of B-cells, NK-cells and/or monocytes after stimulation by said allo-/autoantigen or by antigens from, inter alia, pathological agents, like viruses (viral agents), bacteria, etc.. The term "TGAP7" denotes proteins/polypeptides, in accordance with this invention, which are identical to theTGAP7-α or TGAP7-β proteins/polypeptides as described herein (see SEQ ID NOs: 2, 4, 5 or 6 and Figure 1) and the term comprises, furthermore, functional homologues of said protein/polypeptide.
Studies which had been carried out within the scope of the present invention revealed that antisense polynucleotides directed to the mRNA encoding TGAP7 protein are able to efficiently suppress the proliferation of lymphocytes and antigen presenting cells (monocytes, dentritic cells, B-cells) in response to alloactivation in a mixed lymphocyte culture or in response to mitogens. Alloantigen and mitogen (ConA and PHA) stimulated human lymphocytes and monocytes were incubated in the presence and absence of TGAP7 specific antisense oligonucleotides for 24, 48, 72, 96 and 168 h. For proliferation, thymidine uptake was determined after 6 h which demonstrated a significant inhibition of T-cell activation in the presence TGAP7-specific antisense oligonucleotides whereas the presence of unrelated control antisense oligonucleotides did not exhibit any effect on T-cell proliferation. For activation studies early T-cell specific markers such as CD69 and IL2 receptor were analyzed by flowcytometry on human peripheral blood lymphocytes in the presence and absence of TGAP7 specific antisense oligonucleotides which demonstrate significant downregulation of activation markers (like e.g. CD25-(IL2 receptor), CD69, UCA class II and transferrin receptor) on immune cells. Based on these results, it can be concluded that TGAP7 molecule is directly involved in the initiating of the immune response and might be an important target molecule for modulating the immune response.

The term "leucocytes" generally denotes all kinds of white blood cells and preferably refers to monocytes and lymphocytes (B, T and NK cells), either in combination or individually. Thus, it should be understood that the term leucocyte may also be used herein so as to refer to individual species of leucocytes such as T-cells only.

The term "biologically active fragment thereof" refers to peptides and polypeptides that are derived from said TGAP7 protein and that are capable of effecting the same or similar activity or at least one of said activities of TGAP7-α [see SEQ ID NO: 3 or 4] or TGAP7-β [see SEQ ID NO: 1 or 2]

In accordance with the present invention, a gene induced in the early stage of T-cell activation has been identified by examining mRNA expression in alloactivated human lymphocytes. Differential display-reverse transcription PCR analysis revealed a 450 bp cDNA fragment which was upregulated 24 h after allostimulation of a human T-cell line; see Example 1. The corresponding complete cDNAs named TGAP7-α (comprising 5965 bp / predicted 1783 amino acids) and TGAP7-β (comprising 6034 bp / predicted 1804 amino acids) amino acid protein share 100% homology with the putative GAP-protein E6TP1 as described by Gao (1999), MCB19, 733-744. TGAP7 mRNA is expressed in a variety of human tissues with various expression levels; see appended Example 2. Strictly, TGAP7 is expressed in all immuno tissues with highest expression levels in spleen, peripheral blood, lymph nodes, appendix, thymus, fetal liver and bone marrow cells. TGAP7 is expected to function in cell proliferation and differentiation events during T-cell and/or general leucocyte activation. More importantly, it has been found in accordance with the present invention that TGAP7 is probably essential in the differentiation events from quiescent to activated leucocytes, particular T-cells or other leucocytes, like B-cells, NK-cells or monocytes after stimulation by allo/autoantigens or in response to antigens, like bacterial or viral antigens. This could be demonstrated by antisense expression experiments with antisense polynucleotides derived from the TGAP7 cDNA. From this experiment, it can be concluded that TGAP7 plays an essential role in activation of immune responses at an early stage.

From the above it is evident that the nucleotide sequence(s) depicted in SEQ ID NOs. 1, 3, 5 or 6 encode(s) a novel class of immune response modulating proteins which were previously described as a putative GAP-protein (see Gao (1999), loc. cit.). By the provision of these nucleotide sequences it is now possible to isolate identical or similar polynucleotides which code for proteins with the biological, immunological activity of TGAP7 from other species or organisms. Said nucleotide sequences may be employed, in accordance with this invention, in the pharmaceutical compositions, uses and/or methods described herein. Well-established approaches for the identification and isolation of such related sequences are, for example, the isolation from genomic or cDNA libraries using the complete or part of the disclosed sequence as a probe or the amplification of corresponding polynucleotides by polymerase chain reaction using specific primers. Thus, the invention also relates to pharmaceutical compositions comprising polynucleotides which hybridize to the above described polynucleotides and differ at one or more positions in comparison to these as long as they encode a TGAP7 protein as defined above. Such molecules comprise those which are changed, for example, by deletion(s), insertion(s), alteration(s) or any other modification known in the art in comparison to the above described polynucleotides either alone or in combination. Methods for introducing such modifications in the polynucleotides of the invention are well-known to the person skilled in the art; see, e.g., Sambrook et al. (Molecular cloning; A Laboratory Manual, Second Edition, Cold Spring Harbor Laboratory Press, Cold Spring Harbor NY (1989)). The invention also relates to polynucleotides the nucleotide sequence of which differs from the nucleotide sequence of any of the above-described polynucleotides due to the degeneracy of the genetic code.

The term "hybridizing" is understood as referring to conventional hybridization conditions, preferably such as hybridization in 50%formamide/6xSSC/0.1 %SDS/10µg/ml ssDNA, in which temperatures for hybridization are above 37°C and temperatures for washing in 0.1xSSC/0.1%SDS are above 55°C. Most preferably, the term "hybridizing" refers to stringent hybridization conditions, for example such as described in Sambrook, supra.

Particularly preferred are polynucleotides which share at least 70%, preferably at least 85%, more preferably 90-95%, and most preferably 96-99% sequence identity with one of the above-mentioned polynucleotides and have the same biological activity. Such polynucleotides also comprise those which are altered, for example by nucleotide deletion(s), insertion(s), substitution(s), addition(s), and/or recombination(s) and/or any other modification(s) known in the art either alone or in combination in comparison to the above-described polynucleotides. Methods for introducing such modifications in the nucleotide sequence of the polynucleotide of the invention are well known to the person skilled in the art. Thus, the pharmaceutical composition(s), use(s) and method(s) of the present invention may comprise any polynucleotide that can be derived from the above described polynucleotides by way of genetic engineering and that encode upon expression a TGAP7 protein or a biologically active fragment thereof.

It is also immediately evident to the person skilled in the art that regulatory sequences may be added to the polynucleotide as defined herein and employed in the pharmaceutical composition, uses and/or methods of the invention. For example, promoters, transcriptional enhancers and/or sequences which allow for induced expression of the polynucleotide of the invention may be employed. A suitable inducible system is for example tetracycline-regulated gene expression as described, e.g., by Gossen and Bujard (Proc. Natl. Acad. Sci. USA 89 (1992), 5547-5551) and Gossen et al. (Trends Biotech. 12 (1994), 58-62).

In a further embodiment, the invention relates to a pharmaceutical composition comprising nucleic acid molecules of at least 15 nucleotides in length hybridizing specifically with a polynucleotide as described above or with a complementary strand thereof. Such nucleic acid molecules are often referred to as antisense (nucleic acid) molecules although they do not have to be necessarily "antisense" to the coding region of a gene but can have the same polarity as the cDNA. Repression of gene expression can still take place for example because of triple helix effects and/or sense supression, etc. Specific hybridization occurs preferably under stringent conditions and implies no or very little cross-hybridization with nucleotide sequences encoding no or substantially different proteins. Such nucleic acid molecules may be used as probes and/or for the control of gene expression. Nucleic acid probe technology is well known to those skilled in the art who will readily appreciate that such probes may vary in length. Preferred are nucleic acid probes of 17 to 35 nucleotides in length. Of course, it may also be appropriate to use nucleic acids of up to 100 and more nucleotides in length. Said nucleic acid probes are particularly useful for various pharmaceutical and/or diagnostic applications. On the one hand, they may be used as PCR primers for amplification of polynucleotides encoding TGAP7 proteins and/or its homologues and may, thereby, serve as useful diagnostic tools. Another application is the use as a hybridization probe to identify polynucleotides hybridizing to the polynucleotides encoding TGAP7 by homology screening of genomic DNA libraries. Nucleic acid molecules employed in this preferred embodiment of the invention which are complementary to a polynucleotide as described above may also be used for repression of expression of a gene comprising such a polynucleotide, for example due to an antisense or triple helix effect or for the construction of appropriate ribozymes (see, e.g., EP-A1 0 291 533, EP-A1 0 321 201, EP-A2 0 360 257) which specifically cleave the (pre)-mRNA of a gene comprising a polynucleotide as described herein above. Selection of appropriate target sites and corresponding ribozymes can be done as described for example in Steinecke, Ribozymes, Methods in Cell Biology 50, Galbraith et al. eds Academic Press, Inc. (1995), 449-460. Standard methods relating to antisense technology have also been described (Melani, Cancer Res. 51 (1991), 2897-2901). Said antisense or triple helix effect as well as the construction of relevant ribozymes is/are particularly useful in pharmaceutical compositions to be employed for the suppression of the immune system, e.g., in autoimmune diseases, for the treatment of rejection events during or after transplantation, etc.. Furthermore, the person skilled in the art is well aware that it is also possible to label such a nucleic acid probe with an appropriate marker for specific (inter alia, diagnostic) applications, such as for the detection of the presence of a polynucleotide as described herein above in a sample derived from an organism.
The above described nucleic acid molecules may either be DNA or RNA or a hybrid thereof. Furthermore, said nucleic acid molecule may contain, for example, thioester bonds and/or nucleotide analogues, commonly used in oligonucleotide anti-sense approaches. Said modifications may be useful for the stabilization of the nucleic acid molecule against endo- and/or exonucleases in the cell. Said nucleic acid molecules may be transcribed by an appropriate vector containing a chimeric gene which allows for the transcription of said nucleic acid molecule in the cell. Such nucleic acid molecules may further contain ribozyme sequences as described above.

In this respect, it is also to be understood that the polynucleotide to be used in the invention can be employed for "gene targeting" and/or "gene replacement", for restoring a mutant gene or for creating a mutant gene via homologous recombination; see for example Mouellic, Proc. Natl. Acad. Sci. USA, 87 (1990), 4712-4716; Joyner, Gene Targeting, A Practical Approach, Oxford University Press.

In a particular preferred embodiment of the present invention, the pharmaceutical composition comprising polynucleotides as defined herein above may be employed in vaccination approaches. Such vaccination approaches may be, inter alia, useful in prevention or treatment of malignant diseases, for example in the prevention or therapy of tumors of the hematopoietic system. Vaccination approaches employing nucleic acid molecules are well known in the art and are described, inter alia, in Leither, Vaccine 18 (2000), 765-777.

In a preferred embodiment said nucleic acid molecules to be employed in the pharmaceutical composition(s), uses and/or methods of the invention are labeled. Said labels may comprise radiolabels or fluorescence labels. In another preferred embodiment said nucleic acid molecules may be used for the suppression of TGAP7 expression. Particularly preferred in this embodiment are the above described hybridizing nucleic acid molecules.

The polynucleotide as employed in accordance with this invention and encoding the above described TGAP7 protein or (a) biologically active fragment(s) thereof may be, e.g., DNA, cDNA, RNA or synthetically produced DNA or RNA or a recombinantly produced chimeric nucleic acid molecule comprising any of those polynucleotides either alone or in combination. Preferably the polynucleotides including antisense molecules are part of a vector. Such vectors may comprise further genes such as marker genes which allow for the selection of said vector in a suitable host cell and under suitable conditions. Preferably, the polynucleotides are operatively linked to expression control sequences allowing expression in prokaryotic or eukaryotic cells. Expression of said polynucleotide comprises transcription of the polynucleotide into a translatable mRNA. Regulatory elements ensuring expression in eukaryotic cells, preferably mammalian cells, are well known to those skilled in the art. They usually comprise regulatory sequences ensuring initiation of transcription and optionally poly-A signals ensuring termination of transcription and stabilization of the transcript. Additional regulatory elements may include transcriptional as well as translational enhancers, and/or naturally-associated or heterologous promoter regions. Possible regulatory elements permitting expression in prokaryotic host cells comprise, e.g., the P_{L}, lac, trp or tac promoter in E. coli, and examples for regulatory elements permitting expression in eukaryotic host cells are the AOX1 or GAL1 promoter in yeast or the CMV-, SV40-, RSV-promoter (Rous sarcoma virus), CMV-enhancer, SV40-enhancer or a globin intron in mammalian and other animal cells. Beside elements which are responsible for the initiation of transcription such regulatory elements may also comprise transcription termination signals, such as the SV40-poly-A site or the tk-poly-A site, downstream of the polynucleotide. Furthermore, depending on the expression system used leader sequences capable of directing the polypeptide to a cellular compartment or secreting it into the medium may be added to the coding sequence of the polynucleotide of the invention and are well known in the art. The leader sequence(s) is (are) assembled in appropriate phase with translation, initiation and termination sequences, and preferably, a leader sequence capable of directing secretion of translated protein, or a portion thereof, into the periplasmic space or extracellular medium. Optionally, the heterologous sequence can encode a fusion protein including an C- or N-terminal identification peptide imparting desired characteristics, e.g., stabilization or simplified purification of expressed recombinant product. In this context, suitable expression vectors are known in the art such as Okayama-Berg cDNA expression vector pcDV1 (Pharmacia), pCDM8, pRc/CMV, pcDNA1, pcDNA3 (In-vitrogene), or pSPORT1 (GIBCO BRL).
Preferably, the expression control sequences will be eukaryotic promoter systems in vectors capable of transforming or transfecting eukaryotic host cells, but control sequences for prokaryotic hosts may also be used. Once the vector has been incorporated into the appropriate host, the host is maintained under conditions suitable for high level expression of the nucleotide sequences, and, as desired, the collection and purification of the protein of the invention may follow; see, e.g., the appended examples. In one preferred embodiment of the present invention antisense constructs are made based on the polynucleotide encoding TGAP7 (or (a) biologically active fragment(s) thereof) and combined with an appropriate expression control sequence.

In accordance with the above, the present invention relates to pharmaceutical compositions comprising (a) vector(s), particularly (a) plasmid(s), cosmid(s), virus(es) and bacteriophage(s) used conventionally in genetic engineering that comprise a polynucleotide encoding a TGAP7 protein and/or (a) functional fragment(s) thereof (as defined herein above). Methods which are well known to those skilled in the art can be used to construct recombinant vectors; see, for example, the techniques described in Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.Y. (1989). Alternatively, the polynucleotides and vectors to be employed in accordance with this invention can be reconstituted into liposomes for delivery to target-cells preferably to cells of the immune system. The here described vectors containing the polynucleotides described herein above can be transferred into the host-cell by well-known methods, which vary depending on the type of cellular host. For example, calcium chloride transfection is commonly utilized for prokaryotic cells, whereas calcium phosphate treatment or electroporation may be used for other cellular hosts; see Sambrook, supra.

In a more preferred embodiment, the present invention provides for a pharmaceutical composition comprising a vector as defined herein above, wherein said polynucleotide or nucleic acid molecule is operably linked to regulatory sequences allowing for the transcription and, optionally, expression of said nucleic acid molecules.

In a still further embodiment, the present invention relates to a pharmaceutical composition comprising a cell, preferably a host cell, containing the polynucleotide or vector described above. Preferably, said cell is a eukaryotic, most preferably a mammalian cell if therapeutic uses are envisaged. Of course, yeast and less preferred prokaryotic, e.g., bacterial cells may serve as well, in particular if the produced protein is used as a diagnostic means or if said protein is employed in methods as described herein above.
The polynucleotide or vector described herein which is present in the host cell may either be integrated into the genome of the host-cell or it may be maintained extrachromosomally.
The term "prokaryotic" is meant to include all bacteria which can be transformed or transfected with a DNA or RNA molecules for the expression of a protein of the invention. Prokaryotic hosts may include gram negative as well as gram positive bacteria such as, for example, E. coli, S. typhimurium, Serratia marcescens and Bacillus subtilis. The term "eukaryotic" is meant to include yeast, higher plant, insect and preferably mammalian cells. Depending upon the host employed in a recombinant production procedure, the protein encoded by the polynucleotide of the present invention may be glycosylated or may be non-glycosylated. TGAP7 proteins as employed in accordance with the present invention may also include an initial methionine amino acid residue. A polynucleotide as described herein can be used to transform or transfect the host using any of the techniques commonly known to those of ordinary skill in the art. Furthermore, methods for preparing fused, operably linked genes and expressing them in, e.g., mammalian cells and bacteria are well-known in the art (Sambrook, Molecular Cloning: A Laboratory Manual, Cold Spring Harbor Laboratory, Cold Spring Harbor, NY, 1989). The genetic constructs and methods described therein can be utilized for expression of the TGAP7 protein in eukaryotic or prokaryotic hosts. In general, expression vectors containing promoter sequences which facilitate the efficient transcription of the inserted polynucleotide are used in connection with the host. The expression vector typically contains an origin of replication, a promoter, and a terminator, as well as specific genes which are capable of providing phenotypic selection of the transformed cells. Furthermore, transgenic animals, preferably mammals, comprising nucleic acid molecules/polynucleotides as defined herein may be used for the large scale production of the TGAP7 protein and/or for the large scale production of pharmaceutical compositions described herein.
Alternatively, an animal, preferably mammalian cell naturally having a polynucleotide described herein present in its genome can be used and modified such that said cell expresses the endogenous gene corresponding to the polynucleotide described herein above under the control of an heterologous promoter. The introduction of the heterologous promoter which does not naturally control the expression of the polynucleotide of the invention can be done according to standard methods, see supra. Suitable promoter include those mentioned hereinbefore.

In this context, it should be mentioned that a method for the production of a TGAP7 protein or a biologically active fragment thereof may comprise:
(a) culturing a host described herein above under conditions allowing for the expression of the protein; or
(b) in vitro translation of the polynucleotide encoding TGAP7 and/or a biologically active fragment thereof;
and recovering the protein (or a fragment thereof) produced in (a) or (b).

The transformed hosts can be grown in fermentors and cultured according to techniques known in the art to achieve optimal cell growth. The TGAP7 protein and/or biologically active fragments thereof to be employed in pharmaceutical compositions, uses and/or methods of this invention can then be isolated from the growth medium, cellular lysates, or cellular membrane fractions. Once expressed, the protein of the present invention can be purified according to standard procedures of the art, including ammonium sulfate precipitation, affinity columns, column chromatography, gel electrophoresis and the like; see, Scopes, "Protein Purification", Springer-Verlag, N.Y. (1982). Substantially pure proteins of at least about 90 to 95% homogeneity are preferred, and 98 to 99% or more homogeneity are most preferred, for pharmaceutical uses. Once purified, partially or to homogeneity as desired, the proteins may then be used therapeutically (including extracorporeally) or in developing and performing assay procedures.

Hence, in a still further embodiment, the present invention relates to a pharmaceutical composition comprising a TGAP7 protein or a biologically active fragment thereof encoded by the polynucleotide described herein above or produced by a method of as above. It will be apparent to those skilled in the art that the TGAP7 protein or a (biologically active) fragment thereof can be further coupled to other moieties as described above for, e.g., drug targeting and imaging applications, i.e. for pharmaceutical and/or diagnostic uses. Such coupling may be conducted chemically after expression of the protein to site of attachment or the coupling product may be engineered into the protein of the invention at the DNA level. The DNAs are then expressed in a suitable host system, and the expressed proteins are collected and renatured, if necessary. TGAP7 proteins/polypeptides may by particularly useful in (a) pharmaceutical setting(s) where specific leucocyte activation should be controlled. As mentioned herein below, specific overexpression of TGAP7 proteins or (biologically acitve) fragments thereof may be obtained by gene therapeutic approaches. As documented in the appended examples, TGAP7 expression is induced by activation of cells of the immune system. Without being bound by theory, it is therefore envisaged that one function of TGAP7 is the control of cell activation events in the immune system.

Furthermore, the provision of the TGAP7 protein as described herein above enables the production of TGAP7 specific antibodies. In this respect, hybridoma technology enables production of cell lines secreting antibody to essentially any desired substance that produces an immune response. RNA encoding the light and heavy chains of the immunoglobulin can then be obtained from the cytoplasm of the hybridoma. The 5' end portion of the mRNA can be used to prepare cDNA to be inserted into an expression vector. The DNA encoding the antibody or its immunoglobulin chains can subsequently be expressed in cells, preferably mammalian cells.
Depending on the host-cell, renaturation techniques may be required to attain proper conformation of the antibody. If necessary, point substitutions seeking to optimize binding may be made in the DNA using conventional cassette mutagenesis or other protein engineering methodology such as is disclosed herein.

Thus, the present invention also relates to a pharmaceutical composition comprising an antibody specifically recognizing TGAP7 protein or (a) fragment(s) (peptides, polypeptides) thereof.

In a preferred embodiment of the invention, said antibody comprised in said pharmaceutical composition is a monoclonal antibody, a polyclonal antibody, a single chain antibody, humanized antibody, a xenogeneic antibody or fragment thereof that specifically binds said peptide or polypeptide also including bispecific antibody, synthetic antibody, antibody fragment, such as Fab, Fv or scFv fragments etc., or a chemically modified derivative of any of these. Monoclonal antibodies can be prepared, for example, by the techniques as originally described in Köhler and Milstein, Nature 256 (1975), 495, and Galfré, Meth. Enzymol. 73 (1981), 3, which comprise the fusion of mouse myeloma cells to spleen cells derived from immunized mammals with modifications developed by the art. Furthermore, antibodies or fragments thereof to the aforementioned peptides can be obtained by using methods which are described, e.g., in Harlow and Lane "Antibodies, A Laboratory Manual", CSH Press, Cold Spring Harbor, 1988. When derivatives of said antibodies are obtained by the phage display technique, surface plasmon resonance as employed in the BlAcore system can be used to increase the efficiency of phage antibodies which bind to an epitope of the peptide or polypeptide of the invention (Schier, Human Antibodies Hybridomas 7 (1996), 97-105; Malmborg, J. Immunol. Methods 183 (1995), 7-13). The production of chimeric antibodies is described, for example, in WO89/09622. Methods for the production of humanized antibodies are described in, e.g., EP-A1 0 239 400 and WO90/07861 A further source of antibodies to be utilized in accordance with the present invention are so-called xenogenic antibodies. The general principle for the production of xenogenic antibodies such as human antibodies in mice is described in, e.g., WO 91/10741, WO 94/02602, WO 96/34096 and WO 96/33735. Antibodies to be employed in accordance with the invention or their corresponding immunoglobulin chain(s) can be further modified using conventional techniques known in the art, for example, by using amino acid deletion(s), insertion(s), substitution(s), addition(s), and/or recombination(s) and/or any other modification(s) known in the art either alone or in combination. Methods for introducing such modifications in the DNA sequence underlying the amino acid sequence of an immunoglobulin chain are well known to the person skilled in the art; see, e.g., Sambrook, Molecular Cloning A Laboratory Manual, Cold Spring Harbor Laboratory (1989) N.Y. It is particularly preferred that the here described pharmaceutical compositions comprise antibodies/antibody contructs which may be employed in intracellular settings. Such antibody constructs/antibodies are well known in the art and are, inter alia, described in Lener (2000), Eur. J. Biochem. 267, 1196-1205, who described intracellular antibodies against p21 ras.

In a still further embodiment, the present invention relates to a cell that has been modified to express a TGAP7 protein or an antibody as described herein. This embodiment may be well suited for, e.g., restoring B and/or T-cell responsiveness to an antigen, in particular if the antibody of the invention capable of stimulating T-cell proliferation is expressed in a form suitable to be presented on the cell surface.

The present invention furthermore relates to a pharmaceutical composition comprising an antisense construct capable of inhibiting the expression of a polynucleotide encoding TGAP7 (and/or (a) biologically active fragment(s) thereof) as defined herein above. As illustration in the appended example (Example 3) such antisense constructs/oligonucleotides are particularly useful in the down regulation of leucocyte/lymphocyte responses/activations. Therefore, the here described pharmaceutical compositions comprising (specific) antisense constructs which are capable of inhibiting the expression of TGAP7 may be particularly useful in the treatment and/or prevention of pathological or medical situations where an immunoactivation is not desired. These situations comprise, but are not limited to, treatment of acute and chronic rejections of allo- and xeno- (organ)transplants or bone marrow transplantations, inflammation processes and/or allergies. The use of antisense oligonucleotides/constructs is well known in the art and described, inter alia, in Irizawa (1995), Clin. Exp. Immunology 100, 383-389 or Boeve (1994), J. Leucocyte Biol. 55, 169-174. Methods and computer programs for the preparation and rational selection of antisense oligonucleotide sequences are described in the prior art; see for example Smith, Eur. J. Pharm. Sci. 11 (2000), 191-198; Toschi, Methods 22 (2000), 261-269; Sohail, Adv. Drug Deliv. Rev. 44 (2000), 23-34; Moulton, J. Comput. Biol. 7 (2000), 277-292. These procedures comprise how to find optimal hybridization sites, and on how to select sequences that bind to for example TGAP7 mRNA. These methods can include the more empirical testing of large numbers of mRNA complementary sequences to the more systematic techniques, i.e. RNase H mapping, use of combinatorial arrays and prediction of secondary structure of mRNA by computational methods. Structures that bind to structured RNA, i.e. aptastrucs and tethered oligonucleotide probes, and foldback triplex-forming oligonucleotides can also be employed for the purpose of the present invention. Relating to selection of antisense sequences by aid of computational analysis, valuable www addresses are given in the above-identified prior art.
Secondary structure prediction and in vitro accessibility of mRNA as tools in the selection of target sites for ribozymes is described for example in Amarzguioui, Nucleic Acids Res. 28 (2000), 4113-4124. Minimising the secondary structure of DNA targets by incorporation of a modified deoxynucleoside and implications for nucleic acid analysis by hybridisation is described in Nguyen, Nucleic Acids Res. 28 (2000), 3904-3909. Preferably, the antisense molecules comprise at least 14 or 15, more preferably about 17 to 20 or more, and most preferably about at least 20, 25 or 30 or more consecutive nucleotides (including nucleotide analogs) of or complementary to any one of the above described polynucleotides encoding TGAP7 or corresponding genomic sequences, including 5'- and 3'-untranslated regions, introns, transcriptional regulatory sequences and the like. In a preferred embodiment the antisense molecule comprises said at least 14 or 15 nucleotides complementary to any one of SEQ ID NOS: 1, 3 and 5.

In yet another embodiment the present invention relates to the use of the pharmaceutical composition(s) described herein for use in cell or organ transplantation, for the treatment of autoimmune, allergic or infectious diseases or for the treatment of tumors.

An example for the use of the pharmaceutical composition of the invention for improving allograft or xenograft tolerance is described with respect to administration of an LFA-3 and CD2 binding protein, respectively, in WO93/06852.

The pharmaceutical composition of the present invention may further comprise a pharmaceutically acceptable carrier. Examples of suitable pharmaceutical carriers are well known in the art and include phosphate buffered saline solutions, water, emulsions, such as oil/water emulsions, various types of wetting agents, sterile solutions etc. Compositions comprising such carriers can be formulated by well known conventional methods. These pharmaceutical compositions can be administered to the subject at a suitable dose. Administration of the suitable compositions may be effected by different ways, e.g., by intravenous, intraperitoneal, subcutaneous, intramuscular, topical or intradermal administration. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. A typical dose can be, for example, in the range of 0.001 to 1000 µg (or of nucleic acid for expression or for inhibition of expression in this range); however, doses below or above this exemplary range are envisioned, especially considering the aforementioned factors. Generally, the regimen as a regular administration of the pharmaceutical composition should be in the range of 1 µg to 10 mg units per day. If the regimen is a continuous infusion, it should also be in the range of 1 µg to 10 mg units per kilogram of body weight per minute, respectively. Progress can be monitored by periodic assessment. Dosages will vary but a preferred dosage for intravenous administration of DNA is from approximately 10⁶ to 10¹² copies of the DNA molecule. The compositions of the invention may be administered locally or systemically. Administration will generally be parenterally, e.g., intravenously; DNA may also be administered directly to the target site, e.g., by biolistic delivery to an internal or external target site or by catheter to a site in an artery. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like. Furthermore, the pharmaceutical composition of the invention may comprise further agents such as T-cell, B-cell, NK-cell or monocyte costimulatory molecules or cytokines known in the art, or their inhibitors or activators depending on the intended use of the pharmaceutical composition.

Furthermore, it is envisaged by the present invention that the various polynucleotides and vectors encoding the above described peptides or polypeptides are administered either alone or in any combination using standard vectors and/or gene delivery systems, and optionally together with a pharmaceutically acceptable carrier or excipient. For example, the polynucleotide of the invention can be used alone or as part of a vector to express the (poly)peptide described herein in cells, for, e.g., gene therapy or diagnostics of diseases related to disorders of the immune system. The polynucleotides or vectors described herein are introduced into the cells which in turn produce the TGAP7 protein (or (a) fragment(s) thereof). Subsequent to administration, said polynucleotides or vectors may be stably integrated into the genome of the subject. On the other hand, viral vectors may be used which are specific for certain cells or tissues and persist in said cells. Suitable pharmaceutical carriers and excipients are well known in the art. The pharmaceutical compositions prepared according to the invention can be used for the prevention or treatment or delaying of different kinds of diseases, which are related to leucocyte, lymphocyte and/or monocyte related immunodeficiencies and malignancies such as multiple myeloma, T-, B-cell leukemia, infectious diseases related to T-, B-, NK-cell and monocyte proliferation, immune activation in rejection of transplants, autoimmune disorders, Allergy.

In another embodiment the present invention relates to a diagnostic composition comprising any one of the above described proteins, antibodies, (poly)peptides, polynucleotides, vectors or cells, and optionally suitable means for detection. The (poly)peptides and antibodies described above are, for example, suited for use in immunoassays in which they can be utilized in liquid phase or bound to a solid phase carrier. Examples of immunoassays which can utilize said (poly)peptide are competitive and non-competitive immunoassays in either a direct or indirect format. Examples of such immunoassays are the radioimmunoassay (RIA), the sandwich (immunometric assay) and the Western blot assay. The (poly)peptides and antibodies can be bound to many different carriers and used to isolate cells specifically bound to said polypeptides. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention.
There are many different labels and methods of labeling known to those of ordinary skill in the art. Examples of the types of labels which can be used in the present invention include enzymes, radioisotopes, colloidal metals, fluorescent compounds, chemiluminescent compounds, and bioluminescent compounds. The here described diagnostic compositions are particularly useful for the detection of an activated status of the immune system, in particular to detect activation of T-cells, B-cells, NK-cells and/or monocytes.

Said diagnostic compositions may also be used for methods for detecting expression of a polynucleotide encoding TGAP7 (or its homologues) by detecting the presence of mRNA coding for a TGAP7 protein which comprises obtaining mRNA from a cell and contacting the mRNA so obtained with a probe comprising a nucleic acid molecule of at least 15 nucleotides capable of specifically hybridizing with a polynucleotide encoding TGAP7 (or its homologues) under suitable hybridizing conditions (see also supra), detecting the presence of mRNA hybridized to the probe, and thereby detecting the expression of the TGAP7 protein (or its homologues) by the cell.
Furthermore, the invention comprises methods of detecting the presence of a TGAP7 protein in a sample, for example, a cell sample, which comprises obtaining a cell sample from a subject, contacting said sample with one of the aforementioned antibodies under conditions permitting binding of the antibody to the TGAP7 protein, and detecting the presence of the antibody so bound, for example, using immuno assay techniques such as radio-immunoassay or enzyme-immunoassay. Furthermore, one skilled in the art may specifically detect and distinguish polypeptides which are functional TGAP7 proteins from mutated forms which have lost or altered their leucocyte (T-cell, B-cell, etc.) stimulatory activity by using an antibody which either specifically recognizes a (poly)peptide which has TGAP7 activity but does not recognize an inactive form thereof or which specifically, recognizes an inactive form but not the corresponding polypeptide having TGAP7 activity. The antibodies as described in the present invention may also be used in affinity chromatography for purifying the TGAP7 protein or above described (poly)peptides and isolating them from various sources. Said purified proteins/(poly)peptides may be employed in the pharmaceutical compositions, uses and/or methods of the present invention.

The invention also relates to diagnosing in a subject a predisposition (susceptibility) to a disorder associated with the expression of a TGAP7 allele which comprises isolating DNA from victims of the the disorder associated with the under-or over-expression of a TGAP7 protein; digesting the isolated DNA with at least one restriction enzyme; electrophoretically separating the resulting DNA fragments on a sizing gel; contacting the resulting gel with a nucleic acid probe as described above capable of specifically hybridizing to DNA encoding a TGAP7 protein and labeled with a detectable marker; detecting labeled bands on the gel which have hybridized to the labeled probe to create a band pattern specific to the DNA of victims of the disorder associated with the expression of a TGAP7 protein; preparing the subject's DNA according to the above-mentioned steps to produce detectable labeled bands on a gel; and comparing the band pattern specific to the DNA of victims of the disorder associated with the expression of a TGAP7 protein and the subject's DNA to determine whether the patterns are the same or different and to diagnose thereby predisposition to the disorder if the patterns are the same. The detectable markers of the present invention may be labeled with commonly employed radioactive labels, such as, for example, ³²P and ³⁵S, although other labels such as biotin or mercury as well as those described above may be employed as well. Various methods well-known to the person skilled in the art may be used to label the detectable markers. For example, DNA sequences and RNA sequences may be labeled with ³²P or ³⁵S using the random primer method. Once a suitable detectable marker has been obtained, various methods well-known to the person skilled in the art may be employed for contacting the detectable marker with the sample of interest. For example, DNA-DNA, RNA-RNA and DNA-RNA hybridizations may be performed using standard procedures. Various methods for the detection of nucleic acids are well-known in the art, e.g., Southern and northern blotting, PCR, primer extension and the like. Furthermore, the mRNA, cRNA, cDNA or genomic DNA obtained from the subject may be sequenced to identify mutations which may be characteristic fingerprints of TGAP7 mutations in disorders associated with the expression of TGAP7 or mutated versions thereof. The present invention further comprises methods, wherein such a fingerprint may be generated by RFLPs of DNA or RNA obtained from the subject, optionally the DNA or RNA may be amplified prior to analysis, the methods of which are well known in the art. RNA fingerprints may be performed by, for example, digesting an RNA sample obtained from the subject with a suitable RNA-Enzyme, for example RNase T₁, RNase T₂ or the like or a ribozyme and, for example, electrophoretically separating and detecting the RNA fragments on PAGE as described above or in the appended examples.

In another embodiment, the present invention relates to a pharmaceutical composition comprising an agent which stimulates a leucocyte through the TGAP7 protein as described herein, and optionally a pharmaceutically acceptable carrier. As is immediately evident to the person skilled in the art, the provision of the TGAP7 as an immunomodulating molecule opens up the way of alternative approaches for leucocytes stimulation and treating corresponding diseases. The agent that stimulates the proliferation of leucocytes or lymphocytes through the TGAP7 protein is expected to markedly enhance the proliferation of leucocytes or lymphocytes of, e.g., (activated) T-cells and thus is capable of augmenting the immune response. Examples for this type of "vaccine" are described, e.g., in WO91/11194 and in the literature, e.g., referred to above. The agents to be employed in accordance with the present invention usually specifically bind and/or interact to TGAP7 protein in order to exert their effect. Such agents can be identified in accordance with a method of the invention described below. Such agents also comprise promoters which can be inserted in front of the coding region of the TGAP7 protein encoding gene, e.g., via gene transfer and homologous recombination in the 5' untranslated region of the gene, see also supra. Such promoter may be regulated and thus permit the controlled expression of the TGAP7 protein in certain cells.

Therefore, in a further aspect the present invention relates to a method for identifying a binding partner to a TGAP7 polypeptide comprising:
(a) contacting a TGAP7 polypeptide (protein) of the invention with a compound to be screened; and
(b) determining whether the compound effects an activity of the polypeptide (protein).

TGAP7 polypeptides may be used to screen for molecules that bind to TGAP7 or for molecules to which TGAP7 binds. The binding of TGAP7 and the molecule may activate (agonist), increase, inhibit (antagonist), or decrease activity of the TGAP7 or the molecule bound. Examples of such molecules include antibodies (including single-chain antibodies), oligonucleotides, proteins (e.g., receptors), or small molecules.
Preferably, the molecule is closely related to the natural binding partner of TGAP7, e.g., a fragment of the binding partner, or a natural substrate, a "ligand", a structural or functional mimetic; see, e.g., Coligan, Current Protocols in Immunology 1(2) (1991); Chapter 5. Similarly, the molecule can be closely related to the natural binding partner(s) with which TGAP7 interacts, or at least, a fragment of said binding and/or interaction partner capable of being bound by TGAP7 (e.g., active site). In either case, the molecule can be rationally designed using known techniques; see also infra. (A) potential binding partner(s) of TGAP7 is/are G-protein interacting molecule(s).
Preferably, the screening for these molecules involves producing appropriate cells which express TGAP7, either as a secreted protein or as a protein in or on the cell membrane. Preferred cells include cells from mammals, yeast, Drosophila, or E. coli. Cells expressing TGAP7 (or cell membrane(s) containing the expressed polypeptide) are then preferably contacted with a test compound potentially containing the molecule to observe binding, stimulation, or inhibition of activity of either TGAP7 or the molecule.
The assay may simply test binding of a candidate compound to TGAP7, wherein binding is detected by a label, or in an assay involving competition with a labeled competitor. Further, the assay may test whether the candidate compound results in a signal generated by binding to TGAP7.

Alternatively, the assay can be carried out using cell-free preparations, polypeptide/molecule affixed to a solid support, chemical libraries, or natural product mixtures. The assay may also simply comprise the steps of mixing a candidate compound with a solution containing TGAP7, measuring TGAP7/molecule activity or binding, and comparing the TGAP7/molecule activity or binding to a standard.
Preferably, an ELISA assay can measure TGAP7 level or activity in a sample (e.g., biological sample) using a monoclonal or polyclonal antibody. The antibody can measure TGAP7 level or activity by either binding, directly or indirectly, to TGAP7 or by competing with TGAP7 for a substrate.
All of these above assays can be used as diagnostic or prognostic markers. The molecules discovered using these assays can be used to treat disease or to bring about a particular result in a patient (e.g., increase of immune response) by activating or inhibiting the TGAP7/molecule: Moreover, the assays can discover agents which may inhibit or enhance the production of TGAP7 from suitably manipulated cells or tissues.

Therefore, the invention includes a method of identifying compounds which bind to TGAP7 comprising the steps of:
(a) incubating a candidate binding compound with TGAP7; and
(b) determining if binding has occurred.

Moreover, the invention includes a method of identifying agonists/antagonists comprising the steps of:
(a) incubating a candidate compound with TGAP7;
(b) assaying a biological activity as described above, and
(c) determining if a biological activity of TGAP7 has been altered.

As mentioned hereinbefore, the polynucleotides encoding TGAP7 (or (a) fragment(s) thereof) and polypeptides representing TGAP7 (or (a) fragment(s) thereof) provide a basis for the development of mimetic compounds that may be inhibitors or activators of TGAP7 or their encoding genes. It will be appreciated that the present invention also provides cell based screening methods that allow a high-throughput-screening (HTS) of compounds that may be candidates for such inhibitors and activators.

Furthermore, the invention relates to a method for identifying leucocyte activating or co-stimulating compounds or for identifying inhibitors of leucocyte activation and stimulation comprising
(a) culturing leucocytes, lymphocytes or monocytes in the presence of the TGAP7 protein, (poly)peptide, antibody, cell and/or the antisense construct described above and, optionally, in the presence of a component capable of providing a detectable signal in response to leucocyte proliferation/activation, with a compound to be screened under conditions permitting interaction of the compound with the TGAP7 protein, (poly)peptide, antibody or cell(s); and
(b) detecting the presence or absence of a signal generated from the interaction of the compound with the cells.

The term "compound" in the method of the invention includes a single substance or a plurality of substances which may or may not be identical.
Said compound(s) may be comprised in, for example, samples, e.g., cell extracts from, e.g., plants, animals or microorganisms. Furthermore, said compounds may be known in the art but hitherto not known to be capable of inhibiting proliferation of leucocytes or not known to be useful as an immune response costimulatory factor, respectively. The plurality of compounds may be, e.g., added to a simple in vitro, to the culture medium or injected into the cell.
If a sample containing (a) compound(s) is identified in the method of the invention, then it is either possible to isolate the compound from the original sample identified as containing the compound, in question or one can further subdivide the original sample, for example, if it consists of a plurality of different compounds, so as to reduce the number of different substances per sample and repeat the method with the subdivisions of the original sample. It can then be determined whether said sample or compound displays the desired properties by methods known in the art such as described herein and in the appended examples. Depending on the complexity of the samples, the steps described above can be performed several times, preferably until the sample identified according to the method of the invention only comprises a limited number of or only one substance(s). Preferably said sample comprises substances of similar chemical and/or physical properties, and most preferably said substances are identical. The methods of the present invention can be easily performed and designed by the person skilled in the art, for example in accordance with other cell based assays described in the prior art (see, e.g., EP-A-0 403 506) or by using and modifying the methods as described in the appended examples. Furthermore, the person skilled in the art will readily recognize which further compounds and/or cells may be used in order to perform the methods of the invention, for example, B cells, interleukins, or enzymes, if necessary, that, e.g., convert a certain compound into the precursor which in turn stimulates or suppresses lymphocyte or monocyte activation or that provide for (co)stimulatory signals. Such adaptation of the method of the invention is well within the skill of the person skilled in the art and can be performed without undue experimentation.

Compounds which can be used in accordance with the method of the present invention include peptides, proteins, nucleic acids including cDNA expression libraries, antibodies, small organic compounds, ligands, peptidomimetics, PNAs and the like. Said compounds can also be functional derivatives or analogues of known leucocyte, lymphocyte (B-, T- or NK-cell) or monocyte activators or inhibitors. Methods for the preparation of chemical derivatives and analogues are well known to those skilled in the art and are described in, for example, Beilstein, Handbook of Organic Chemistry, Springer edition New York Inc., 175 Fifth Avenue, New York, N.Y. 10010 U.S.A. and Organic Synthesis, Wiley, New York, USA. Furthermore, said derivatives and analogues can be tested for their effects according to methods known in the art or as described, for example, in the appended examples. Furthermore, peptidomimetics and/or computer aided design of appropriate activators or inhibitors of leucocytes, lymphocytes, monocytes (like T-cell, B-cell, NK-cell) activation can be used, for example, according to the methods described below. Appropriate computer programs can be used for the identification of interactive sites of a putative inhibitor and the TGAP7 protein (or its biologically active fragment(s)) by computer assistant searches for complementary structural motifs (Fassina, Immunomethods 5 (1994), 114-120). Further appropriate computer systems for the computer aided design of protein and peptides are described in the prior art, for example, in Berry, Biochem. Soc. Trans. 22 (1994), 1033-1036; Wodak, Ann. N. Y. Acad. Sci. 501 (1987), 1-13; Pabo, Biochemistry 25 (1986), 5987-5991. The results obtained from the above-described computer analysis can be used in combination with the method of the invention for, e.g., optimizing known leucocyte activators or inhibitors. Appropriate peptidomimetics can also be identified by the synthesis of peptidomimetic combinatorial libraries through successive chemical modification and testing the resulting compounds, e.g., according to the methods described herein and in the appended examples. Methods for the generation and use of peptidomimetic combinatorial libraries are described in the prior art, for example in Ostresh, Methods in Enzymology 267 (1996), 220-234 and Dorner, Bioorg. Med. Chem. 4 (1996), 709-715. Furthermore, the three-dimensional and/or crystallographic structure of inhibitors or activators of leucocyte stimulation can be used for the design of peptidomimetic inhibitors or activators of leucocyte activation to be tested in the method of the invention (Rose, Biochemistry 35 (1996), 12933-12944; Rutenber, Bioorg. Med. Chem. 4 (1996), 1545-1558).

In summary, the present invention provides methods for identifying compounds which are capable of modulating immune responses. Accordingly compounds identified in accordance with the method of the present invention to be inhibitors and activators, respectively, of immune response are also within the scope of the present invention.
Compounds found to enhance leucocyte proliferation may be used in the treatment of cancer or infections and related diseases. In addition, it may also be possible to specifically inhibit viral diseases, thereby preventing viral infection or viral spread. Compounds identified as suppressors of leucocyte proliferation can be used, e.g., for treating skin conditions (see, e.g., WO93/06866) or in allogenic or xenogenic cell or organ transplantation in order to avoid graft rejection; see also supra.
The compounds identified or obtained according to the method of the present invention are thus expected to be very useful in diagnostic and in particular for therapeutic applications.

The therapeutically useful compounds identified according to the method of the invention may be administered to a patient by any appropriate method for the particular compound, e.g., orally, intravenously, parenterally, transdermally, transmucosally, or by surgery or implantation (e.g., with the compound being in the form of a solid or semi-solid biologically compatible and resorbable matrix) at or near the site where the effect of the compound is desired. Therapeutic doses are determined to be appropriate by one skilled in the art, see also supra.

Such useful compounds can be for example transacting factors which bind to the TGAP7 protein described herein. Identification of transacting factors can be carried out using standard methods in the art (see, e.g., Sambrook, supra, and Ausubel, supra). To determine whether a protein binds to the TGAP7 protein, standard native gel-shift analyses can be carried out. In order to identify a transacting factor which binds to the TGAP7 protein, the polypeptides and peptides described in this invention can be used as an affinity reagent in standard protein purification methods, or as a probe for screening an expression library. Once the transacting factor is identified, modulation of its binding to the TGAP7 protein as described herein can be pursued, beginning with, for example, screening for inhibitors against the binding of the transacting factor to the TGAP7 protein. Activation or repression of TGAP7 specific genes could then be achieved in subjects by applying the transacting factor (or its inhibitor) or the gene encoding it, e.g., in a vector described in the embodiments hereinbefore. In addition, if the active form of the transacting factor is a dimer, dominant-negative mutants of the transacting factor could be made in order to inhibit its activity. Furthermore, upon identification of the transacting factor, further components in the pathway leading to activation (e.g. signal transduction) or repression of a gene encoding the TGAP7 protein described herein can then be identified. Modulation of the activities of these components can then be pursued, in order to develop additional drugs and methods for modulating the expression or activity of the TGAP7 protein.

Beside the above described possibilities to use the polynucleotides according to the invention for gene therapy and their use to identify homologous molecules, the described polynucleotides may also be used for several other applications, for example, for the identification of nucleic acid molecules which encode proteins which interact with the TGAP7 protein described above. This can be achieved by assays well known in the art, for example, as described in Scofield (Science 274 (1996), 2063-2065) by use of the so-called yeast "two-hybrid system". In this system the (poly)peptide encoded by the polynucleotides according to the invention or a smaller part thereof is linked to the DNA-binding domain of the GAL4 transcription factor. A yeast strain expressing this fusion protein and comprising a lacZ reporter gene driven by an appropriate promoter, which is recognized by the GAL4 transcription factor, is transformed with a library of cDNAs which will express animal, preferably mammal proteins or peptides thereof fused to an activation domain. Thus, if a peptide encoded by one of the cDNAs is able to interact with the fusion protein comprising a (poly)peptide of the invention, the complex is able to direct expression of the reporter gene. In this way the polynucleotide according to the invention and the encoded peptide can be used to identify peptides and proteins interacting with TGAP7 proteins.
Other methods for identifying compounds which interact with the TGAP7 protein according to the invention or nucleic acid molecules encoding such molecules are, for example, the in vitro screening with the phage display system as well as filter binding assays or "real time" measuring of interaction using, for example, the BIAcore apparatus (Pharmacia); see references cited supra.

Furthermore, the present invention relates to the use of the polynucleotide, the nucleic acid molecule, the vectors, peptides, polypeptides, antibodies and cells described herein for the preparation of a composition for diagnosing and/or the treatment of acute and chronic diseases involving T-cell activation and associated with Th1 and Th2 immune response, for the treatment of acute and chronic rejection of allo-and xeno organ transplants and bone marrow transplantation, for the treatment of rheumatoid arthritis, lupus erythramatodes, multiple sklerosis, encephalitis, vasculitis, diabetes mellitus, pancreatitis, gastritis, thyroiditis, for the treatment of disorders (inter alia malignant disorders) of T-, B- or NK-cells, for the treatment of asthma, lepramatosis, Helicobacter pylori associated gastritis or for the treatment of skin tumors, adrenal tumors or lung tumors, wound healing, growth disorders, inflammatory and/or infectious diseases. It is particularly preferred that the polynucleotide encoding TGAP7 (or (a) fragment(s) thereof) or the antibody as defined herein above is employed for the detection of leucocyte activation and/or for the treatment of diseases linked to leucocyte activation.

As described before, the present invention for the first time provided a novel route of therapeutic intervention via modulating, preferably inhibiting the activity of the above-described TGAP7 protein. Therefore, the present invention generally relates to the use of an effective amount of a compound capable of suppressing TGAP7 activity for treating disease, disorder or condition as described above. Preferably, said suppressing of TGAP7 activity results in inhibiting of the proliferation of PHA activated T-cell-lymphocytes. This can be tested e.g. according to the method described in Example 3. Compounds that may be used for the above-described methods include those identified by the methods of the present invention and comprise for example TGAP7 antisense molecules, an anti-TGAP7 antibodies, peptides or peptide mimetics of TGAP7 protein, ligands, substrates or binding partners of TGAP7.

The polynucleotides, vectors, cells, proteins, (poly)peptides, antibodies, inhibitors, activators, pharmaceutical and diagnosis compositions, uses described herein above and methods of the invention can be used for the treatment of all kinds of diseases hitherto unknown as being related to or dependent on the modulation of TGAP7. The pharmaceutical compositions, methods and uses of the present invention may be desirably employed in humans, although animal treatment is also encompassed by the methods and uses described herein.

These and other embodiments are disclosed and encompassed by the description and Examples of the present invention. Further literature concerning any one of the antibodies, methods, uses and compounds to be employed in accordance with the present invention may be retrieved from public libraries and databases, using for example electronic devices. For example the public database "Medline" may be utilized which is available on the Internet, for example under http://www.ncbi.nlm.nih.gov/PubMed/medline.html. Further databases and addresses, such as http://www.ncbi.nlm.nih.gov/, http://www.infobiogen.fr/, http://www.fmi.ch/biology/research-tools.html, http://www.tigr.org/, are known to the person skilled in the art and can also be obtained using, e.g., hftp://www.lycos.com. An overview of patent information in biotechnology and a survey of relevant sources of patent information useful for retrospective searching and for current awareness is given in Berks, TIBTECH 12 (1994), 352-364.

### BRIEF DESCRIPTION OF DRAWINGS

This disclosure may best be understood in conjunction with the accompanying drawings, incorporated herein by references, which show:
**Figure 1****:**
   Sequence comparison of amino acids between TGAP7 alpha and beta.
**Figure 2a****:**
   Expression of the 6 and 9 kb mRNAs of TGAP7 in different tissues of the immune system.
**Figure 2b****:**
   TGAP7 is induced after allostimulation of human lymphocytes (MLC 24). Furthermore, TGAP7 is expressed in EBV-transformed B-cell lines as well as in Jurkat cells (Jr, Ja).
**Figure 3****:**
   Antiproliferative effects of TGAP7 antisense oligonucleotides on PHA activated T-cells in vitro. Intense proliferative inhibition of T-GAP-anti-Sense 2 (5 M) in contrast to the control-oligo and the further control without oligo-addition.

A better understanding of the present invention and of its many advantages will be had from the following examples, given by way of illustration.

### Example 1: Cloning of a novel cDNA TGAP7, that is differentially expressed in allo- and mitogen activated human T cells

To identify novel genes induced during the early stages of T cell activation in response to alloantigens, differential display RT-PCR analysis of mRNA expression was performed at time 0 and 24 h after initiation of a human mixed lymphocyte culture (MLR). In conformance with institutional policies regarding human experimentation, peripheral blood lymphocytes (PBLs, "mixed lymphcoytes") were isolated from healthy human volunteers using standard Ficoll centrifugation methods and diluted into RPMI containing 10% fetal calf serum. Responder PBLs were stimulated with equal numbers of irradiated (3000 rad, 13 min) stimulator PBLs. Cells were co-cultured for 24 h in tissue flasks at an initial concentration of 10⁶ cells/ml for RNA isolation. For mitogen stimulation, cells were cultured in PHA or ConA (1 mg/l) in culture flasks at 37°C at an initial concentration of 10 Million cells/ml for 24 h prior to isolation of RNA. Total RNA was isolated from MLR at 0 and 24 h using the RNAzol B method (Tel-Test, Inc) and differential display was performed as described previously (Kojima et al., 1996 [10]). Briefly, 2 µg of total RNA was reverse transcribed using an oligo-dT primer and 200 U MMLV reverse transcriptase (Gibco/BRL). A 40 cycle PCR amplification with a total volume of 10 µl was performed by using 1 µg of cDNA, 1.25 mM MgCl₂, 50 mM KCI, 10 mM Tris-HCl (pH 8.3), 2.5 nM primer, 5 µCi ³⁵S-dATP, and 0.3 U Taq polymerase. The primers for the PCR amplification were: 5'- GACGGAACAGCTTC -3' [SEQ ID NO: 7] and 5'- TGCGTCTGGTTCT-3' [SEQ ID NO: 8].
The PCR products were stored at 4°C and separated by electrophoresis in 6% polyacrylamide-urea gels, transferred to filter paper, dried, and autoradiographed. The differentially expressed cDNA fragment was excised from the gel, eluted, reamplifed, cloned into pBluescriptSK⁺ plasmid, and sequenced. Homology searches were performed using BLAST at NCBl. Alignments were performed using Geneworks 2.1.1.

### Results:

DDRTPCR analysis (Ref: Utku (1998) Prevention of acute allograft rejection by antibody targeting of TIRC7, a novel T cell membrane protein, Immunity, 509-518) of alloactivated human lymphocytes revealed a 450 bp fragment which was used to isolate the full-length cDNA from human T cell library. Said human T-cell cDNA library (obtained from Clontech) was screened by using the 450 bp TGAP7 cDNA fragment which revealed several clones. TGAP7 alpha (5965 bp) and TGAP7 beta (6034 bp) show identity to recently published cDNA clones obtained by yeast two hybrid screening (Gao (1999), loc. cit.)

### Example 2: Expression studies for TGAP7

Northern blots containing RNA from various human tissues were purchased from Clontech. Northern blots were probed with TGAP7 cDNA. Overnight hybridizations were performed with ³²P labeled cDNA probes (10⁶ cpm/µl) at 42°C in 40% formamide, 10% dextran sulfate, 4 X SSC, 7 mM Tris (pH 7.6), 0.8 X Denhardt's solution, 0.02 mg/ml salmon sperm DNA, and 10% SDS. Blots were washed twice in 2 X SSC and 0.1 % SDS for 20 min at room temperature, once at 65°C in 0.2 X SSC, 0.1 % SDS and autoradiographed at -80°C.

Northern blots were prepared with 7-10 µg of total RNA as described previously (Kojima et al., 1996 [10]). Northern blots were probed with the full-length TGAP7 cDNA. Overnight hybridizations were performed with ³²P labeled cDNA probes (10⁶ cpm/µl) at 42°C in 40% formamide, 10% dextran sulfate, 4 X SSC, 7 mM Tris (pH 7.6), 0.8 X Denhardt's solution, 0.02 mg/ml salmon sperm DNA, and 10% SDS. Blots were washed twice in 2 X SSC and 0.1% SDS for 20 min at room temperature, once at 65°C in 0.2 X SSC, 0.1% SDS and autoradiographed at -80°C.

### Results:

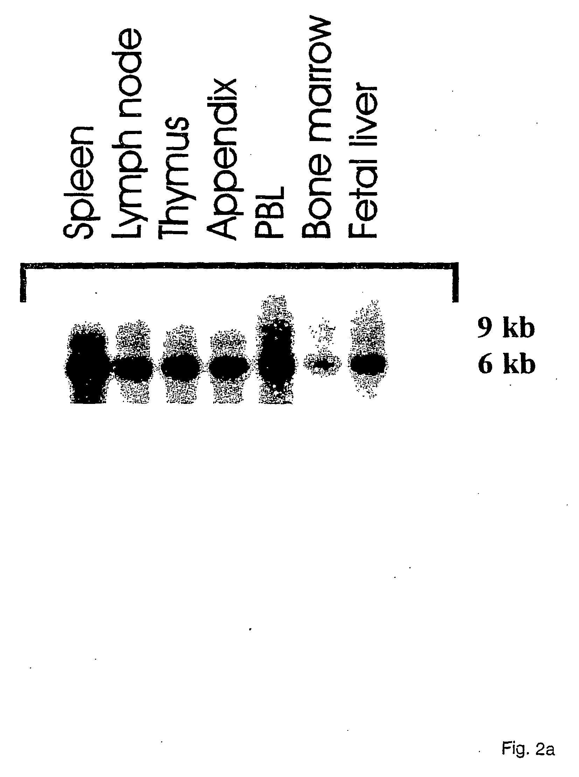

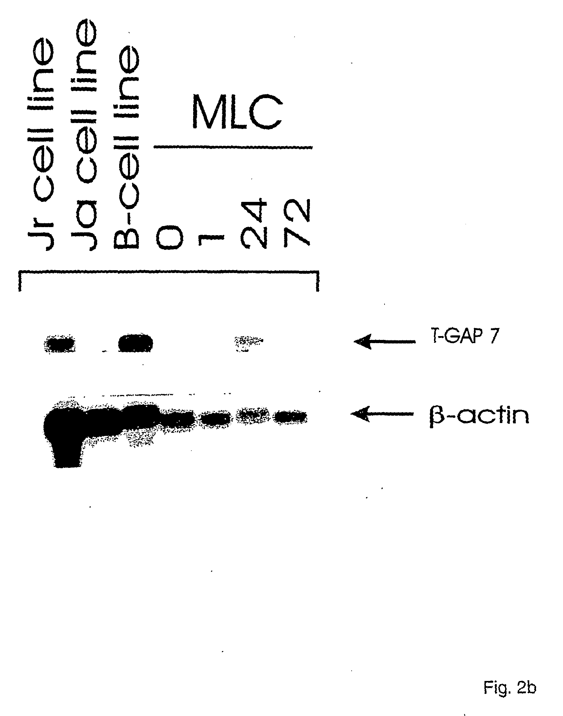

A TGAP7 specific cDNA probe detected 6 and 9 kb transcripts. Northern analysis revealed that TGAP7 6 kb cDNA is expressed in all immune tissues and exhibits highest levels of mRNA expression in spleen, and peripheral blood, followed by lymph nodes, appendix, thymus, and fetal liver and bone marrow cells, respectively. (Figure 2a).
To determine the expression kinetics of TGAP7, Northern blot analysis of total RNA from alloantigen activated lymphocytes was performed.
A TGAP7 specific cDNA probe detected only the 6 kb transcript which resulted after alloactivation of PBL in a 10-fold upregulation of TGAP7 expression at 24 h. The mRNA is significantly present in EBV transformed B cells and Jurkat cells, respectively (Figure 2b).

### Example 3: Effects of TGAP7 specific antisense oligonucleotids in T-cell proliferation

Peripheral blood lymphocytes (PBMC) were isolated from human volunteers using standard Ficoll centrifugation methods and diluted into RPMI 1640 containing 10% FKS. Number of cells was determined with Neubauer hemocytometer.
PBMC were exposed to phytohemagglutinin (PHA) (1 µg/ml). 20.000 cells in a final volume of 0,1 ml of complete RPMI were added to individual wells of a 96-well microtiter plate.

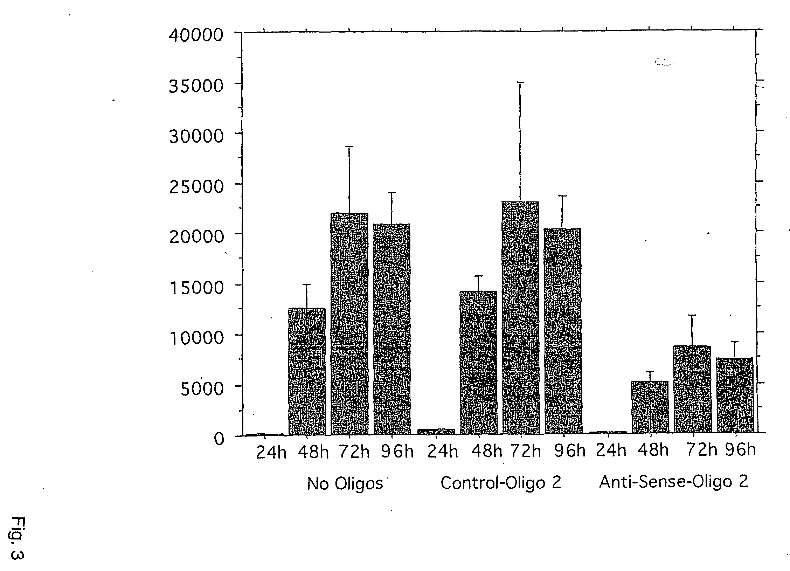

To study influence of TGAP7-antisense oligonucleotides on proliferative response of PHA-stimulated lymphocytes, cells were incubated in the presence and absence of an antisense oligonucleotide A2, a sense oligonucleotide (A1) complementary to A2 and two control oligonucleotides (control oligo C1 and C2) in a concentration of 5 µM oligos (diluted into 1 x TE-Puffer, pH 7.2). The 96-well plates were incubated at 37 °C and 5% CO₂ 24 hr, 48 hr, 72 hr, 96 hr and 168 hr, pulsed with 1 µCi of [³H] thymidine per well and harvested 6 hrs later. Incorporated radioactivity was determined in a scintillation counter and the datas were evaluated with StatView (Figure 3).

### Results:

The creation of immune response was analyzed in the presence and absence of TGAP7 specific antisense oligonucleotides in in vitro cultures of human lymphocytes such as in mixed lymphocyte culture including T, B, NK and monocytes and mitogen activated cells. As shown in Figure 2, the results demonstrate a significant downregulation of immune response to alloantigen and mitogens in the presence of TGAP7 specific antisense (A2), whereas sense oligonucleotide, A1, or other control oligonucleotides did not exhibit any immunomodulatory effect.

### References

[1] G.R. Crabtree, Contingent genetic regulatory events in T lymphocyte activation, Science 243 (1989) 355-361.
[2] C.H. June, Signal transduction in T-cells, Curr. Opin. Immunol. 3 (1991) 287-293.
[3] R. H. Schwartz, Costimulation of T lymphocytes: the role of CD28, CTLA-4, and B7/BB1 in Interleukin-2 production and immunotherapy, Cell 71 (1992) 1065-1068.
[4] J. Banchereau, F. Bazan, D. Blanchard, F. Briere, J. Galizzi, C. van Kooten, Y. Liu, F. Rousset, S. Seeland, The CD40 antigen and its ligand, Annu. Rev. Immunol. 12 (1994) 881-922.
[5] D.J. Lenschow, T. Walunas, J. Bluestone, CD28/B7 system of T-cell costimulation, Annu. Rev. Immunol. 14 (1996) 233-258.
[6] P. Linsley, J. Ledbetter, The role of the CD28 receptor during T-cell responses to antigen, Annu. Rev. Immunol. 11 (1993) 191-212.
[7] A. Kupfer, S.L. Swain, S.J. Singer, The specific direct interaction of helper T-cells and antigen-presenting B cells. II. Reorientation of the microtubule organizing center and reorganization of the membrane-associated cytoskeleton inside the bound helper T-cells, J. Exp. Med. 165 (1987) 1565-1580.
[8] M.V. Parsey, G.K. Lewis, Actin polymerization and pseudopod reorganization accompany anti-CD3-induced growth arrest in Jurkat T-cells, J. Immunol. 151 (1993) 1881-1893.
[9] N. Selliah, W.H. Brooks, T.L. Roszman, Proteolytic cleavage of -actinin by calpain in T-cells stimulated with anti-CD3 monoclonal antibody, J. Immunol. 156 (1996) 3215-3221.
[10] R. Kojima, J. Randall, B.M. Brenner, S.R. Gullans, Osmotic stress protein 94 (Osp94): A new member of the Hsp110/SSE gene subfamily, J. Biol. Chem. 271 (1996) 12327-12332.

<170> PatentIn Ver. 2.1
<210> 1
   <211> 6028
   <212> DNA
   <213> Homo sapiens
<220>
   <221> CDS
   <222> (349)..(5760)
<400> 1
   ggtgtggacg ttgtctaaat ttcggtagcc atggcacaag aatataagaa agcatgggat 60
   tatggcaacc acagaatctc agtagtacaa gttccattca gttttttctg aaagaaagcc 120
   ctctgttaaa gtgaagcaaa gaaactgttg tggattataa cgtttagaag ttccaatttt 180
   tcagtgcttt acaaataaag catcatttaa ccttttaaat gaaaaagatt aagatctcat 240
   gcaactgttg tattttctgg aagccattct ccaaaaggga agtgcacatt taaaacacag 300
   atatgatggt ccttgctgca gggatttaag tctacttgct tttacatc atg acc agc  357
                                                        Met Thr Ser
                                                         1
   ttg aaa cgg tca cag aca gaa agg cct ctt gcc act gac agg gcc tct   405
   Leu Lys Arg Ser Gln Thr Glu Arg Pro Leu Ala Thr Asp Arg Ala Ser
        5                  10                  15
   gtt gtt ggc aca gac ggc acc ccc aaa gtc cac act gat gat ttc tac   453
   Val Val Gly Thr Asp Gly Thr Pro Lys Val His Thr Asp Asp Phe Tyr
    20                  25                  30                  35
   atg cgg cgc ttc cgg tcc caa aat ggc agc tta gga tca tca gtt atg   501
   Met Arg Arg Phe Arg Ser Gln Asn Gly Ser Leu Gly Ser Ser Val Met
                    40                  45                  50
   gct cct gta gga ccc ccc cga agt gaa ggt tct cac cat ata acc tca   549
   Ala Pro Val Gly Pro Pro Arg Ser Glu Gly Ser His His Ile Thr Ser
                55                  60                  65
   acc ccc gga gtc cca aaa atg ggg gta agg gca agg att gca gat tgg   597
   Thr Pro Gly Val Pro Lys Met Gly Val Arg Ala Arg Ile Ala Asp Trp
            70                  75                  80
   ccc cca aga aag gaa aac ata aaa gaa tct agc cgt tca agc cag gaa   645
   Pro Pro Arg Lys Glu Asn Ile Lys Glu Ser Ser Arg Ser Ser Gln Glu
        85                  90                  95
   ata gaa acc tca agt tgc ctt gat agc ctg tcc tcc aaa agc agt cct   693
   Ile Glu Thr Ser Ser Cys Leu Asp Ser Leu Ser Ser Lys Ser Ser Pro
   100                 105                 110                 115
   gtg agt cag gga agt tct gtt agc ctc aat tcc aat gac tca gcc atg   741
   Val Ser Gln Gly Ser Ser Val Ser Leu Asn Ser Asn Asp Ser Ala Met
                   120                 125                 130
   ctg aaa agc ata cag aac acg ctg aaa aac aag aca aga ccg tcg gag   789
   Leu Lys Ser Ile Gln Asn Thr Leu Lys Asn Lys Thr Arg Pro Ser Glu
               135                 140                 145
   aac atg gac tcc aga ttt ctc atg cct gaa gcc tac ccc agc tcc ccc     837
   Asn Met Asp Ser Arg Phe Leu Met Pro Glu Ala Tyr Pro Ser Ser Pro
           150                 155                 160
   aga aaa gct ctt cgc aga ata cgc cag cga agc aac agt gat atc acc     885
   Arg Lys Ala Leu Arg Arg Ile Arg Gln Arg Ser Asn Ser Asp Ile Thr
       165                 170                 175
   ata agt gaa ctt gat gtg gat agc ttt gat gaa tgt atc tca cct aca     933
   Ile Ser Glu Leu Asp Val Asp Ser Phe Asp Glu Cys Ile Ser Pro Thr
   180                 185                 190                 195
   tac aag act gga cca tca ctg cac agg gaa tat ggt agc aca tct tca     981
   Tyr Lys Thr Gly Pro Ser Leu His Arg Glu Tyr Gly Ser Thr Ser Ser
                   200                 205                 210
   att gat aaa cag gga aca tct gga gaa agc ttt ttt gat ttg tta aag     1029
   Ile Asp Lys Gln Gly Thr Ser Gly Glu Ser Phe Phe Asp Leu Leu Lys
               215                 220                 225
   ggc tac aaa gat gac aaa tct gat cga ggt cca act cca acc aag ctc     1077
   Gly Tyr Lys Asp Asp Lys Ser Asp Arg Gly Pro Thr Pro Thr Lys Leu
           230                 235                 240
   agt gac ttt ctc att act ggt ggt ggc aag ggt tct ggt ttc tct ttg     1125
   Ser Asp Phe Leu Ile Thr Gly Gly Gly Lys Gly Ser Gly Phe Ser Leu
       245                 250                 255
   gat gta ata gac ggg cct atc tca cag aga gag aac ctc agg ctt ttt     1173
   Asp Val Ile Asp Gly Pro Ile Ser Gln Arg Glu Asn Leu Arg Leu Phe
   260                 265                 270                 275
   aag gaa agg gaa aaa cca ctc aag cga cgt tca aaa tct gaa act gga     1221
   Lys Glu Arg Glu Lys Pro Leu Lys Arg Arg Ser Lys Ser Glu Thr Gly
                   280                 285                 290
   gac tcc tct att ttt cgt aaa ttg cgc aat gcc aaa ggt gaa gaa ctt     1269
   Asp Ser Ser Ile Phe Arg Lys Leu Arg Asn Ala Lys Gly Glu Glu Leu
               295                 300                 305
   ggg aag tca tca gat ctt gaa gat aac cga tca gaa gac tct gtc agg     1317
   Gly Lys Ser Ser Asp Leu Glu Asp Asn Arg Ser Glu Asp Ser Val Arg
           310                 315                 320
   ccc tgg aca tgt cca aag tgc ttt gcc cac tat gat gtc cag agt ata     1365
   Pro Trp Thr Cys Pro Lys Cys Phe Ala His Tyr Asp Val Gln Ser Ile
       325                 330                 335
   tta ttt gat ttg aat gag gca att atg aac agg cac aat gtt att aag     1413
   Leu Phe Asp Leu Asn Glu Ala Ile Met Asn Arg His Asn Val Ile Lys
   340                 345                 350                 355
   agg aga aac acc acc act gga gct tcc gca gct gcc gtg gca tcc ttg     1461
   Arg Arg Asn Thr Thr Thr Gly Ala Ser Ala Ala Ala Val Ala Ser Leu
                   360                 365                 370
   gtc tct gga cct ctg tct cat tca gcc agt ttt agc tcc cca atg ggc    1509
   Val Ser Gly Pro Leu Ser His Ser Ala Ser Phe Ser Ser Pro Met Gly
               375                 380                 385
   agc aca gag gac ctg aat tcc aaa gga agc ctc agc atg gac cag gga    1557
   Ser Thr Glu Asp Leu Asn Ser Lys Gly Ser Leu Ser Met Asp Gln Gly
           390                 395                 400
   gat gat aaa agc aat gag ctt gta atg agc tgt cca tat ttt cgg aat    1605
   Asp Asp Lys Ser Asn Glu Leu Val Met Ser Cys Pro Tyr Phe Arg Asn
       405                 410                 415
   gag ata ggt gga gaa ggg gag agg aaa atc agc ctt tca aaa tca aat    1653
   Glu Ile Gly Gly Glu Gly Glu Arg Lys Ile Ser Leu Ser Lys Ser Asn
   420                 425                 430                 435
   tct ggc tcc ttt agt gga tgt gaa agt gcc tcc ttt gag tct acc ctt    1701
   Ser Gly Ser Phe Ser Gly Cys Glu Ser Ala Ser Phe Glu Ser Thr Leu
                   440                 445                 450
   agt tcc cat tgc aca aat gca gga gtg gca gta ctt gaa gtg ccc aag    1749
   Ser Ser His Cys Thr Asn Ala Gly Val Ala Val Leu Glu Val Pro Lys
               455                 460                 465
   gag aac ttg gtg ttg cac cta gat aga gtg aaa aga tac atc gtg gaa    1797
   Glu Asn Leu Val Leu His Leu Asp Arg Val Lys Arg Tyr Ile Val Glu
           470                 475                 480
   cac gta gat ctg ggt gca tac tat tat aga aaa ttt ttc tac cag aag    1845
   His Val Asp Leu Gly Ala Tyr Tyr Tyr Arg Lys Phe Phe Tyr Gln Lys
       485                 490                 495
   gaa cac tgg aac tat ttt ggg gct gat gag aat ctt ggt cca gtg gct    1893
   Glu His Trp Asn Tyr Phe Gly Ala Asp Glu Asn Leu Gly Pro Val Ala
   500                 505                 510                 515
   gtg agc att cga agg gaa aaa cca gat gaa atg aaa gaa aat gga tct    1941
   Val Ser Ile Arg Arg Glu Lys Pro Asp Glu Met Lys Glu Asn Gly Ser
                   520                 525                 530
   ccg tac aac tac cga ata att ttt aga act agt gag ctc atg aca ctg    1989
   Pro Tyr Asn Tyr Arg Ile Ile Phe Arg Thr Ser Glu Leu Met Thr Leu
               535                 540                 545
   aga ggt tcg gtc ctg gag gac gcc att ccg tcg aca gcc aag cac tcg    2037
   Arg Gly Ser Val Leu Glu Asp Ala Ile Pro Ser Thr Ala Lys His Ser
           550                 555                 560
   aca gcc aga ggc ctg cct ctc aaa gaa gtg ctg gag cac gtg gtt cct    2085
   Thr Ala Arg Gly Leu Pro Leu Lys Glu Val Leu Glu His Val Val Pro
       565                 570                 575
   gag ctc aat gtc cag tgc ctg cgg ttg gcc ttc aac aca ccc aag gtc    2133
   Glu Leu Asn Val Gln Cys Leu Arg Leu Ala Phe Asn Thr Pro Lys Val
   580                 585                 590                 595
   aca gag cag ctc atg aaa ctg gat gaa caa ggg ctg aac tac cag cag    2181
   Thr Glu Gln Leu Met Lys Leu Asp Glu Gln Gly Leu Asn Tyr Gln Gln
                   600                 605                 610
   aaa gta ggc atc atg tac tgc aaa gct gga cag agc act gaa gaa gag   2229
   Lys Val Gly Ile Met Tyr Cys Lys Ala Gly Gln Ser Thr Glu Glu Glu
               615                 620                 625
   atg tac aac aat gag tca gct ggc cca gcc ttt gaa gaa ttc ctt caa   2277
   Met Tyr Asn Asn Glu Ser Ala Gly Pro Ala Phe Glu Glu Phe Leu Gln
           630                 635                 640
   cta ttg gga gag cga gtt cgg ctc aaa gga ttt gag aag tat cga gca   2325
   Leu Leu Gly Glu Arg Val Arg Leu Lys Gly Phe Glu Lys Tyr Arg Ala
       645                 650                 655
   cag ctt gat acc aaa act gac tcc act gga acc cat tct ctg tac aca   2373
   Gln Leu Asp Thr Lys Thr Asp Ser Thr Gly Thr His Ser Leu Tyr Thr
   660                 665                 670                 675
   aca tac aaa gat tat gaa att atg ttc cat gtt tct acc atg ctg cca   2421
   Thr Tyr Lys Asp Tyr Glu Ile Met Phe His Val Ser Thr Met Leu Pro
                   680                 685                 690
   tac aca ccc aac aac aaa caa cag ctc ctg agg aag cgg cac att gga   2469
   Tyr Thr Pro Asn Asn Lys Gln Gln Leu Leu Arg Lys Arg His Ile Gly
               695                 700                 705
   aat gat atc gta aca att gtt ttc caa gag cct gga gca cag cca ttc   2517
   Asn Asp Ile Val Thr Ile Val Phe Gln Glu Pro Gly Ala Gln Pro Phe
           710                 715                 720
   agc cca aaa aac atc cga tcc cac ttc cag cac gtt ttc gtc atc gtc   2565
   Ser Pro Lys Asn Ile Arg Ser His Phe Gln His Val Phe Val Ile Val
       725                 730                 735
   agg gtg cac aat ccg tgc tct gac agt gtc tgt tat agt gtg gct gtt   2613
   Arg Val His Asn Pro Cys Ser Asp Ser Val Cys Tyr Ser Val Ala Val
   740                 745                 750                 755
   acc agg tcc aga gat gtg cct tcc ttt ggg cct ccc att cct aaa ggg   2661
   Thr Arg Ser Arg Asp Val Pro Ser Phe Gly Pro Pro Ile Pro Lys Gly
                   760                 765                 770
   gtc act ttc cct aag tca aat gtg ttc agg gac ttc ctt ttg gcg aaa   2709
   Val Thr Phe Pro Lys Ser Asn Val Phe Arg Asp Phe Leu Leu Ala Lys
               775                 780                 785
   gtg att aat gca gaa aat gct gct cat aaa tcg gag aag ttt cgg gcc   2757
   Val Ile Asn Ala Glu Asn Ala Ala His Lys Ser Glu Lys Phe Arg Ala
           790                 795                 800
   atg gca act cgg acc cgc cag gaa tac ctg aaa gat ctg gca gaa aag   2805
   Met Ala Thr Arg Thr Arg Gln Glu Tyr Leu Lys Asp Leu Ala Glu Lys
       805                 810                 815
   aat gtc acc aac acc cct atc gac cct tct ggc aag ttt ccg ttc atc   2853
   Asn Val Thr Asn Thr Pro Ile Asp Pro Ser Gly Lys Phe Pro Phe Ile
   820                 825                 830                 835
   tct ctg gct tcc aag aag aag gaa aag tct aag cca tat cca gga gcc   2901
   Ser Leu Ala Ser Lys Lys Lys Glu Lys Ser Lys Pro Tyr Pro Gly Ala
                   840                 845                 850
   gag ctc agc agc atg ggg gcc att gta tgg gca gtc cgg gct gaa gac   2949
   Glu Leu Ser Ser Met Gly Ala Ile Val Trp Ala Val Arg Ala Glu Asp
               855                 860                 865
   tac aac aag gcc atg gaa cta gac tgc ctt tta ggg atc tcc aat gag   2997
   Tyr Asn Lys Ala Met Glu Leu Asp Cys Leu Leu Gly Ile Ser Asn Glu
           870                 875                 880
   ttc att gtg ctc att gaa cag gaa aca aag agc gtg gtc ttc aat tgt   3045
   Phe Ile Val Leu Ile Glu Gln Glu Thr Lys Ser Val Val Phe Asn Cys
       885                 890                 895
   tcc tgt aga gat gtg ata ggg tgg act tca act gac acc agc ctc aaa   3093
   Ser Cys Arg Asp Val Ile Gly Trp Thr Ser Thr Asp Thr Ser Leu Lys
   900                 905                 910                 915
   atc ttc tat gaa cga gga gaa tgt gtt tca gtg ggt agt ttt att aac   3141
   Ile Phe Tyr Glu Arg Gly Glu Cys Val Ser Val Gly Ser Phe Ile Asn
                   920                 925                 930
   att gag gag atc aaa gag att gtc aaa agg ttg cag ttt gtt tca aaa   3189
   Ile Glu Glu Ile Lys Glu Ile Val Lys Arg Leu Gln Phe Val Ser Lys
               935                 940                 945
   ggc tgt gaa tcg gtg gag atg act ctg cga aga aat ggg cta gga cag   3237
   Gly Cys Glu Ser Val Glu Met Thr Leu Arg Arg Asn Gly Leu Gly Gln
           950                 955                 960
   ctt ggc ttc cat gtc aac tat gag ggc att gtg gcg gat gtg gag ccc   3285
   Leu Gly Phe His Val Asn Tyr Glu Gly Ile Val Ala Asp Val Glu Pro
       965                 970                 975
   tac ggt tat gcc tgg cag gca ggg ctg agg cag ggc agt cgc ctg gtg   3333
   Tyr Gly Tyr Ala Trp Gln Ala Gly Leu Arg Gln Gly Ser Arg Leu Val
   980                 985                 990                 995
   gag atc tgc aag gtg gcg gta gcc act ctg agc cat gag cag atg atc   3381
   Glu Ile Cys Lys Val Ala Val Ala Thr Leu Ser His Glu Gln Met Ile
                   1000                1005                1010
   gac ctc ctg aga aca tct gtc acg gtg aag gtt gtc atc att ccc ccg   3429
   Asp Leu Leu Arg Thr Ser Val Thr Val Lys Val Val Ile Ile Pro Pro
               1015                1020                1025
   cat gat gac tgc acc ccg cgg agg agt tgc tct gaa acc tac cgc atg   3477
   His Asp Asp Cys Thr Pro Arg Arg Ser Cys Ser Glu Thr Tyr Arg Met
           1030                1035                1040
   cca gtg atg gag tac aaa atg aat gaa ggt gtt tca tac gaa ttc aag   3525
   Pro Val Met Glu Tyr Lys Met Asn Glu Gly Val Ser Tyr Glu Phe Lys
       1045                1050                1055
   ttt ccc ttc cga aat aat aac aag tgg cag agg aac gcc agc aag ggg   3573
   Phe Pro Phe Arg Asn Asn Asn Lys Trp Gln Arg Asn Ala Ser Lys Gly
   1060                1065                1070                1075
   cct cat tca cct caa gtc ccg tcc cag gtg cag agt ccc atg acc tcg   3621
   Pro His Ser Pro Gln Val Pro Ser Gln Val Gln Ser Pro Met Thr Ser
                   1080                1085                1090
   cgg ctg aat gct gga aaa gga gat ggg aag atg cct cct cca gaa aga     3669
   Arg Leu Asn Ala Gly Lys Gly Asp Gly Lys Met Pro Pro Pro Glu Arg
               1095                1100                1105
   gcc gcc aac atc cct cga agc atc tcc agt gac ggg cgc cca cta gag     3717
   Ala Ala Asn Ile Pro Arg Ser Ile Ser Ser Asp Gly Arg Pro Leu Glu
           1110                1115                1120
   agg cgg ctg tct cct ggt tcg gac atc tat gtg acg gtc tca tcc atg     3765
   Arg Arg Leu Ser Pro Gly Ser Asp Ile Tyr Val Thr Val Ser Ser Met
       1125                1130                1135
   gct tta gca aga tcc cag tgt cgg aac tct cct agc aac ttg tct tca     3813
   Ala Leu Ala Arg Ser Gln Cys Arg Asn Ser Pro Ser Asn Leu Ser Ser
   1140                1145                1150                1155
   tcc agt gat act ggt tct gtg ggg ggc act tac agg cag aag tcc atg     3861
   Ser Ser Asp Thr Gly Ser Val Gly Gly Thr Tyr Arg Gln Lys Ser Met
                   1160                1165                1170
   ccc gaa ggg ttt gga gtg agc cgt aga tcc cca gcc tcc att gac agg     3909
   Pro Glu Gly Phe Gly Val Ser Arg Arg Ser Pro Ala Ser Ile Asp Arg
               1175                1180                1185
   cag aac acc cag tca gat att ggt ggc agc gga aaa tcc acg cct agc     3957
   Gln Asn Thr Gln Ser Asp Ile Gly Gly Ser Gly Lys Ser Thr Pro Ser
           1190                1195                1200
   tgg caa aga agt gag gat agc att gct gac cag atg gct tac agt tat     4005
   Trp Gln Arg Ser Glu Asp Ser Ile Ala Asp Gln Met Ala Tyr Ser Tyr
       1205                1210                1215
   aga gga cct cag gat ttc aat tct ttt gtc ctc gag cag cat gaa tat     4053
   Arg Gly Pro Gln Asp Phe Asn Ser Phe Val Leu Glu Gln His Glu Tyr
   1220                1225                1230                1235
   aca gag cca aca tgc cat ctc cca gca gta tca aag gta ctg cca gct     4101
   Thr Glu Pro Thr Cys His Leu Pro Ala Val Ser Lys Val Leu Pro Ala
                   1240                1245                1250
   ttc cga gag agc ccc agt ggg aga tta atg cgg cag gat cca gtg gtt     4149
   Phe Arg Glu Ser Pro Ser Gly Arg Leu Met Arg Gln Asp Pro Val Val
               1255                1260                1265
   cat ttg tct cca aac aaa caa ggg cat tct gat agc cac tac tcg agc     4197
   His Leu Ser Pro Asn Lys Gln Gly His Ser Asp Ser His Tyr Ser Ser
           1270                1275                1280
   cac tcc agt agc aat act ctc tcc agc aat gcg tca agt gcc cat agt     4245
   His Ser Ser Ser Asn Thr Leu Ser Ser Asn Ala Ser Ser Ala His Ser
       1285                1290                1295
   gat gag aag tgg tac gat ggg gac cgc aca gaa tcc gaa ctc aac agc     4293
   Asp Glu Lys Trp Tyr Asp Gly Asp Arg Thr Glu Ser Glu Leu Asn Ser
   1300                1305                1310                1315
   tat aac tat ctg caa ggc acc tct gct gac agt ggc att gac acc acc    4341
   Tyr Asn Tyr Leu Gln Gly Thr Ser Ala Asp Ser Gly Ile Asp Thr Thr
                   1320                1325                1330
   tct tat ggc ccc agc cac ggc agc aca gcc tcg ctg ggg gct gcc aca    4389
   Ser Tyr Gly Pro Ser His Gly Ser Thr Ala Ser Leu Gly Ala Ala Thr
               1335                1340                1345
   tcg tca cct cgc tca ggg cca ggc aag gag aaa gtg gca ccc cta tgg    4437
   Ser Ser Pro Arg Ser Gly Pro Gly Lys Glu Lys Val Ala Pro Leu Trp
           1350                1355                1360
   cac agc tcc agt gaa gta atc tcc atg gca gat cgg act ttg gag aca    4485
   His Ser Ser Ser Glu Val Ile Ser Met Ala Asp Arg Thr Leu Glu Thr
       1365                1370                1375
   gag agc cac ggc ctg gac cgg aaa aca gag tct tcc ctg agc tta gac    4533
   Glu Ser His Gly Leu Asp Arg Lys Thr Glu Ser Ser Leu Ser Leu Asp
   1380                1385                1390                1395
   ata cac agc aag agc caa gcc ggc tcg acc cct ctg aca agg gag aac    4581
   Ile His Ser Lys Ser Gln Ala Gly Ser Thr Pro Leu Thr Arg Glu Asn
                   1400                1405                1410
   agc acc ttc agt ata aac gat gct gct tcc cac aca agt acc atg agc    4629
   Ser Thr Phe Ser Ile Asn Asp Ala Ala Ser His Thr Ser Thr Met Ser
               1415                1420                1425
   tcc cga cac tct gcc agc cca gtg gtt ttc acc agt gcc cgg agt tca    4677
   Ser Arg His Ser Ala Ser Pro Val Val Phe Thr Ser Ala Arg Ser Ser
           1430                1435                1440
   cct aaa gaa gag ctt cat cca gct gcc ccc tca cag ctc gca cca tcc    4725
   Pro Lys Glu Glu Leu His Pro Ala Ala Pro Ser Gln Leu Ala Pro Ser
       1445                1450                1455
   ttc tcc tcc tct tcc tcc tcc tcc tct ggt cct agg agt ttt tac cct    4773
   Phe Ser Ser Ser Ser Ser Ser Ser Ser Gly Pro Arg Ser Phe Tyr Pro
   1460                1465                1470                1475
   cgc cag ggc gct act agc aag tac ctg att gga tgg aaa aaa ccc gaa    4821
   Arg Gln Gly Ala Thr Ser Lys Tyr Leu Ile Gly Trp Lys Lys Pro Glu
                   1480                1485                1490
   gga acc ata aac tcc gtg gga ttt atg gac acg aga aag cgt cat cag    4869
   Gly Thr Ile Asn Ser Val Gly Phe Met Asp Thr Arg Lys Arg His Gln
               1495                1500                1505
   agc gat ggc aat gaa ata gcc cac acc agg ctg cgt gcc tca acc aga    4917
   Ser Asp Gly Asn Glu Ile Ala His Thr Arg Leu Arg Ala Ser Thr Arg
           1510                1515                1520
   gac ctc cgg gca tct cct aag cca acc tcc aag tcc acc att gaa gaa    4965
   Asp Leu Arg Ala Ser Pro Lys Pro Thr Ser Lys Ser Thr Ile Glu Glu
       1525                1530                1535
   gat cta aag aaa cta att gat ctt gaa agc cca act cct gaa tca cag    5013
   Asp Leu Lys Lys Leu Ile Asp Leu Glu Ser Pro Thr Pro Glu Ser Gln
   1540                1545                1550                1555
   aag agt ttt aag ttc cac gca ctc tcc tct cct cag tct cct ttc ccc 5061
   Lys Ser Phe Lys Phe His Ala Leu Ser Ser Pro Gln Ser Pro Phe Pro
   1560 1565 1570
   agc acc ccc acc tca cgg cgg gcc ttg cac aga aca ctg tcg gac gag 5109
   Ser Thr Pro Thr Ser Arg Arg Ala Leu His Arg Thr Leu Ser Asp Glu
   1575 1580 1585
   agc att tac aat agc cag agg gag cac ttt ttc acc tcc agg gcg tca 5157
   Ser Ile Tyr Asn Ser Gln Arg Glu His Phe Phe Thr Ser Arg Ala Ser
   1590 1595 1600
   ctt ctg gac caa gcc ctg ccc aac gac gtc ctc ttc agt agc acg tac 5205
   Leu Leu Asp Gln Ala Leu Pro Asn Asp Val Leu Phe Ser Ser Thr Tyr
   1605 1610 1615
   cct tct ctc ccc aag tcg ctc ccg ttg agg agg cct tct tac acc tta 5253
   Pro Ser Leu Pro Lys Ser Leu Pro Leu Arg Arg Pro Ser Tyr Thr Leu
   1620 1625 1630 1635
   gga atg aaa tcg ctg cat gga gag ttc tca gcc tcg gac agc tcc ctc 5301
   Gly Met Lys Ser Leu His Gly Glu Phe Ser Ala Ser Asp Ser Ser Leu
   1640 1645 1650
   act gac atc cag gag acc cgc agg cag cct atg ccc gac cct ggc ctg 5349
   Thr Asp Ile Gln Glu Thr Arg Arg Gln Pro Met Pro Asp Pro Gly Leu
   1655 1660 1665
   atg ccc ctg cct gac act gct gca gac ttg gat tgg tcc aac ctg gta 5397
   Met Pro Leu Pro Asp Thr Ala Ala Asp Leu Asp Trp Ser Asn Leu Val
   1670 1675 1680
   gat gct gcc aaa gcc tat gag gtc cag aga gcc tca ttt ttt gct gct 5445
   Asp Ala Ala Lys Ala Tyr Glu Val Gln Arg Ala Ser Phe Phe Ala Ala
   1685 1690 1695
   agt gat gaa aac cat cgc ccc ttg agt gct gca tcc aac agt gat cag 5493
   Ser Asp Glu Asn His Arg Pro Leu Ser Ala Ala Ser Asn Ser Asp Gln
   1700 1705 1710 1715
   ctg gag gac cag gct ctg gcc cag atg aag cct tac agc agc agt aaa 5541
   Leu Glu Asp Gln Ala Leu Ala Gln Met Lys Pro Tyr Ser Ser Ser Lys
   1720 1725 1730
   gac tcc tct ccc act ctg gct tct aaa gtg gac cag ctg gaa ggt atg 5589
   Asp Ser Ser Pro Thr Leu Ala Ser Lys Val Asp Gln Leu Glu Gly Met
   1735 1740 1745
   ctg aag atg ctt cgg gaa gat ttg aag aag gaa aaa gaa gac aaa gct 5637
   Leu Lys Met Leu Arg Glu Asp Leu Lys Lys Glu Lys Glu Asp Lys Ala
   1750 1755 1760
   cac ctt cag gcg gag gtg cag cac ctg cga gag gac aac ctg agg cta 5685
   His Leu Gln Ala Glu Val Gln His Leu Arg Glu Asp Asn Leu Arg Leu
   1765 1770 1775
   cag gag gag tcc cag aac gcc tcg gac aag ctg aag aag ttc aca gaa 5733
   Gln Glu Glu Ser Gln Asn Ala Ser Asp Lys Leu Lys Lys Phe Thr Glu
   1780                1785                1790                1795
   tgg gtc ttc aac acc ata gac atg agc tagggaaggc tgaggaggac        5780
   Trp Val Phe Asn Thr Ile Asp Met Ser
                   1800
   aggagaaggg cccagacact ccctccagtg agtgtcctgc agcccttatt ccctccatag 5840
   aaagcatcct cagagcacct tccctggctt cctactctgc cccctttcgg ggagtgcaca 5900
   acacaatagt tgcagatcaa caatcatcac ctgccttttg tagaaaagaa aaacaaaaaa 5960
   agtaaataaa aattttaaac agtaaaataa aagtttaact gctaaaaaaa aaaaaaaaaa 6020
   aaaaaaaa                                                         6028
<210> 2
   <211> 1804
   <212> PRT
   <213> Homo sapiens
<400> 2
   Met Thr Ser Leu Lys Arg Ser Gln Thr Glu Arg Pro Leu Ala Thr Asp
    1               5                  10                  15
   Arg Ala Ser Val Val Gly Thr Asp Gly Thr Pro Lys Val His Thr Asp
               20                  25                  30
   Asp Phe Tyr Met Arg Arg Phe Arg Ser Gln Asn Gly Ser Leu Gly Ser
           35                  40                  45
   Ser Val Met Ala Pro Val Gly Pro Pro Arg Ser Glu Gly Ser His His
       50                  55                  60
   Ile Thr Ser Thr Pro Gly Val Pro Lys Met Gly Val Arg Ala Arg Ile
   65                  70                  75                  80
   Ala Asp Trp Pro Pro Arg Lys Glu Asn Ile Lys Glu Ser Ser Arg Ser
                   85                  90                  95
   Ser Gln Glu Ile Glu Thr Ser Ser Cys Leu Asp Ser Leu Ser Ser Lys
               100                 105                 110
   Ser Ser Pro Val Ser Gln Gly Ser Ser Val Ser Leu Asn Ser Asn Asp
           115                 120                 125
   Ser Ala Met Leu Lys Ser Ile Gln Asn Thr Leu Lys Asn Lys Thr Arg
       130                 135                 140
   Pro Ser Glu Asn Met Asp Ser Arg Phe Leu Met Pro Glu Ala Tyr Pro
   145                 150                 155                 160
   Ser Ser Pro Arg Lys Ala Leu Arg Arg Ile Arg Gln Arg Ser Asn Ser
                   165                 170                 175
   Asp Ile Thr Ile Ser Glu Leu Asp Val Asp Ser Phe Asp Glu Cys Ile
               180                 185                 190
   Ser Pro Thr Tyr Lys Thr Gly Pro Ser Leu His Arg Glu Tyr Gly Ser
           195                 200                 205
   Thr Ser Ser Ile Asp Lys Gln Gly Thr Ser Gly Glu Ser Phe Phe Asp
       210                 215                 220
   Leu Leu Lys Gly Tyr Lys Asp Asp Lys Ser Asp Arg Gly Pro Thr Pro
   225                 230                 235                 240
   Thr Lys Leu Ser Asp Phe Leu Ile Thr Gly Gly Gly Lys Gly Ser Gly
                   245                 250                 255
   Phe Ser Leu Asp Val Ile Asp Gly Pro Ile Ser Gln Arg Glu Asn Leu
               260                 265                 270
   Arg Leu Phe Lys Glu Arg Glu Lys Pro Leu Lys Arg Arg Ser Lys Ser
           275                 280                 285
   Glu Thr Gly Asp Ser Ser Ile Phe Arg Lys Leu Arg Asn Ala Lys Gly
       290                 295                 300
   Glu Glu Leu Gly Lys Ser Ser Asp Leu Glu Asp Asn Arg Ser Glu Asp
   305                 310                 315                 320
   Ser Val Arg Pro Trp Thr Cys Pro Lys Cys Phe Ala His Tyr Asp Val
                   325                 330                 335
   Gln Ser Ile Leu Phe Asp Leu Asn Glu Ala Ile Met Asn Arg His Asn
               340                 345                 350
   Val Ile Lys Arg Arg Asn Thr Thr Thr Gly Ala Ser Ala Ala Ala Val
           355                 360                 365
   Ala Ser Leu Val Ser Gly Pro Leu Ser His Ser Ala Ser Phe Ser Ser
       370                 375                 380
   Pro Met Gly Ser Thr Glu Asp Leu Asn Ser Lys Gly Ser Leu Ser Met
   385                 390                 395                 400
   Asp Gln Gly Asp Asp Lys Ser Asn Glu Leu Val Met Ser Cys Pro Tyr
                   405                 410                 415
   Phe Arg Asn Glu Ile Gly Gly Glu Gly Glu Arg Lys Ile Ser Leu Ser
               420                 425                 430
   Lys Ser Asn Ser Gly Ser Phe Ser Gly Cys Glu Ser Ala Ser Phe Glu
           435                 440                 445
   Ser Thr Leu Ser Ser His Cys Thr Asn Ala Gly Val Ala Val Leu Glu
       450                 455                 460
   Val Pro Lys Glu Asn Leu Val Leu His Leu Asp Arg Val Lys Arg Tyr
   465                 470                 475                 480
   Ile Val Glu His Val Asp Leu Gly Ala Tyr Tyr Tyr Arg Lys Phe Phe
                   485                 490                 495
   Tyr Gln Lys Glu His Trp Asn Tyr Phe Gly Ala Asp Glu Asn Leu Gly
               500                 505                 510
   Pro Val Ala Val Ser Ile Arg Arg Glu Lys Pro Asp Glu Met Lys Glu
           515                 520                 525
   Asn Gly Ser Pro Tyr Asn Tyr Arg Ile Ile Phe Arg Thr Ser Glu Leu
       530                 535                 540
   Met Thr Leu Arg Gly Ser Val Leu Glu Asp Ala Ile Pro Ser Thr Ala
   545                 550                 555                 560
   Lys His Ser Thr Ala Arg Gly Leu Pro Leu Lys Glu Val Leu Glu His
                   565                 570                 575
   Val Val Pro Glu Leu Asn Val Gln Cys Leu Arg Leu Ala Phe Asn Thr
               580                 585                 590
   Pro Lys Val Thr Glu Gln Leu Met Lys Leu Asp Glu Gln Gly Leu Asn
           595                 600                 605
   Tyr Gln Gln Lys Val Gly Ile Met Tyr Cys Lys Ala Gly Gln Ser Thr
       610                 615                 620
   Glu Glu Glu Met Tyr Asn Asn Glu Ser Ala Gly Pro Ala Phe Glu Glu
   625                 630                 635                 640
   Phe Leu Gln Leu Leu Gly Glu Arg Val Arg Leu Lys Gly Phe Glu Lys
                   645                 650                 655
   Tyr Arg Ala Gln Leu Asp Thr Lys Thr Asp Ser Thr Gly Thr His Ser
               660                 665                 670
   Leu Tyr Thr Thr Tyr Lys Asp Tyr Glu Ile Met Phe His Val Ser Thr
           675                 680                 685
   Met Leu Pro Tyr Thr Pro Asn Asn Lys Gln Gln Leu Leu Arg Lys Arg
       690                 695                 700
   His Ile Gly Asn Asp Ile Val Thr Ile Val Phe Gln Glu Pro Gly Ala
   705                 710                 715                 720
   Gln Pro Phe Ser Pro Lys Asn Ile Arg Ser His Phe Gln His Val Phe
                   725                 730                 735
   Val Ile Val Arg Val His Asn Pro Cys Ser Asp Ser Val Cys Tyr Ser
               740                 745                 750
   Val Ala Val Thr Arg Ser Arg Asp Val Pro Ser Phe Gly Pro Pro Ile
           755                 760                 765
   Pro Lys Gly Val Thr Phe Pro Lys Ser Asn Val Phe Arg Asp Phe Leu
       770                 775                 780
   Leu Ala Lys Val Ile Asn Ala Glu Asn Ala Ala His Lys Ser Glu Lys
   785                 790                 795                 800
   Phe Arg Ala Met Ala Thr Arg Thr Arg Gln Glu Tyr Leu Lys Asp Leu
                   805                 810                 815
   Ala Glu Lys Asn Val Thr Asn Thr Pro Ile Asp Pro Ser Gly Lys Phe
   820 825 830
   Pro Phe Ile Ser Leu Ala Ser Lys Lys Lys Glu Lys Ser Lys Pro Tyr
   835 840 845
   Pro Gly Ala Glu Leu Ser Ser Met Gly Ala Ile Val Trp Ala Val Arg
   850 855 860
   Ala Glu Asp Tyr Asn Lys Ala Met Glu Leu Asp Cys Leu Leu Gly Ile
   865 870 875 880
   Ser Asn Glu Phe Ile Val Leu Ile Glu Gln Glu Thr Lys Ser Val Val
   885 890 895
   Phe Asn Cys Ser Cys Arg Asp Val Ile Gly Trp Thr Ser Thr Asp Thr
   900 905 910
   Ser Leu Lys Ile Phe Tyr Glu Arg Gly Glu Cys Val Ser Val Gly Ser
   915 920 925
   Phe Ile Asn Ile Glu Glu Ile Lys Glu Ile Val Lys Arg Leu Gln Phe
   930 935 940
   Val Ser Lys Gly Cys Glu Ser Val Glu Met Thr Leu Arg Arg Asn Gly
   945 950 955 960
   Leu Gly Gln Leu Gly Phe His Val Asn Tyr Glu Gly Ile Val Ala Asp
   965 970 975
   Val Glu Pro Tyr Gly Tyr Ala Trp Gln Ala Gly Leu Arg Gln Gly Ser
   980 985 990
   Arg Leu Val Glu Ile Cys Lys Val Ala Val Ala Thr Leu Ser His Glu
   995 1000 1005
   Gln Met Ile Asp Leu Leu Arg Thr Ser Val Thr Val Lys Val Val Ile
   1010 1015 1020
   Ile Pro Pro His Asp Asp Cys Thr Pro Arg Arg Ser Cys Ser Glu Thr
   025 1030 1035 1040
   Tyr Arg Met Pro Val Met Glu Tyr Lys Met Asn Glu Gly Val Ser Tyr
   1045 1050 1055
   Glu Phe Lys Phe Pro Phe Arg Asn Asn Asn Lys Trp Gln Arg Asn Ala
   1060 1065 1070
   Ser Lys Gly Pro His Ser Pro Gln Val Pro Ser Gln Val Gln Ser Pro
   1075 1080 1085
   Met Thr Ser Arg Leu Asn Ala Gly Lys Gly Asp Gly Lys Met Pro Pro
   1090 1095 1100
   Pro Glu Arg Ala Ala Asn Ile Pro Arg Ser Ile Ser Ser Asp Gly Arg
   105 1110 1115 1120
   Pro Leu Glu Arg Arg Leu Ser Pro Gly Ser Asp Ile Tyr Val Thr Val
   1125 1130 1135
   Ser Ser Met Ala Leu Ala Arg Ser Gln Cys Arg Asn Ser Pro Ser Asn
   1140 1145 1150
   Leu Ser Ser Ser Ser Asp Thr Gly Ser Val Gly Gly Thr Tyr Arg Gln
   1155 1160 1165
   Lys Ser Met Pro Glu Gly Phe Gly Val Ser Arg Arg Ser Pro Ala Ser
   1170 1175 1180
   Ile Asp Arg Gln Asn Thr Gln Ser Asp Ile Gly Gly Ser Gly Lys Ser
   185 1190 1195 1200
   Thr Pro Ser Trp Gln Arg Ser Glu Asp Ser Ile Ala Asp Gln Met Ala
   1205 1210 1215
   Tyr Ser Tyr Arg Gly Pro Gln Asp Phe Asn Ser Phe Val Leu Glu Gln
   1220 1225 1230
   His Glu Tyr Thr Glu Pro Thr Cys His Leu Pro Ala Val Ser Lys Val
   1235 1240 1245
   Leu Pro Ala Phe Arg Glu Ser Pro Ser Gly Arg Leu Met Arg Gln Asp
   1250 1255 1260
   Pro Val Val His Leu Ser Pro Asn Lys Gln Gly His Ser Asp Ser His
   265 1270 1275 1280
   Tyr Ser Ser His Ser Ser Ser Asn Thr Leu Ser Ser Asn Ala Ser Ser
   1285 1290 1295
   Ala His Ser Asp Glu Lys Trp Tyr Asp Gly Asp Arg Thr Glu Ser Glu
   1300 1305 1310
   Leu Asn Ser Tyr Asn Tyr Leu Gln Gly Thr Ser Ala Asp Ser Gly Ile
   1315 1320 1325
   Asp Thr Thr Ser Tyr Gly Pro Ser His Gly Ser Thr Ala Ser Leu Gly
   1330 1335 1340
   Ala Ala Thr Ser Ser Pro Arg Ser Gly Pro Gly Lys Glu Lys Val Ala
   345 1350 1355 1360
   Pro Leu Trp His Ser Ser Ser Glu Val Ile Ser Met Ala Asp Arg Thr
   1365 1370 1375
   Leu Glu Thr Glu Ser His Gly Leu Asp Arg Lys Thr Glu Ser Ser Leu
   1380 1385 1390
   Ser Leu Asp Ile His Ser Lys Ser Gln Ala Gly Ser Thr Pro Leu Thr
   1395 1400 1405
   Arg Glu Asn Ser Thr Phe Ser Ile Asn Asp Ala Ala Ser His Thr Ser
   1410 1415 1420
   Thr Met Ser Ser Arg His Ser Ala Ser Pro Val Val Phe Thr Ser Ala
   425 1430 1435 1440
   Arg Ser Ser Pro Lys Glu Glu Leu His Pro Ala Ala Pro Ser Gln Leu
   1445 1450 1455
   Ala Pro Ser Phe Ser Ser Ser Ser Ser Ser Ser Ser Gly Pro Arg Ser
   1460 1465 1470
   Phe Tyr Pro Arg Gln Gly Ala Thr Ser Lys Tyr Leu Ile Gly Trp Lys
   1475 1480 1485
   Lys Pro Glu Gly Thr Ile Asn Ser Val Gly Phe Met Asp Thr Arg Lys
   1490 1495 1500
   Arg His Gln Ser Asp Gly Asn Glu Ile Ala His Thr Arg Leu Arg Ala
   505 1510 1515 1520
   Ser Thr Arg Asp Leu Arg Ala Ser Pro Lys Pro Thr Ser Lys Ser Thr
   1525 1530 1535
   Ile Glu Glu Asp Leu Lys Lys Leu Ile Asp Leu Glu Ser Pro Thr Pro
   1540 1545 1550
   Glu Ser Gln Lys Ser Phe Lys Phe His Ala Leu Ser Ser Pro Gln Ser
   1555 1560 1565
   Pro Phe Pro Ser Thr Pro Thr Ser Arg Arg Ala Leu His Arg Thr Leu
   1570 1575 1580
   Ser Asp Glu Ser Ile Tyr Asn Ser Gln Arg Glu His Phe Phe Thr Ser
   585 1590 1595 1600
   Arg Ala Ser Leu Leu Asp Gln Ala Leu Pro Asn Asp Val Leu Phe Ser
   1605 1610 1615
   Ser Thr Tyr Pro Ser Leu Pro Lys Ser Leu Pro Leu Arg Arg Pro Ser
   1620 1625 1630
   Tyr Thr Leu Gly Met Lys Ser Leu His Gly Glu Phe Ser Ala Ser Asp
   1635 1640 1645
   Ser Ser Leu Thr Asp Ile Gln Glu Thr Arg Arg Gln Pro Met Pro Asp
   1650 1655 1660
   Pro Gly Leu Met Pro Leu Pro Asp Thr Ala Ala Asp Leu Asp Trp Ser
   665 1670 1675 1680
   Asn Leu Val Asp Ala Ala Lys Ala Tyr Glu Val Gln Arg Ala Ser Phe
   1685 1690 1695
   Phe Ala Ala Ser Asp Glu Asn His Arg Pro Leu Ser Ala Ala Ser Asn
   1700 1705 1710
   Ser Asp Gln Leu Glu Asp Gln Ala Leu Ala Gln Met Lys Pro Tyr Ser
   1715 1720 1725
   Ser Ser Lys Asp Ser Ser Pro Thr Leu Ala Ser Lys Val Asp Gln Leu
   1730 1735 1740
   Glu Gly Met Leu Lys Met Leu Arg Glu Asp Leu Lys Lys Glu Lys Glu
   745 1750 1755 1760
   Asp Lys Ala His Leu Gln Ala Glu Val Gln His Leu Arg Glu Asp Asn
   1765 1770 1775
   Leu Arg Leu Gln Glu Glu Ser Gln Asn Ala Ser Asp Lys Leu Lys Lys
   1780 1785 1790
   Phe Thr Glu Trp Val Phe Asn Thr Ile Asp Met Ser
   1795 1800
<210> 3
   <211> 5833
   <212> DNA
   <213> Homo sapiens
<220>
   <221> CDS
   <222> (349)..(5697)
<400> 3
   ggtgtggacg ttgtctaaat ttcggtagcc atggcacaag aatataagaa agcatgggat 60
   tatggcaacc acagaatctc agtagtacaa gttccattca gttttttctg aaagaaagcc 120
   ctctgttaaa gtgaagcaaa gaaactgttg tggattataa cgtttagaag ttccaatttt 180
   tcagtgcttt acaaataaag catcatttaa ccttttaaat gaaaaagatt aagatctcat 240
   gcaactgttg tattttctgg aagccattct ccaaaaggga agtgcacatt taaaacacag 300
   atatgatggt ccttgctgca gggatttaag tctacttgct tttacatc atg acc agc  357
                                                        Met Thr Ser
                                                         1
   ttg aaa cgg tca cag aca gaa agg cct ctt gcc act gac agg gcc tct   405
   Leu Lys Arg Ser Gln Thr Glu Arg Pro Leu Ala Thr Asp Arg Ala Ser
       5                   10                  15
   gtt gtt ggc aca gac ggc acc ccc aaa gtc cac act gat gat ttc tac   453
   Val Val Gly Thr Asp Gly Thr Pro Lys Val His Thr Asp Asp Phe Tyr
    20                  25                  30                  35
   atg cgg cgc ttc cgg tcc caa aat ggc agc tta gga tca tca gtt atg   501
   Met Arg Arg Phe Arg Ser Gln Asn Gly Ser Leu Gly Ser Ser Val Met
                   40                  45                  50
   gct cct gta gga ccc ccc cga agt gaa ggt tct cac cat ata acc tca   549
   Ala Pro Val Gly Pro Pro Arg Ser Glu Gly Ser His His Ile Thr Ser
               55                  60                  65
   acc ccc gga gtc cca aaa atg ggg gta agg gca agg att gca gat tgg   597
   Thr Pro Gly Val Pro Lys Met Gly Val Arg Ala Arg Ile Ala Asp Trp
           70                  75                  80
   ccc cca aga aag gaa aac ata aaa gaa tct agc cgt tca agc cag gaa   645
   Pro Pro Arg Lys Glu Asn Ile Lys Glu Ser Ser Arg Ser Ser Gln Glu
       85                  90                  95
   ata gaa acc tca agt tgc ctt gat agc ctg tcc tcc aaa agc agt cct   693
   Ile Glu Thr Ser Ser Cys Leu Asp Ser Leu Ser Ser Lys Ser Ser Pro
   100                 105                 110                 115
   gtg agt cag gga agt tct gtt agc ctc aat tcc aat gac tca gcc atg      741
   Val Ser Gln Gly Ser Ser Val Ser Leu Asn Ser Asn Asp Ser Ala Met
                   120                 125                 130
   ctg aaa agc ata cag aac acg ctg aaa aac aag aca aga ccg tcg gag      789
   Leu Lys Ser Ile Gln Asn Thr Leu Lys Asn Lys Thr Arg Pro Ser Glu
               135                 140                 145
   aac atg gac tcc aga ttt ctc atg cct gaa gcc tac ccc agc tcc ccc      837
   Asn Met Asp Ser Arg Phe Leu Met Pro Glu Ala Tyr Pro Ser Ser Pro
           150                 155                 160
   aga aaa gct ctt cgc aga ata cgc cag cga agc aac agt gat atc acc      885
   Arg Lys Ala Leu Arg Arg Ile Arg Gln Arg Ser Asn Ser Asp Ile Thr
       165                 170                 175
   ata agt gaa ctt gat gtg gat agc ttt gat gaa tgt atc tca cct aca      933
   Ile Ser Glu Leu Asp Val Asp Ser Phe Asp Glu Cys Ile Ser Pro Thr
   180                 185                 190                 195
   tac aag act gga cca tca ctg cac agg gaa tat ggt agc aca tct tca      981
   Tyr Lys Thr Gly Pro Ser Leu His Arg Glu Tyr Gly Ser Thr Ser Ser
                   200                 205                 210
   att gat aaa cag gga aca tct gga gaa agc ttt ttt gat ttg tta aag      1029
   Ile Asp Lys Gln Gly Thr Ser Gly Glu Ser Phe Phe Asp Leu Leu Lys
               215                 220                 225
   ggc tac aaa gat gac aaa tct gat cga ggt cca act cca acc aag ctc      1077
   Gly Tyr Lys Asp Asp Lys Ser Asp Arg Gly Pro Thr Pro Thr Lys Leu
           230                 235                 240
   agt gac ttt ctc att act ggt ggt ggc aag ggt tct ggt ttc tct ttg      1125
   Ser Asp Phe Leu Ile Thr Gly Gly Gly Lys Gly Ser Gly Phe Ser Leu
       245                 250                 255
   gat gta ata gac ggg cct atc tca cag aga gag aac ctc agg ctt ttt      1173
   Asp Val Ile Asp Gly Pro Ile Ser Gln Arg Glu Asn Leu Arg Leu Phe
   260                 265                 270                 275
   aag gaa agg gaa aaa cca ctc aag cga cgt tca aaa tct gaa act gga      1221
   Lys Glu Arg Glu Lys Pro Leu Lys Arg Arg Ser Lys Ser Glu Thr Gly
                   280                 285                 290
   gac tcc tct att ttt cgt aaa ttg cgc aat gcc aaa ggt gaa gaa ctt      1269
   Asp Ser Ser Ile Phe Arg Lys Leu Arg Asn Ala Lys Gly Glu Glu Leu
               295                 300                 305
   ggg aag tca tca gat ctt gaa gat aac cga tca gaa gac tct gtc agg      1317
   Gly Lys Ser Ser Asp Leu Glu Asp Asn Arg Ser Glu Asp Ser Val Arg
           310                 315                 320
   ccc tgg aca tgt cca aag tgc ttt gcc cac tat gat gtc cag agt ata      1365
   Pro Trp Thr Cys Pro Lys Cys Phe Ala His Tyr Asp Val Gln Ser Ile
       325                 330                 335
   tta ttt gat ttg aat gag gca att atg aac agg cac aat gtt att aag     1413
   Leu Phe Asp Leu Asn Glu Ala Ile Met Asn Arg His Asn Val Ile Lys
   340                 345                 350                 355
   agg aga aac acc acc act gga gct tcc gca gct gcc gtg gca tcc ttg     1461
   Arg Arg Asn Thr Thr Thr Gly Ala Ser Ala Ala Ala Val Ala Ser Leu
                   360                 365                 370
   gtc tct gga cct ctg tct cat tca gcc agt ttt agc tcc cca atg ggc     1509
   Val Ser Gly Pro Leu Ser His Ser Ala Ser Phe Ser Ser Pro Met Gly
               375                 380                 385
   agc aca gag gac ctg aat tcc aaa gga agc ctc agc atg gac cag gga     1557
   Ser Thr Glu Asp Leu Asn Ser Lys Gly Ser Leu Ser Met Asp Gln Gly
           390                 395                 400
   gat gat aaa agc aat gag ctt gta atg agc tgt cca tat ttt cgg aat     1605
   Asp Asp Lys Ser Asn Glu Leu Val Met Ser Cys Pro Tyr Phe Arg Asn
       405                 410                 415
   gag ata ggt gga gaa ggg gag agg aaa atc agc ctt tca aaa tca aat     1653
   Glu Ile Gly Gly Glu Gly Glu Arg Lys Ile Ser Leu Ser Lys Ser Asn
   420                 425                 430                 435
   tct ggc tcc ttt agt gga tgt gaa agt gcc tcc ttt gag tct acc ctt     1701
   Ser Gly Ser Phe Ser Gly Cys Glu Ser Ala Ser Phe Glu Ser Thr Leu
                   440                 445                 450
   agt tcc cat tgc aca aat gca gga gtg gca gta ctt gaa gtg ccc aag     1749
   Ser Ser His Cys Thr Asn Ala Gly Val Ala Val Leu Glu Val Pro Lys
               455                 460                 465
   gag aac ttg gtg ttg cac cta gat aga gtg aaa aga tac atc gtg gaa     1797
   Glu Asn Leu Val Leu His Leu Asp Arg Val Lys Arg Tyr Ile Val Glu
           470                 475                 480
   cac gta gat ctg ggt gca tac tat tat aga aaa ttt ttc tac cag aag     1845
   His Val Asp Leu Gly Ala Tyr Tyr Tyr Arg Lys Phe Phe Tyr Gln Lys
       485                 490                 495
   gaa cac tgg aac tat ttt ggg gct gat gag aat ctt ggt cca gtg gct     1893
   Glu His Trp Asn Tyr Phe Gly Ala Asp Glu Asn Leu Gly Pro Val Ala
   500                 505                 510                 515
   gtg agc att cga agg gaa aaa cca gat gaa atg aaa gaa aat gga tct     1941
   Val Ser Ile Arg Arg Glu Lys Pro Asp Glu Met Lys Glu Asn Gly Ser
                   520                 525                 530
   ccg tac aac tac cga ata att ttt aga act agt gag ctc atg aca ctg     1989
   Pro Tyr Asn Tyr Arg Ile Ile Phe Arg Thr Ser Glu Leu Met Thr Leu
               535                 540                 545
   aga ggt tcg gtc ctg gag gac gcc att ccg tcg aca gcc aag cac tcg     2037
   Arg Gly Ser Val Leu Glu Asp Ala Ile Pro Ser Thr Ala Lys His Ser
           550                 555                 560
   aca gcc aga ggc ctg cct ctc aaa gaa gtg ctg gag cac gtg gtt cct     2085
   Thr Ala Arg Gly Leu Pro Leu Lys Glu Val Leu Glu His Val Val Pro
       565                 570                 575
   gag ctc aat gtc cag tgc ctg cgg ttg gcc ttc aac aca ccc aag gtc     2133
   Glu Leu Asn Val Gln Cys Leu Arg Leu Ala Phe Asn Thr Pro Lys Val
   580                 585                 590                 595
   aca gag cag ctc atg aaa ctg gat gaa caa ggg ctg aac tac cag cag     2181
   Thr Glu Gln Leu Met Lys Leu Asp Glu Gln Gly Leu Asn Tyr Gln Gln
                   600                 605                 610
   aaa gta ggc atc atg tac tgc aaa gct gga cag agc act gaa gaa gag     2229
   Lys Val Gly Ile Met Tyr Cys Lys Ala Gly Gln Ser Thr Glu Glu Glu
               615                 620                 625
   atg tac aac aat gag tca gct ggc cca gcc ttt gaa gaa ttc ctt caa     2277
   Met Tyr Asn Asn Glu Ser Ala Gly Pro Ala Phe Glu Glu Phe Leu Gln
           630                 635                 640
   cta ttg gga gag cga gtt cgg ctc aaa gga ttt gag aag tat cga gca     2325
   Leu Leu Gly Glu Arg Val Arg Leu Lys Gly Phe Glu Lys Tyr Arg Ala
       645                 650                 655
   cag ctt gat acc aaa act gac tcc act gga acc cat tct ctg tac aca     2373
   Gln Leu Asp Thr Lys Thr Asp Ser Thr Gly Thr His Ser Leu Tyr Thr
   660                 665                 670                 675
   aca tac aaa gat tat gaa att atg ttc cat gtt tct acc atg ctg cca     2421
   Thr Tyr Lys Asp Tyr Glu Ile Met Phe His Val Ser Thr Met Leu Pro
                   680                 685                 690
   tac aca ccc aac aac aaa caa cag ctc ctg agg aag cgg cac att gga     2469
   Tyr Thr Pro Asn Asn Lys Gln Gln Leu Leu Arg Lys Arg His Ile Gly
               695                 700                 705
   aat gat atc gta aca att gtt ttc caa gag cct gga gca cag cca ttc     2517
   Asn Asp Ile Val Thr Ile Val Phe Gln Glu Pro Gly Ala Gln Pro Phe
           710                 715                 720
   agc cca aaa aac atc cga tcc cac ttc cag cac gtt ttc gtc atc gtc     2565
   Ser Pro Lys Asn Ile Arg Ser His Phe Gln His Val Phe Val Ile Val
       725                 730                 735
   agg gtg cac aat ccg tgc tct gac agt gtc tgt tat agt gtg gct gtt     2613
   Arg Val His Asn Pro Cys Ser Asp Ser Val Cys Tyr Ser Val Ala Val
   740                 745                 750                 755
   acc agg tcc aga gat gtg cct tcc ttt ggg cct ccc att cct aaa ggg     2661
   Thr Arg Ser Arg Asp Val Pro Ser Phe Gly Pro Pro Ile Pro Lys Gly
                   760                 765                 770
   gtc act ttc cct aag tca aat gtg ttc agg gac ttc ctt ttg gcg aaa     2709
   Val Thr Phe Pro Lys Ser Asn Val Phe Arg Asp Phe Leu Leu Ala Lys
               775                 780                 785
   gtg att aat gca gaa aat gct gct cat aaa tcg gag aag ttt cgg gcc     2757
   Val Ile Asn Ala Glu Asn Ala Ala His Lys Ser Glu Lys Phe Arg Ala
           790                 795                 800
   atg gca act cgg acc cgc cag gaa tac ctg aaa gat ctg gca gaa aag     2805
   Met Ala Thr Arg Thr Arg Gln Glu Tyr Leu Lys Asp Leu Ala Glu Lys
       805                 810                 815
   aat gtc acc aac acc cct atc gac cct tct ggc aag ttt ccg ttc atc    2853
   Asn Val Thr Asn Thr Pro Ile Asp Pro Ser Gly Lys Phe Pro Phe Ile
   820                 825                 830                 835
   tct ctg gct tcc aag aag aag gaa aag tct aag cca tat cca gga gcc    2901
   Ser Leu Ala Ser Lys Lys Lys Glu Lys Ser Lys Pro Tyr Pro Gly Ala
                   840                 845                 850
   gag ctc agc agc atg ggg gcc att gta tgg gca gtc cgg gct gaa gac    2949
   Glu Leu Ser Ser Met Gly Ala Ile Val Trp Ala Val Arg Ala Glu Asp
               855                 860                 865
   tac aac aag gcc atg gaa cta gac tgc ctt tta ggg atc tcc aat gag    2997
   Tyr Asn Lys Ala Met Glu Leu Asp Cys Leu Leu Gly Ile Ser Asn Glu
           870                 875                 880
   ttc att gtg ctc att gaa cag gaa aca aag agc gtg gtc ttc aat tgt    3045
   Phe Ile Val Leu Ile Glu Gln Glu Thr Lys Ser Val Val Phe Asn Cys
       885                 890                 895
   tcc tgt aga gat gtg ata ggg tgg act tca act gac acc agc ctc aaa    3093
   Ser Cys Arg Asp Val Ile Gly Trp Thr Ser Thr Asp Thr Ser Leu Lys
   900                 905                 910                 915
   atc ttc tat gaa cga gga gaa tgt gtt tca gtg ggt agt ttt att aac    3141
   Ile Phe Tyr Glu Arg Gly Glu Cys Val Ser Val Gly Ser Phe Ile Asn
                   920                 925                 930
   att gag gag atc aaa gag att gtc aaa agg ttg cag ttt gtt tca aaa    3189
   Ile Glu Glu Ile Lys Glu Ile Val Lys Arg Leu Gln Phe Val Ser Lys
               935                 940                 945
   ggc tgt gaa tcg gtg gag atg act ctg cga aga aat ggg cta gga cag    3237
   Gly Cys Glu Ser Val Glu Met Thr Leu Arg Arg Asn Gly Leu Gly Gln
           950                 955                 960
   ctt ggc ttc cat gtc aac tat gag ggc att gtg gcg gat gtg gag ccc    3285
   Leu Gly Phe His Val Asn Tyr Glu Gly Ile Val Ala Asp Val Glu Pro
       965                 970                 975
   tac ggt tat gcc tgg cag gca ggg ctg agg cag ggc agt cgc ctg gtg    3333
   Tyr Gly Tyr Ala Trp Gln Ala Gly Leu Arg Gln Gly Ser Arg Leu Val
   980                 985                 990                 995
   gag atc tgc aag gtg gcg gta gcc act ctg agc cat gag cag atg atc    3381
   Glu Ile Cys Lys Val Ala Val Ala Thr Leu Ser His Glu Gln Met Ile
                   1000                1005                1010
   gac ctc ctg aga aca tct gtc acg gtg aag gtt gtc atc att ccc ccg    3429
   Asp Leu Leu Arg Thr Ser Val Thr Val Lys Val Val Ile Ile Pro Pro
               1015                1020                1025
   cat gat gac tgc acc ccg cgg agg agt tgc tct gaa acc tac cgc atg    3477
   His Asp Asp Cys Thr Pro Arg Arg Ser Cys Ser Glu Thr Tyr Arg Met
           1030                1035                1040
   cca gtg atg gag tac aaa atg aat gaa ggt gtt tca tac gaa ttc aag    3525
   Pro Val Met Glu Tyr Lys Met Asn Glu Gly Val Ser Tyr Glu Phe Lys
       1045                1050                1055
   ttt ccc ttc cga aat aat aac aag tgg cag agg aac gcc agc aag ggg     3573
   Phe Pro Phe Arg Asn Asn Asn Lys Trp Gln Arg Asn Ala Ser Lys Gly
   1060                1065                1070                1075
   cct cat tca cct caa gtc ccg tcc cag gtg cag agt ccc atg acc tcg     3621
   Pro His Ser Pro Gln Val Pro Ser Gln Val Gln Ser Pro Met Thr Ser
                   1080                1085                1090
   cgg ctg aat gct gga aaa gga gat ggg aag atg cct cct cca gaa aga     3669
   Arg Leu Asn Ala Gly Lys Gly Asp Gly Lys Met Pro Pro Pro Glu Arg
               1095                1100                1105
   gcc gcc aac atc cct cga agc atc tcc agt gac ggg cgc cca cta gag     3717
   Ala Ala Asn Ile Pro Arg Ser Ile Ser Ser Asp Gly Arg Pro Leu Glu
           1110                1115                1120
   agg cgg ctg tct cct ggt tcg gac atc tat gtg acg gtc tca tcc atg     3765
   Arg Arg Leu Ser Pro Gly Ser Asp Ile Tyr Val Thr Val Ser Ser Met
       1125                1130                1135
   gct tta gca aga tcc cag tgt cgg aac tct cct agc aac ttg tct tca     3813
   Ala Leu Ala Arg Ser Gln Cys Arg Asn Ser Pro Ser Asn Leu Ser Ser
   1140                1145                1150                1155
   tcc agt gat act ggt tct gtg ggg ggc act tac agg cag aag tcc atg     3861
   Ser Ser Asp Thr Gly Ser Val Gly Gly Thr Tyr Arg Gln Lys Ser Met
                   1160                1165                1170
   ccc gaa ggg ttt gga gtg agc cgt aga tcc cca gcc tcc att gac agg     3909
   Pro Glu Gly Phe Gly Val Ser Arg Arg Ser Pro Ala Ser Ile Asp Arg
               1175                1180                1185
   cag aac acc cag tca gat att ggt ggc agc gga aaa tcc acg cct agc     3957
   Gln Asn Thr Gln Ser Asp Ile Gly Gly Ser Gly Lys Ser Thr Pro Ser
           1190                1195                1200
   tgg caa aga agt gag gat agc att gct gac cag atg gag cca aca tgc     4005
   Trp Gln Arg Ser Glu Asp Ser Ile Ala Asp Gln Met Glu Pro Thr Cys
       1205                1210                1215
   cat ctc cca gca gta tca aag gta ctg cca gct ttc cga gag agc ccc     4053
   His Leu Pro Ala Val Ser Lys Val Leu Pro Ala Phe Arg Glu Ser Pro
   1220                1225                1230                1235
   agt ggg aga tta atg cgg cag gat cca gtg gtt cat ttg tct cca aac     4101
   Ser Gly Arg Leu Met Arg Gln Asp Pro Val Val His Leu Ser Pro Asn
                   1240                1245                1250
   aaa caa ggg cat tct gat agc cac tac tcg agc cac tcc agt agc aat     4149
   Lys Gln Gly His Ser Asp Ser His Tyr Ser Ser His Ser Ser Ser Asn
               1255                1260                1265
   act ctc tcc agc aat gcg tca agt gcc cat agt gat gag aag tgg tac     4197
   Thr Leu Ser Ser Asn Ala Ser Ser Ala His Ser Asp Glu Lys Trp Tyr
           1270                1275                1280
   gat ggg gac cgc aca gaa tcc gaa ctc aac agc tat aac tat ctg caa 4245
   Asp Gly Asp Arg Thr Glu Ser Glu Leu Asn Ser Tyr Asn Tyr Leu Gln
   1285 1290 1295
   ggc acc tct gct gac agt ggc att gac acc acc tct tat ggc ccc agc 4293
   Gly Thr Ser Ala Asp Ser Gly Ile Asp Thr Thr Ser Tyr Gly Pro Ser
   1300 1305 1310 1315
   cac ggc agc aca gcc tcg ctg ggg gct gcc aca tcg tca cct cgc tca 4341
   His Gly Ser Thr Ala Ser Leu Gly Ala Ala Thr Ser Ser Pro Arg Ser
   1320 1325 1330
   ggg cca ggc aag gag aaa gtg gca ccc cta tgg cac agc tcc agt gaa 4389
   Gly Pro Gly Lys Glu Lys Val Ala Pro Leu Trp His Ser Ser Ser Glu
   1335 1340 1345
   gta atc tcc atg gca gat cgg act ttg gag aca gag agc cac ggc ctg 4437
   Val Ile Ser Met Ala Asp Arg Thr Leu Glu Thr Glu Ser His Gly Leu
   1350 1355 1360
   gac cgg aaa aca gag tct tcc ctg agc tta gac ata cac agc aag agc 4485
   Asp Arg Lys Thr Glu Ser Ser Leu Ser Leu Asp Ile His Ser Lys Ser
   1365 1370 1375
   caa gcc ggc tcg acc cct ctg aca agg gag aac agc acc ttc agt ata 4533
   Gln Ala Gly Ser Thr Pro Leu Thr Arg Glu Asn Ser Thr Phe Ser Ile
   1380 1385 1390 1395
   aac gat gct gct tcc cac aca agt acc atg agc tcc cga cac tct gcc 4581
   Asn Asp Ala Ala Ser His Thr Ser Thr Met Ser Ser Arg His Ser Ala
   1400 1405 1410
   agc cca gtg gtt ttc acc agt gcc cgg agt tca cct aaa gaa gag ctt 4629
   Ser Pro Val Val Phe Thr Ser Ala Arg Ser Ser Pro Lys Glu Glu Leu
   1415 1420 1425
   cat cca gct gcc ccc tca cag ctc gca cca tcc ttc tcc tcc tct tcc 4677
   His Pro Ala Ala Pro Ser Gln Leu Ala Pro Ser Phe Ser Ser Ser Ser
   1430 1435 1440
   tcc tcc tcc tct ggt cct agg agt ttt tac cct cgc cag ggc gct act 4725
   Ser Ser Ser Ser Gly Pro Arg Ser Phe Tyr Pro Arg Gln Gly Ala Thr
   1445 1450 1455
   agc aag tac ctg att gga tgg aaa aaa ccc gaa gga acc ata aac tcc 4773
   Ser Lys Tyr Leu Ile Gly Trp Lys Lys Pro Glu Gly Thr Ile Asn Ser
   1460 1465 1470 1475
   gtg gga ttt atg gac acg aga aag cgt cat cag agc gat ggc aat gaa 4821
   Val Gly Phe Met Asp Thr Arg Lys Arg His Gln Ser Asp Gly Asn Glu
   1480 1485 1490
   ata gcc cac acc agg ctg cgt gcc tca acc aga gac ctc cgg gca tct 4869
   Ile Ala His Thr Arg Leu Arg Ala Ser Thr Arg Asp Leu Arg Ala Ser
   1495 1500 1505
   cct aag cca acc tcc aag tcc acc att gaa gaa gat cta aag aaa cta 4917
   Pro Lys Pro Thr Ser Lys Ser Thr Ile Glu Glu Asp Leu Lys Lys Leu
   1510 1515 1520
   att gat ctt gaa agc cca act cct gaa tca cag aag agt ttt aag ttc     4965
   Ile Asp Leu Glu Ser Pro Thr Pro Glu Ser Gln Lys Ser Phe Lys Phe
       1525                1530                1535
   cac gca ctc tcc tct cct cag tct cct ttc ccc agc acc ccc acc tca     5013
   His Ala Leu Ser Ser Pro Gln Ser Pro Phe Pro Ser Thr Pro Thr Ser
   1540                1545                1550                1555
   cgg cgg gcc ttg cac aga aca ctg tcg gac gag agc att tac aat agc     5061
   Arg Arg Ala Leu His Arg Thr Leu Ser Asp Glu Ser Ile Tyr Asn Ser
                   1560                1565                1570
   cag agg gag cac ttt ttc acc tcc agg gcg tca ctt ctg gac caa gcc     5109
   Gln Arg Glu His Phe Phe Thr Ser Arg Ala Ser Leu Leu Asp Gln Ala
               1575                1580                1585
   ctg ccc aac gac gtc ctc ttc agt agc acg tac cct tct ctc ccc aag     5157
   Leu Pro Asn Asp Val Leu Phe Ser Ser Thr Tyr Pro Ser Leu Pro Lys
           1590                1595                1600
   tcg ctc ccg ttg agg agg cct tct tac acc tta gga atg aaa tcg ctg     5205
   Ser Leu Pro Leu Arg Arg Pro Ser Tyr Thr Leu Gly Met Lys Ser Leu
       1605                1610                1615
   cat gga gag ttc tca gcc tcg gac agc tcc ctc act gac atc cag gag     5253
   His Gly Glu Phe Ser Ala Ser Asp Ser Ser Leu Thr Asp Ile Gln Glu
   1620                1625                1630                1635
   acc cgc agg cag cct atg ccc gac cct ggc ctg atg ccc ctg cct gac     5301
   Thr Arg Arg Gln Pro Met Pro Asp Pro Gly Leu Met Pro Leu Pro Asp
                   1640                1645                1650
   act gct gca gac ttg gat tgg tcc aac ctg gta gat gct gcc aaa gcc     5349
   Thr Ala Ala Asp Leu Asp Trp Ser Asn Leu Val Asp Ala Ala Lys Ala
               1655                1660                1665
   tat gag gtc cag aga gcc tca ttt ttt gct gct agt gat gaa aac cat     5397
   Tyr Glu Val Gln Arg Ala Ser Phe Phe Ala Ala Ser Asp Glu Asn His
           1670                1675                1680
   cgc ccc ttg agt gct gca tcc aac agt gat cag ctg gag gac cag gct     5445
   Arg Pro Leu Ser Ala Ala Ser Asn Ser Asp Gln Leu Glu Asp Gln Ala
       1685                1690                1695
   ctg gcc cag atg aag cct tac agc agc agt aaa gac tcc tct ccc act     5493
   Leu Ala Gln Met Lys Pro Tyr Ser Ser Ser Lys Asp Ser Ser Pro Thr
   1700                1705                1710                1715
   ctg gct tct aaa gtg gac cag ctg gaa ggt atg ctg aag atg ctt cgg     5541
   Leu Ala Ser Lys Val Asp Gln Leu Glu Gly Met Leu Lys Met Leu Arg
                   1720                1725                1730
   gaa gat ttg aag aag gaa aaa gaa gac aaa gct cac ctt cag gcg gag     5589
   Glu Asp Leu Lys Lys Glu Lys Glu Asp Lys Ala His Leu Gln Ala Glu
               1735                1740                1745
   gtg cag cac ctg cga gag gac aac ctg agg cta cag gag gag tcc cag     5637
   Val Gln His Leu Arg Glu Asp Asn Leu Arg Leu Gln Glu Glu Ser Gln
   1750 1755 1760
   aac gcc tcg gac aag ctg aag aag ttc aca gaa tgg gtc ttc aac acc 5685
   Asn Ala Ser Asp Lys Leu Lys Lys Phe Thr Glu Trp Val Phe Asn Thr
   1765 1770 1775
   ata gac atg agc tagggaaggc tgaggaggac aggagaaggg cccagacact 5737
   Ile Asp Met Ser
   1780
   ccctccagtg agtgtcctgc agcccttatt ccctccatag aaagcatcct cagagcacct 5797
   tccctggctt cctactctgc cccctttcgg ggagtg 5833
<210> 4
   <211> 1783
   <212> PRT
   <213> Homo sapiens
<400> 4
   Met Thr Ser Leu Lys Arg Ser Gln Thr Glu Arg Pro Leu Ala Thr Asp
    1               5                  10                  15
   Arg Ala Ser Val Val Gly Thr Asp Gly Thr Pro Lys Val His Thr Asp
               20                  25                  30
   Asp Phe Tyr Met Arg Arg Phe Arg Ser Gln Asn Gly Ser Leu Gly Ser
           35                  40                  45
   Ser Val Met Ala Pro Val Gly Pro Pro Arg Ser Glu Gly Ser His His
       50                  55                  60
   Ile Thr Ser Thr Pro Gly Val Pro Lys Met Gly Val Arg Ala Arg Ile
   65                  70                  75                  80
   Ala Asp Trp Pro Pro Arg Lys Glu Asn Ile Lys Glu Ser Ser Arg Ser
                   85                  90                  95
   Ser Gln Glu Ile Glu Thr Ser Ser Cys Leu Asp Ser Leu Ser Ser Lys
               100                 105                 110
   Ser Ser Pro Val Ser Gln Gly Ser Ser Val Ser Leu Asn Ser Asn Asp
           115                 120                 125
   Ser Ala Met Leu Lys Ser Ile Gln Asn Thr Leu Lys Asn Lys Thr Arg
       130                 135                 140
   Pro Ser Glu Asn Met Asp Ser Arg Phe Leu Met Pro Glu Ala Tyr Pro
   145                 150                 155                 160
   Ser Ser Pro Arg Lys Ala Leu Arg Arg Ile Arg Gln Arg Ser Asn Ser
                   165                 170                 175
   Asp Ile Thr Ile Ser Glu Leu Asp Val Asp Ser Phe Asp Glu Cys Ile
               180                 185                 190
   Ser Pro Thr Tyr Lys Thr Gly Pro Ser Leu His Arg Glu Tyr Gly Ser
           195                 200                 205
   Thr Ser Ser Ile Asp Lys Gln Gly Thr Ser Gly Glu Ser Phe Phe Asp
       210                 215                 220
   Leu Leu Lys Gly Tyr Lys Asp Asp Lys Ser Asp Arg Gly Pro Thr Pro
   225                 230                 235                 240
   Thr Lys Leu Ser Asp Phe Leu Ile Thr Gly Gly Gly Lys Gly Ser Gly
                   245                 250                 255
   Phe Ser Leu Asp Val Ile Asp Gly Pro Ile Ser Gln Arg Glu Asn Leu
               260                 265                 270
   Arg Leu Phe Lys Glu Arg Glu Lys Pro Leu Lys Arg Arg Ser Lys Ser
           275                 280                 285
   Glu Thr Gly Asp Ser Ser Ile Phe Arg Lys Leu Arg Asn Ala Lys Gly
       290                 295                 300
   Glu Glu Leu Gly Lys Ser Ser Asp Leu Glu Asp Asn Arg Ser Glu Asp
   305                 310                 315                 320
   Ser Val Arg Pro Trp Thr Cys Pro Lys Cys Phe Ala His Tyr Asp Val
                   325                 330                 335
   Gln Ser Ile Leu Phe Asp Leu Asn Glu Ala Ile Met Asn Arg His Asn
               340                 345                 350
   Val Ile Lys Arg Arg Asn Thr Thr Thr Gly Ala Ser Ala Ala Ala Val
           355                 360                 365
   Ala Ser Leu Val Ser Gly Pro Leu Ser His Ser Ala Ser Phe Ser Ser
       370                 375                 380
   Pro Met Gly Ser Thr Glu Asp Leu Asn Ser Lys Gly Ser Leu Ser Met
   385                 390                 395                 400
   Asp Gln Gly Asp Asp Lys Ser Asn Glu Leu Val Met Ser Cys Pro Tyr
                   405                 410                 415
   Phe Arg Asn Glu Ile Gly Gly Glu Gly Glu Arg Lys Ile Ser Leu Ser
               420                 425                 430
   Lys Ser Asn Ser Gly Ser Phe Ser Gly Cys Glu Ser Ala Ser Phe Glu
           435                 440                 445
   Ser Thr Leu Ser Ser His Cys Thr Asn Ala Gly Val Ala Val Leu Glu
       450                 455                 460
   Val Pro Lys Glu Asn Leu Val Leu His Leu Asp Arg Val Lys Arg Tyr
   465                 470                 475                 480
   Ile Val Glu His Val Asp Leu Gly Ala Tyr Tyr Tyr Arg Lys Phe Phe
                   485                 490                 495
   Tyr Gln Lys Glu His Trp Asn Tyr Phe Gly Ala Asp Glu Asn Leu Gly
               500                 505                 510
   Pro Val Ala Val Ser Ile Arg Arg Glu Lys Pro Asp Glu Met Lys Glu
           515                 520                 525
   Asn Gly Ser Pro Tyr Asn Tyr Arg Ile Ile Phe Arg Thr Ser Glu Leu
       530                 535                 540
   Met Thr Leu Arg Gly Ser Val Leu Glu Asp Ala Ile Pro Ser Thr Ala
   545                 550                 555                 560
   Lys His Ser Thr Ala Arg Gly Leu Pro Leu Lys Glu Val Leu Glu His
                   565                 570                 575
   Val Val Pro Glu Leu Asn Val Gln Cys Leu Arg Leu Ala Phe Asn Thr
               580                 585                 590
   Pro Lys Val Thr Glu Gln Leu Met Lys Leu Asp Glu Gln Gly Leu Asn
           595                 600                 605
   Tyr Gln Gln Lys Val Gly Ile Met Tyr Cys Lys Ala Gly Gln Ser Thr
       610                 615                 620
   Glu Glu Glu Met Tyr Asn Asn Glu Ser Ala Gly Pro Ala Phe Glu Glu
   625                 630                 635                 640
   Phe Leu Gln Leu Leu Gly Glu Arg Val Arg Leu Lys Gly Phe Glu Lys
                   645                 650                 655
   Tyr Arg Ala Gln Leu Asp Thr Lys Thr Asp Ser Thr Gly Thr His Ser
               660                 665                 670
   Leu Tyr Thr Thr Tyr Lys Asp Tyr Glu Ile Met Phe His Val Ser Thr
           675                 680                 685
   Met Leu Pro Tyr Thr Pro Asn Asn Lys Gln Gln Leu Leu Arg Lys Arg
       690                 695                 700
   His Ile Gly Asn Asp Ile Val Thr Ile Val Phe Gln Glu Pro Gly Ala
   705                 710                 715                 720
   Gln Pro Phe Ser Pro Lys Asn Ile Arg Ser His Phe Gln His Val Phe
                   725                 730                 735
   Val Ile Val Arg Val His Asn Pro Cys Ser Asp Ser Val Cys Tyr Ser
               740                 745                 750
   Val Ala Val Thr Arg Ser Arg Asp Val Pro Ser Phe Gly Pro Pro Ile
           755                 760                 765
   Pro Lys Gly Val Thr Phe Pro Lys Ser Asn Val Phe Arg Asp Phe Leu
       770                 775                 780
   Leu Ala Lys Val Ile Asn Ala Glu Asn Ala Ala His Lys Ser Glu Lys
   785                 790                 795                 800
   Phe Arg Ala Met Ala Thr Arg Thr Arg Gln Glu Tyr Leu Lys Asp Leu
                   805                 810                 815
   Ala Glu Lys Asn Val Thr Asn Thr Pro Ile Asp Pro Ser Gly Lys Phe
               820                 825                 830
   Pro Phe Ile Ser Leu Ala Ser Lys Lys Lys Glu Lys Ser Lys Pro Tyr
   835 840 845
   Pro Gly Ala Glu Leu Ser Ser Met Gly Ala Ile Val Trp Ala Val Arg
   850 855 860
   Ala Glu Asp Tyr Asn Lys Ala Met Glu Leu Asp Cys Leu Leu Gly Ile
   865 870 875 880
   Ser Asn Glu Phe Ile Val Leu Ile Glu Gln Glu Thr Lys Ser Val Val
   885 890 895
   Phe Asn Cys Ser Cys Arg Asp Val Ile Gly Trp Thr Ser Thr Asp Thr
   900 905 910
   Ser Leu Lys Ile Phe Tyr Glu Arg Gly Glu Cys Val Ser Val Gly Ser
   915 920 925
   Phe Ile Asn Ile Glu Glu Ile Lys Glu Ile Val Lys Arg Leu Gln Phe
   930 935 940
   Val Ser Lys Gly Cys Glu Ser Val Glu Met Thr Leu Arg Arg Asn Gly
   945 950 955 960
   Leu Gly Gln Leu Gly Phe His Val Asn Tyr Glu Gly Ile Val Ala Asp
   965 970 975
   Val Glu Pro Tyr Gly Tyr Ala Trp Gln Ala Gly Leu Arg Gln Gly Ser
   980 985 990
   Arg Leu Val Glu Ile Cys Lys Val Ala Val Ala Thr Leu Ser His Glu
   995 1000 1005
   Gln Met Ile Asp Leu Leu Arg Thr Ser Val Thr Val Lys Val Val Ile
   1010 1015 1020
   Ile Pro Pro His Asp Asp Cys Thr Pro Arg Arg Ser Cys Ser Glu Thr
   025 1030 1035 1040
   Tyr Arg Met Pro Val Met Glu Tyr Lys Met Asn Glu Gly Val Ser Tyr
   1045 1050 1055
   Glu Phe Lys Phe Pro Phe Arg Asn Asn Asn Lys Trp Gln Arg Asn Ala
   1060 1065 1070
   Ser Lys Gly Pro His Ser Pro Gln Val Pro Ser Gln Val Gln Ser Pro
   1075 1080 1085
   Met Thr Ser Arg Leu Asn Ala Gly Lys Gly Asp Gly Lys Met Pro Pro
   1090 1095 1100
   Pro Glu Arg Ala Ala Asn Ile Pro Arg Ser Ile Ser Ser Asp Gly Arg
   105 1110 1115 1120
   Pro Leu Glu Arg Arg Leu Ser Pro Gly Ser Asp Ile Tyr Val Thr Val
   1125 1130 1135
   Ser Ser Met Ala Leu Ala Arg Ser Gln Cys Arg Asn Ser Pro Ser Asn
   1140 1145 1150
   Leu Ser Ser Ser Ser Asp Thr Gly Ser Val Gly Gly Thr Tyr Arg Gln
   1155 1160 1165
   Lys Ser Met Pro Glu Gly Phe Gly Val Ser Arg Arg Ser Pro Ala Ser
   1170 1175 1180
   Ile Asp Arg Gln Asn Thr Gln Ser Asp Ile Gly Gly Ser Gly Lys Ser
   185 1190 1195 1200
   Thr Pro Ser Trp Gln Arg Ser Glu Asp Ser Ile Ala Asp Gln Met Glu
   1205 1210 1215
   Pro Thr Cys His Leu Pro Ala Val Ser Lys Val Leu Pro Ala Phe Arg
   1220 1225 1230
   Glu Ser Pro Ser Gly Arg Leu Met Arg Gln Asp Pro Val Val His Leu
   1235 1240 1245
   Ser Pro Asn Lys Gln Gly His Ser Asp Ser His Tyr Ser Ser His Ser
   1250 1255 1260
   Ser Ser Asn Thr Leu Ser Ser Asn Ala Ser Ser Ala His Ser Asp Glu
   265 1270 1275 1280
   Lys Trp Tyr Asp Gly Asp Arg Thr Glu Ser Glu Leu Asn Ser Tyr Asn
   1285 1290 1295
   Tyr Leu Gln Gly Thr Ser Ala Asp Ser Gly Ile Asp Thr Thr Ser Tyr
   1300 1305 1310
   Gly Pro Ser His Gly Ser Thr Ala Ser Leu Gly Ala Ala Thr Ser Ser
   1315 1320 1325
   Pro Arg Ser Gly Pro Gly Lys Glu Lys Val Ala Pro Leu Trp His Ser
   1330 1335 1340
   Ser Ser Glu Val Ile Ser Met Ala Asp Arg Thr Leu Glu Thr Glu Ser
   345 1350 1355 1360
   His Gly Leu Asp Arg Lys Thr Glu Ser Ser Leu Ser Leu Asp Ile His
   1365 1370 1375
   Ser Lys Ser Gln Ala Gly Ser Thr Pro Leu Thr Arg Glu Asn Ser Thr
   1380 1385 1390
   Phe Ser Ile Asn Asp Ala Ala Ser His Thr Ser Thr Met Ser Ser Arg
   1395 1400 1405
   His Ser Ala Ser Pro Val Val Phe Thr Ser Ala Arg Ser Ser Pro Lys
   1410 1415 1420
   Glu Glu Leu His Pro Ala Ala Pro Ser Gln Leu Ala Pro Ser Phe Ser
   425 1430 1435 1440
   Ser Ser Ser Ser Ser Ser Ser Gly Pro Arg Ser Phe Tyr Pro Arg Gln
   1445 1450 1455
   Gly Ala Thr Ser Lys Tyr Leu Ile Gly Trp Lys Lys Pro Glu Gly Thr
   1460 1465 1470
   Ile Asn Ser Val Gly Phe Met Asp Thr Arg Lys Arg His Gln Ser Asp
   1475 1480 1485
   Gly Asn Glu Ile Ala His Thr Arg Leu Arg Ala Ser Thr Arg Asp Leu
   1490 1495 1500
   Arg Ala Ser Pro Lys Pro Thr Ser Lys Ser Thr Ile Glu Glu Asp Leu
   505 1510 1515 1520
   Lys Lys Leu Ile Asp Leu Glu Ser Pro Thr Pro Glu Ser Gln Lys Ser
   1525 1530 1535
   Phe Lys Phe His Ala Leu Ser Ser Pro Gln Ser Pro Phe Pro Ser Thr
   1540 1545 1550
   Pro Thr Ser Arg Arg Ala Leu His Arg Thr Leu Ser Asp Glu Ser Ile
   1555 1560 1565
   Tyr Asn Ser Gln Arg Glu His Phe Phe Thr Ser Arg Ala Ser Leu Leu
   1570 1575 1580
   Asp Gln Ala Leu Pro Asn Asp Val Leu Phe Ser Ser Thr Tyr Pro Ser
   585 1590 1595 1600
   Leu Pro Lys Ser Leu Pro Leu Arg Arg Pro Ser Tyr Thr Leu Gly Met
   1605 1610 1615
   Lys Ser Leu His Gly Glu Phe Ser Ala Ser Asp Ser Ser Leu Thr Asp
   1620 1625 1630
   Ile Gln Glu Thr Arg Arg Gln Pro Met Pro Asp Pro Gly Leu Met Pro
   1635 1640 1645
   Leu Pro Asp Thr Ala Ala Asp Leu Asp Trp Ser Asn Leu Val Asp Ala
   1650 1655 1660
   Ala Lys Ala Tyr Glu Val Gln Arg Ala Ser Phe Phe Ala Ala Ser Asp
   665 1670 1675 1680
   Glu Asn His Arg Pro Leu Ser Ala Ala Ser Asn Ser Asp Gln Leu Glu
   1685 1690 1695
   Asp Gln Ala Leu Ala Gln Met Lys Pro Tyr Ser Ser Ser Lys Asp Ser
   1700 1705 1710
   Ser Pro Thr Leu Ala Ser Lys Val Asp Gln Leu Glu Gly Met Leu Lys
   1715 1720 1725
   Met Leu Arg Glu Asp Leu Lys Lys Glu Lys Glu Asp Lys Ala His Leu
   1730 1735 1740
   Gln Ala Glu Val Gln His Leu Arg Glu Asp Asn Leu Arg Leu Gln Glu
   745 1750 1755 1760
   Glu Ser Gln Asn Ala Ser Asp Lys Leu Lys Lys Phe Thr Glu Trp Val
   1765 1770 1775
   Phe Asn Thr Ile Asp Met Ser
   1780
<210> 5
   <211> 5412
   <212> DNA
   <213> Homo sapiens
<220>
   <221> CDS
   <222> (1)..(5412)
<400> 5
   atg acc agc ttg aaa cgg tca cag aca gaa agg cct ctt gcc act gac    48
   Met Thr Ser Leu Lys Arg Ser Gln Thr Glu Arg Pro Leu Ala Thr Asp
    1               5                  10                  15
   agg gcc tct gtt gtt ggc aca gac ggc acc ccc aaa gtc cac act gat    96
   Arg Ala Ser Val Val Gly Thr Asp Gly Thr Pro Lys Val His Thr Asp
               20                  25                  30
   gat ttc tac atg cgg cgc ttc cgg tcc caa aat ggc agc tta gga tca   144
   Asp Phe Tyr Met Arg Arg Phe Arg Ser Gln Asn Gly Ser Leu Gly Ser
           35                  40                  45
   tca gtt atg gct cct gta gga ccc ccc cga agt gaa ggt tct cac cat   192
   Ser Val Met Ala Pro Val Gly Pro Pro Arg Ser Glu Gly Ser His His
       50                  55                  60
   ata acc tca acc ccc gga gtc cca aaa atg ggg gta agg gca agg att   240
   Ile Thr Ser Thr Pro Gly Val Pro Lys Met Gly Val Arg Ala Arg Ile
   65                  70                  75                  80
   gca gat tgg ccc cca aga aag gaa aac ata aaa gaa tct agc cgt tca   288
   Ala Asp Trp Pro Pro Arg Lys Glu Asn Ile Lys Glu Ser Ser Arg Ser
                   85                  90                  95
   agc cag gaa ata gaa acc tca agt tgc ctt gat agc ctg tcc tcc aaa   336
   Ser Gln Glu Ile Glu Thr Ser Ser Cys Leu Asp Ser Leu Ser Ser Lys
               100                 105                 110
   agc agt cct gtg agt cag gga agt tct gtt agc ctc aat tcc aat gac   384
   Ser Ser Pro Val Ser Gln Gly Ser Ser Val Ser Leu Asn Ser Asn Asp
           115                 120                 125
   tca gcc atg ctg aaa agc ata cag aac acg ctg aaa aac aag aca aga   432
   Ser Ala Met Leu Lys Ser Ile Gln Asn Thr Leu Lys Asn Lys Thr Arg
       130                 135                 140
   ccg tcg gag aac atg gac tcc aga ttt ctc atg cct gaa gcc tac ccc   480
   Pro Ser Glu Asn Met Asp Ser Arg Phe Leu Met Pro Glu Ala Tyr Pro
   145                 150                 155                 160
   agc tcc ccc aga aaa gct ctt cgc aga ata cgc cag cga agc aac agt   528
   Ser Ser Pro Arg Lys Ala Leu Arg Arg Ile Arg Gln Arg Ser Asn Ser
                   165                 170                 175
   gat atc acc ata agt gaa ctt gat gtg gat agc ttt gat gaa tgt atc    576
   Asp Ile Thr Ile Ser Glu Leu Asp Val Asp Ser Phe Asp Glu Cys Ile
               180                 185                 190
   tca cct aca tac aag act gga cca tca ctg cac agg gaa tat ggt agc    624
   Ser Pro Thr Tyr Lys Thr Gly Pro Ser Leu His Arg Glu Tyr Gly Ser
           195                 200                 205
   aca tct tca att gat aaa cag gga aca tct gga gaa agc ttt ttt gat    672
   Thr Ser Ser Ile Asp Lys Gln Gly Thr Ser Gly Glu Ser Phe Phe Asp
       210                 215                 220
   ttg tta aag ggc tac aaa gat gac aaa tct gat cga ggt cca act cca    720
   Leu Leu Lys Gly Tyr Lys Asp Asp Lys Ser Asp Arg Gly Pro Thr Pro
   225                 230                 235                 240
   acc aag ctc agt gac ttt ctc att act ggt ggt ggc aag ggt tct ggt    768
   Thr Lys Leu Ser Asp Phe Leu Ile Thr Gly Gly Gly Lys Gly Ser Gly
                   245                 250                 255
   ttc tct ttg gat gta ata gac ggg cct atc tca cag aga gag aac ctc    816
   Phe Ser Leu Asp Val Ile Asp Gly Pro Ile Ser Gln Arg Glu Asn Leu
               260                 265                 270
   agg ctt ttt aag gaa agg gaa aaa cca ctc aag cga cgt tca aaa tct    864
   Arg Leu Phe Lys Glu Arg Glu Lys Pro Leu Lys Arg Arg Ser Lys Ser
           275                 280                 285
   gaa act gga gac tcc tct att ttt cgt aaa ttg cgc aat gcc aaa ggt    912
   Glu Thr Gly Asp Ser Ser Ile Phe Arg Lys Leu Arg Asn Ala Lys Gly
       290                 295                 300
   gaa gaa ctt ggg aag tca tca gat ctt gaa gat aac cga tca gaa gac    960
   Glu Glu Leu Gly Lys Ser Ser Asp Leu Glu Asp Asn Arg Ser Glu Asp
   305                 310                 315                 320
   tct gtc agg ccc tgg aca tgt cca aag tgc ttt gcc cac tat gat gtc    1008
   Ser Val Arg Pro Trp Thr Cys Pro Lys Cys Phe Ala His Tyr Asp Val
                   325                 330                 335
   cag agt ata tta ttt gat ttg aat gag gca att atg aac agg cac aat    1056
   Gln Ser Ile Leu Phe Asp Leu Asn Glu Ala Ile Met Asn Arg His Asn
               340                 345                 350
   gtt att aag agg aga aac acc acc act gga gct tcc gca gct gcc gtg    1104
   Val Ile Lys Arg Arg Asn Thr Thr Thr Gly Ala Ser Ala Ala Ala Val
           355                 360                 365
   gca tcc ttg gtc tct gga cct ctg tct cat tca gcc agt ttt agc tcc    1152
   Ala Ser Leu Val Ser Gly Pro Leu Ser His Ser Ala Ser Phe Ser Ser
       370                 375                 380
   cca atg ggc agc aca gag gac ctg aat tcc aaa gga agc ctc agc atg    1200
   Pro Met Gly Ser Thr Glu Asp Leu Asn Ser Lys Gly Ser Leu Ser Met
   385                 390                 395                 400
   gac cag gga gat gat aaa agc aat gag ctt gta atg agc tgt cca tat    1248
   Asp Gln Gly Asp Asp Lys Ser Asn Glu Leu Val Met Ser Cys Pro Tyr
                   405                 410                 415
   ttt cgg aat gag ata ggt gga gaa ggg gag agg aaa atc agc ctt tca      1296
   Phe Arg Asn Glu Ile Gly Gly Glu Gly Glu Arg Lys Ile Ser Leu Ser
               420                 425                 430
   aaa tca aat tct ggc tcc ttt agt gga tgt gaa agt gcc tcc ttt gag      1344
   Lys Ser Asn Ser Gly Ser Phe Ser Gly Cys Glu Ser Ala Ser Phe Glu
           435                 440                 445
   tct acc ctt agt tcc cat tgc aca aat gca gga gtg gca gta ctt gaa      1392
   Ser Thr Leu Ser Ser His Cys Thr Asn Ala Gly Val Ala Val Leu Glu
       450                 455                 460
   gtg ccc aag gag aac ttg gtg ttg cac cta gat aga gtg aaa aga tac      1440
   Val Pro Lys Glu Asn Leu Val Leu His Leu Asp Arg Val Lys Arg Tyr
   465                 470                 475                 480
   atc gtg gaa cac gta gat ctg ggt gca tac tat tat aga aaa ttt ttc      1488
   Ile Val Glu His Val Asp Leu Gly Ala Tyr Tyr Tyr Arg Lys Phe Phe
                   485                 490                 495
   tac cag aag gaa cac tgg aac tat ttt ggg gct gat gag aat ctt ggt      1536
   Tyr Gln Lys Glu His Trp Asn Tyr Phe Gly Ala Asp Glu Asn Leu Gly
               500                 505                 510
   cca gtg gct gtg agc att cga agg gaa aaa cca gat gaa atg aaa gaa      1584
   Pro Val Ala Val Ser Ile Arg Arg Glu Lys Pro Asp Glu Met Lys Glu
           515                 520                 525
   aat gga tct ccg tac aac tac cga ata att ttt aga act agt gag ctc      1632
   Asn Gly Ser Pro Tyr Asn Tyr Arg Ile Ile Phe Arg Thr Ser Glu Leu
       530                 535                 540
   atg aca ctg aga ggt tcg gtc ctg gag gac gcc att ccg tcg aca gcc      1680
   Met Thr Leu Arg Gly Ser Val Leu Glu Asp Ala Ile Pro Ser Thr Ala
   545                 550                 555                 560
   aag cac tcg aca gcc aga ggc ctg cct ctc aaa gaa gtg ctg gag cac      1728
   Lys His Ser Thr Ala Arg Gly Leu Pro Leu Lys Glu Val Leu Glu His
                   565                 570                 575
   gtg gtt cct gag ctc aat gtc cag tgc ctg cgg ttg gcc ttc aac aca      1776
   Val Val Pro Glu Leu Asn Val Gln Cys Leu Arg Leu Ala Phe Asn Thr
               580                 585                 590
   ccc aag gtc aca gag cag ctc atg aaa ctg gat gaa caa ggg ctg aac      1824
   Pro Lys Val Thr Glu Gln Leu Met Lys Leu Asp Glu Gln Gly Leu Asn
           595                 600                 605
   tac cag cag aaa gta ggc atc atg tac tgc aaa gct gga cag agc act      1872
   Tyr Gln Gln Lys Val Gly Ile Met Tyr Cys Lys Ala Gly Gln Ser Thr
       610                 615                 620
   gaa gaa gag atg tac aac aat gag tca gct ggc cca gcc ttt gaa gaa      1920
   Glu Glu Glu Met Tyr Asn Asn Glu Ser Ala Gly Pro Ala Phe Glu Glu
   625                 630                 635                 640
   ttc ctt caa cta ttg gga gag cga gtt cgg ctc aaa gga ttt gag aag      1968
   Phe Leu Gln Leu Leu Gly Glu Arg Val Arg Leu Lys Gly Phe Glu Lys
                       645                 650                 655
   tat cga gca cag ctt gat acc aaa act gac tcc act gga acc cat tct     2016
   Tyr Arg Ala Gln Leu Asp Thr Lys Thr Asp Ser Thr Gly Thr His Ser
                   660                 665                 670
   ctg tac aca aca tac aaa gat tat gaa att atg ttc cat gtt tct acc     2064
   Leu Tyr Thr Thr Tyr Lys Asp Tyr Glu Ile Met Phe His Val Ser Thr
               675                 680                 685
   atg ctg cca tac aca ccc aac aac aaa caa cag ctc ctg agg aag cgg     2112
   Met Leu Pro Tyr Thr Pro Asn Asn Lys Gln Gln Leu Leu Arg Lys Arg
       690                 695                 700
   cac att gga aat gat atc gta aca att gtt ttc caa gag cct gga gca     2160
   His Ile Gly Asn Asp Ile Val Thr Ile Val Phe Gln Glu Pro Gly Ala
   705                 710                 715                 720
   cag cca ttc agc cca aaa aac atc cga tcc cac ttc cag cac gtt ttc     2208
   Gln Pro Phe Ser Pro Lys Asn Ile Arg Ser His Phe Gln His Val Phe
                   725                 730                 735
   gtc atc gtc agg gtg cac aat ccg tgc tct gac agt gtc tgt tat agt     2256
   Val Ile Val Arg Val His Asn Pro Cys Ser Asp Ser Val Cys Tyr Ser
               740                 745                 750
   gtg gct gtt acc agg tcc aga gat gtg cct tcc ttt ggg cct ccc att     2304
   Val Ala Val Thr Arg Ser Arg Asp Val Pro Ser Phe Gly Pro Pro Ile
           755                 760                 765
   cct aaa ggg gtc act ttc cct aag tca aat gtg ttc agg gac ttc ctt     2352
   Pro Lys Gly Val Thr Phe Pro Lys Ser Asn Val Phe Arg Asp Phe Leu
       770                 775                 780
   ttg gcg aaa gtg att aat gca gaa aat gct gct cat aaa tcg gag aag     2400
   Leu Ala Lys Val Ile Asn Ala Glu Asn Ala Ala His Lys Ser Glu Lys
   785                 790                 795                 800
   ttt cgg gcc atg gca act cgg acc cgc cag gaa tac ctg aaa gat ctg     2448
   Phe Arg Ala Met Ala Thr Arg Thr Arg Gln Glu Tyr Leu Lys Asp Leu
                   805                 810                 815
   gca gaa aag aat gtc acc aac acc cct atc gac cct tct ggc aag ttt     2496
   Ala Glu Lys Asn Val Thr Asn Thr Pro Ile Asp Pro Ser Gly Lys Phe
               820                 825                 830
   ccg ttc atc tct ctg gct tcc aag aag aag gaa aag tct aag cca tat     2544
   Pro Phe Ile Ser Leu Ala Ser Lys Lys Lys Glu Lys Ser Lys Pro Tyr
           835                 840                 845
   cca gga gcc gag ctc agc agc atg ggg gcc att gta tgg gca gtc cgg     2592
   Pro Gly Ala Glu Leu Ser Ser Met Gly Ala Ile Val Trp Ala Val Arg
       850                 855                 860
   gct gaa gac tac aac aag gcc atg gaa cta gac tgc ctt tta ggg atc     2640
   Ala Glu Asp Tyr Asn Lys Ala Met Glu Leu Asp Cys Leu Leu Gly Ile
   865                 870                 875                 880
   tcc aat gag ttc att gtg ctc att gaa cag gaa aca aag agc gtg gtc     2688
   Ser Asn Glu Phe Ile Val Leu Ile Glu Gln Glu Thr Lys Ser Val Val
                   885                 890                 895
   ttc aat tgt tcc tgt aga gat gtg ata ggg tgg act tca act gac acc     2736
   Phe Asn Cys Ser Cys Arg Asp Val Ile Gly Trp Thr Ser Thr Asp Thr
               900                 905                 910
   agc ctc aaa atc ttc tat gaa cga gga gaa tgt gtt tca gtg ggt agt     2784
   Ser Leu Lys Ile Phe Tyr Glu Arg Gly Glu Cys Val Ser Val Gly Ser
           915                 920                 925
   ttt att aac att gag gag atc aaa gag att gtc aaa agg ttg cag ttt     2832
   Phe Ile Asn Ile Glu Glu Ile Lys Glu Ile Val Lys Arg Leu Gln Phe
       930                 935                 940
   gtt tca aaa ggc tgt gaa tcg gtg gag atg act ctg cga aga aat ggg     2880
   Val Ser Lys Gly Cys Glu Ser Val Glu Met Thr Leu Arg Arg Asn Gly
   945                 950                 955                 960
   cta gga cag ctt ggc ttc cat gtc aac tat gag ggc att gtg gcg gat     2928
   Leu Gly Gln Leu Gly Phe His Val Asn Tyr Glu Gly Ile Val Ala Asp
                   965                 970                 975
   gtg gag ccc tac ggt tat gcc tgg cag gca ggg ctg agg cag ggc agt     2976
   Val Glu Pro Tyr Gly Tyr Ala Trp Gln Ala Gly Leu Arg Gln Gly Ser
               980                 985                 990
   cgc ctg gtg gag atc tgc aag gtg gcg gta gcc act ctg agc cat gag     3024
   Arg Leu Val Glu Ile Cys Lys Val Ala Val Ala Thr Leu Ser His Glu
           995                 1000                1005
   cag atg atc gac ctc ctg aga aca tct gtc acg gtg aag gtt gtc atc     3072
   Gln Met Ile Asp Leu Leu Arg Thr Ser Val Thr Val Lys Val Val Ile
       1010                1015                1020
   att ccc ccg cat gat gac tgc acc ccg cgg agg agt tgc tct gaa acc     3120
   Ile Pro Pro His Asp Asp Cys Thr Pro Arg Arg Ser Cys Ser Glu Thr
   1025                1030                1035                1040
   tac cgc atg cca gtg atg gag tac aaa atg aat gaa ggt gtt tca tac     3168
   Tyr Arg Met Pro Val Met Glu Tyr Lys Met Asn Glu Gly Val Ser Tyr
                   1045                1050                1055
   gaa ttc aag ttt ccc ttc cga aat aat aac aag tgg cag agg aac gcc     3216
   Glu Phe Lys Phe Pro Phe Arg Asn Asn Asn Lys Trp Gln Arg Asn Ala
               1060                1065                1070
   agc aag ggg cct cat tca cct caa gtc ccg tcc cag gtg cag agt ccc     3264
   Ser Lys Gly Pro His Ser Pro Gln Val Pro Ser Gln Val Gln Ser Pro
           1075                1080                1085
   atg acc tcg cgg ctg aat gct gga aaa gga gat ggg aag atg cct cct     3312
   Met Thr Ser Arg Leu Asn Ala Gly Lys Gly Asp Gly Lys Met Pro Pro
       1090                1095                1100
   cca gaa aga gcc gcc aac atc cct cga agc atc tcc agt gac ggg cgc     3360
   Pro Glu Arg Ala Ala Asn Ile Pro Arg Ser Ile Ser Ser Asp Gly Arg
   1105                1110                1115                1120
   cca cta gag agg cgg ctg tct cct ggt tcg gac atc tat gtg acg gtc    3408
   Pro Leu Glu Arg Arg Leu Ser Pro Gly Ser Asp Ile Tyr Val Thr Val
                   1125                1130                1135
   tca tcc atg gct tta gca aga tcc cag tgt cgg aac tct cct agc aac    3456
   Ser Ser Met Ala Leu Ala Arg Ser Gln Cys Arg Asn Ser Pro Ser Asn
               1140                1145                1150
   ttg tct tca tcc agt gat act ggt tct gtg ggg ggc act tac agg cag    3504
   Leu Ser Ser Ser Ser Asp Thr Gly Ser Val Gly Gly Thr Tyr Arg Gln
           1155                1160                1165
   aag tcc atg ccc gaa ggg ttt gga gtg agc cgt aga tcc cca gcc tcc    3552
   Lys Ser Met Pro Glu Gly Phe Gly Val Ser Arg Arg Ser Pro Ala Ser
       1170                1175                1180
   att gac agg cag aac acc cag tca gat att ggt ggc agc gga aaa tcc    3600
   Ile Asp Arg Gln Asn Thr Gln Ser Asp Ile Gly Gly Ser Gly Lys Ser
   1185                1190                1195                1200
   acg cct agc tgg caa aga agt gag gat agc att gct gac cag atg gct    3648
   Thr Pro Ser Trp Gln Arg Ser Glu Asp Ser Ile Ala Asp Gln Met Ala
                   1205                1210                1215
   tac agt tat aga gga cct cag gat ttc aat tct ttt gtc ctc gag cag    3696
   Tyr Ser Tyr Arg Gly Pro Gln Asp Phe Asn Ser Phe Val Leu Glu Gln
               1220                1225                1230
   cat gaa tat aca gag cca aca tgc cat ctc cca gca gta tca aag gta    3744
   His Glu Tyr Thr Glu Pro Thr Cys His Leu Pro Ala Val Ser Lys Val
           1235                1240                1245
   ctg cca gct ttc cga gag agc ccc agt ggg aga tta atg cgg cag gat    3792
   Leu Pro Ala Phe Arg Glu Ser Pro Ser Gly Arg Leu Met Arg Gln Asp
       1250                1255                1260
   cca gtg gtt cat ttg tct cca aac aaa caa ggg cat tct gat agc cac    3840
   Pro Val Val His Leu Ser Pro Asn Lys Gln Gly His Ser Asp Ser His
   1265                1270                1275                1280
   tac tcg agc cac tcc agt agc aat act ctc tcc agc aat gcg tca agt    3888
   Tyr Ser Ser His Ser Ser Ser Asn Thr Leu Ser Ser Asn Ala Ser Ser
                   1285                1290                1295
   gcc cat agt gat gag aag tgg tac gat ggg gac cgc aca gaa tcc gaa    3936
   Ala His Ser Asp Glu Lys Trp Tyr Asp Gly Asp Arg Thr Glu Ser Glu
               1300                1305                1310
   ctc aac agc tat aac tat ctg caa ggc acc tct gct gac agt ggc att    3984
   Leu Asn Ser Tyr Asn Tyr Leu Gln Gly Thr Ser Ala Asp Ser Gly Ile
           1315                1320                1325
   gac acc acc tct tat ggc ccc agc cac ggc agc aca gcc tcg ctg ggg    4032
   Asp Thr Thr Ser Tyr Gly Pro Ser His Gly Ser Thr Ala Ser Leu Gly
       1330                1335                1340
   gct gcc aca tcg tca cct cgc tca ggg cca ggc aag gag aaa gtg gca    4080
   Ala Ala Thr Ser Ser Pro Arg Ser Gly Pro Gly Lys Glu Lys Val Ala
   1345                1350                1355                1360
   ccc cta tgg cac agc tcc agt gaa gta atc tcc atg gca gat cgg act     4128
   Pro Leu Trp His Ser Ser Ser Glu Val Ile Ser Met Ala Asp Arg Thr
                   1365                1370                1375
   ttg gag aca gag agc cac ggc ctg gac cgg aaa aca gag tct tcc ctg     4176
   Leu Glu Thr Glu Ser His Gly Leu Asp Arg Lys Thr Glu Ser Ser Leu
               1380                1385                1390
   agc tta gac ata cac agc aag agc caa gcc ggc tcg acc cct ctg aca     4224
   Ser Leu Asp Ile His Ser Lys Ser Gln Ala Gly Ser Thr Pro Leu Thr
           1395                1400                1405
   agg gag aac agc acc ttc agt ata aac gat gct gct tcc cac aca agt     4272
   Arg Glu Asn Ser Thr Phe Ser Ile Asn Asp Ala Ala Ser His Thr Ser
       1410                1415                1420
   acc atg agc tcc cga cac tct gcc agc cca gtg gtt ttc acc agt gcc     4320
   Thr Met Ser Ser Arg His Ser Ala Ser Pro Val Val Phe Thr Ser Ala
   1425                1430                1435                1440
   cgg agt tca cct aaa gaa gag ctt cat cca gct gcc ccc tca cag ctc     4368
   Arg Ser Ser Pro Lys Glu Glu Leu His Pro Ala Ala Pro Ser Gln Leu
                   1445                1450                1455
   gca cca tcc ttc tcc tcc tct tcc tcc tcc tcc tct ggt cct agg agt     4416
   Ala Pro Ser Phe Ser Ser Ser Ser Ser Ser Ser Ser Gly Pro Arg Ser
               1460                1465                1470
   ttt tac cct cgc cag ggc gct act agc aag tac ctg att gga tgg aaa     4464
   Phe Tyr Pro Arg Gln Gly Ala Thr Ser Lys Tyr Leu Ile Gly Trp Lys
           1475                1480                1485
   aaa ccc gaa gga acc ata aac tcc gtg gga ttt atg gac acg aga aag     4512
   Lys Pro Glu Gly Thr Ile Asn Ser Val Gly Phe Met Asp Thr Arg Lys
       1490                1495                1500
   cgt cat cag agc gat ggc aat gaa ata gcc cac acc agg ctg cgt gcc     4560
   Arg His Gln Ser Asp Gly Asn Glu Ile Ala His Thr Arg Leu Arg Ala
   1505                1510                1515                1520
   tca acc aga gac ctc cgg gca tct cct aag cca acc tcc aag tcc acc     4608
   Ser Thr Arg Asp Leu Arg Ala Ser Pro Lys Pro Thr Ser Lys Ser Thr
                   1525                1530                1535
   att gaa gaa gat cta aag aaa cta att gat ctt gaa agc cca act cct     4656
   Ile Glu Glu Asp Leu Lys Lys Leu Ile Asp Leu Glu Ser Pro Thr Pro
               1540                1545                1550
   gaa tca cag aag agt ttt aag ttc cac gca ctc tcc tct cct cag tct     4704
   Glu Ser Gln Lys Ser Phe Lys Phe His Ala Leu Ser Ser Pro Gln Ser
           1555                1560                1565
   cct ttc ccc agc acc ccc acc tca cgg cgg gcc ttg cac aga aca ctg     4752
   Pro Phe Pro Ser Thr Pro Thr Ser Arg Arg Ala Leu His Arg Thr Leu
       1570                1575                1580
   tcg gac gag agc att tac aat agc cag agg gag cac ttt ttc acc tcc     4800
   Ser Asp Glu Ser Ile Tyr Asn Ser Gln Arg Glu His Phe Phe Thr Ser
   1585                1590                1595                1600
   agg gcg tca ctt ctg gac caa gcc ctg ccc aac gac gtc ctc ttc agt     4848
   Arg Ala Ser Leu Leu Asp Gln Ala Leu Pro Asn Asp Val Leu Phe Ser
                   1605                1610                1615
   agc acg tac cct tct ctc ccc aag tcg ctc ccg ttg agg agg cct tct     4896
   Ser Thr Tyr Pro Ser Leu Pro Lys Ser Leu Pro Leu Arg Arg Pro Ser
               1620                1625                1630
   tac acc tta gga atg aaa tcg ctg cat gga gag ttc tca gcc tcg gac     4944
   Tyr Thr Leu Gly Met Lys Ser Leu His Gly Glu Phe Ser Ala Ser Asp
           1635                1640                1645
   agc tcc ctc act gac atc cag gag acc cgc agg cag cct atg ccc gac     4992
   Ser Ser Leu Thr Asp Ile Gln Glu Thr Arg Arg Gln Pro Met Pro Asp
       1650                1655                1660
   cct ggc ctg atg ccc ctg cct gac act gct gca gac ttg gat tgg tcc     5040
   Pro Gly Leu Met Pro Leu Pro Asp Thr Ala Ala Asp Leu Asp Trp Ser
   1665                1670                1675                1680
   aac ctg gta gat gct gcc aaa gcc tat gag gtc cag aga gcc tca ttt     5088
   Asn Leu Val Asp Ala Ala Lys Ala Tyr Glu Val Gln Arg Ala Ser Phe
                   1685                1690                1695
   ttt gct gct agt gat gaa aac cat cgc ccc ttg agt gct gca tcc aac     5136
   Phe Ala Ala Ser Asp Glu Asn His Arg Pro Leu Ser Ala Ala Ser Asn
               1700                1705                1710
   agt gat cag ctg gag gac cag gct ctg gcc cag atg aag cct tac agc     5184
   Ser Asp Gln Leu Glu Asp Gln Ala Leu Ala Gln Met Lys Pro Tyr Ser
           1715                1720                1725
   agc agt aaa gac tcc tct ccc act ctg gct tct aaa gtg gac cag ctg     5232
   Ser Ser Lys Asp Ser Ser Pro Thr Leu Ala Ser Lys Val Asp Gln Leu
       1730                1735                1740
   gaa ggt atg ctg aag atg ctt cgg gaa gat ttg aag aag gaa aaa gaa     5280
   Glu Gly Met Leu Lys Met Leu Arg Glu Asp Leu Lys Lys Glu Lys Glu
   1745                1750                1755                1760
   gac aaa gct cac ctt cag gcg gag gtg cag cac ctg cga gag gac aac     5328
   Asp Lys Ala His Leu Gln Ala Glu Val Gln His Leu Arg Glu Asp Asn
                   1765                1770                1775
   ctg agg cta cag gag gag tcc cag aac gcc tcg gac aag ctg aag aag     5376
   Leu Arg Leu Gln Glu Glu Ser Gln Asn Ala Ser Asp Lys Leu Lys Lys
               1780                1785                1790
   ttc aca gaa tgg gtc ttc aac acc ata gac atg agc                     5412
   Phe Thr Glu Trp Val Phe Asn Thr Ile Asp Met Ser
           1795                1800
<210> 6
   <211> 5349
   <212> DNA
   <213> Homo sapiens
<220>
   <221> CDS
   <222> (1)..(5349)
<400> 6
   atg acc agc ttg aaa cgg tca cag aca gaa agg cct ctt gcc act gac    48
   Met Thr Ser Leu Lys Arg Ser Gln Thr Glu Arg Pro Leu Ala Thr Asp
    1               5                  10                  15
   agg gcc tct gtt gtt ggc aca gac ggc acc ccc aaa gtc cac act gat    96
   Arg Ala Ser Val Val Gly Thr Asp Gly Thr Pro Lys Val His Thr Asp
               20                  25                  30
   gat ttc tac atg cgg cgc ttc cgg tcc caa aat ggc agc tta gga tca    144
   Asp Phe Tyr Met Arg Arg Phe Arg Ser Gln Asn Gly Ser Leu Gly Ser
           35                  40                  45
   tca gtt atg gct cct gta gga ccc ccc cga agt gaa ggt tct cac cat    192
   Ser Val Met Ala Pro Val Gly Pro Pro Arg Ser Glu Gly Ser His His
       50                  55                  60
   ata acc tca acc ccc gga gtc cca aaa atg ggg gta agg gca agg att    240
   Ile Thr Ser Thr Pro Gly Val Pro Lys Met Gly Val Arg Ala Arg Ile
   65                  70                  75                  80
   gca gat tgg ccc cca aga aag gaa aac ata aaa gaa tct agc cgt tca    288
   Ala Asp Trp Pro Pro Arg Lys Glu Asn Ile Lys Glu Ser Ser Arg Ser
                   85                  90                  95
   agc cag gaa ata gaa acc tca agt tgc ctt gat agc ctg tcc tcc aaa    336
   Ser Gln Glu Ile Glu Thr Ser Ser Cys Leu Asp Ser Leu Ser Ser Lys
               100                 105                 110
   agc agt cct gtg agt cag gga agt tct gtt agc ctc aat tcc aat gac    384
   Ser Ser Pro Val Ser Gln Gly Ser Ser Val Ser Leu Asn Ser Asn Asp
           115                 120                 125
   tca gcc atg ctg aaa agc ata cag aac acg ctg aaa aac aag aca aga    432
   Ser Ala Met Leu Lys Ser Ile Gln Asn Thr Leu Lys Asn Lys Thr Arg
       130                 135                 140
   ccg tcg gag aac atg gac tcc aga ttt ctc atg cct gaa gcc tac ccc    480
   Pro Ser Glu Asn Met Asp Ser Arg Phe Leu Met Pro Glu Ala Tyr Pro
   145                 150                 155                 160
   agc tcc ccc aga aaa gct ctt cgc aga ata cgc cag cga agc aac agt    528
   Ser Ser Pro Arg Lys Ala Leu Arg Arg Ile Arg Gln Arg Ser Asn Ser
                   165                 170                 175
   gat atc acc ata agt gaa ctt gat gtg gat agc ttt gat gaa tgt atc    576
   Asp Ile Thr Ile Ser Glu Leu Asp Val Asp Ser Phe Asp Glu Cys Ile
               180                 185                 190
   tca cct aca tac aag act gga cca tca ctg cac agg gaa tat ggt agc    624
   Ser Pro Thr Tyr Lys Thr Gly Pro Ser Leu His Arg Glu Tyr Gly Ser
           195                 200                 205
   aca tct tca att gat aaa cag gga aca tct gga gaa agc ttt ttt gat    672
   Thr Ser Ser Ile Asp Lys Gln Gly Thr Ser Gly Glu Ser Phe Phe Asp
       210                 215                 220
   ttg tta aag ggc tac aaa gat gac aaa tct gat cga ggt cca act cca    720
   Leu Leu Lys Gly Tyr Lys Asp Asp Lys Ser Asp Arg Gly Pro Thr Pro
   225                 230                 235                 240
   acc aag ctc agt gac ttt ctc att act ggt ggt ggc aag ggt tct ggt    768
   Thr Lys Leu Ser Asp Phe Leu Ile Thr Gly Gly Gly Lys Gly Ser Gly
                   245                 250                 255
   ttc tct ttg gat gta ata gac ggg cct atc tca cag aga gag aac ctc    816
   Phe Ser Leu Asp Val Ile Asp Gly Pro Ile Ser Gln Arg Glu Asn Leu
               260                 265                 270
   agg ctt ttt aag gaa agg gaa aaa cca ctc aag cga cgt tca aaa tct    864
   Arg Leu Phe Lys Glu Arg Glu Lys Pro Leu Lys Arg Arg Ser Lys Ser
           275                 280                 285
   gaa act gga gac tcc tct att ttt cgt aaa ttg cgc aat gcc aaa ggt    912
   Glu Thr Gly Asp Ser Ser Ile Phe Arg Lys Leu Arg Asn Ala Lys Gly
       290                 295                 300
   gaa gaa ctt ggg aag tca tca gat ctt gaa gat aac cga tca gaa gac    960
   Glu Glu Leu Gly Lys Ser Ser Asp Leu Glu Asp Asn Arg Ser Glu Asp
   305                 310                 315                 320
   tct gtc agg ccc tgg aca tgt cca aag tgc ttt gcc cac tat gat gtc    1008
   Ser Val Arg Pro Trp Thr Cys Pro Lys Cys Phe Ala His Tyr Asp Val
                   325                 330                 335
   cag agt ata tta ttt gat ttg aat gag gca att atg aac agg cac aat    1056
   Gln Ser Ile Leu Phe Asp Leu Asn Glu Ala Ile Met Asn Arg His Asn
               340                 345                 350
   gtt att aag agg aga aac acc acc act gga gct tcc gca gct gcc gtg    1104
   Val Ile Lys Arg Arg Asn Thr Thr Thr Gly Ala Ser Ala Ala Ala Val
           355                 360                 365
   gca tcc ttg gtc tct gga cct ctg tct cat tca gcc agt ttt agc tcc    1152
   Ala Ser Leu Val Ser Gly Pro Leu Ser His Ser Ala Ser Phe Ser Ser
       370                 375                 380
   cca atg ggc agc aca gag gac ctg aat tcc aaa gga agc ctc agc atg    1200
   Pro Met Gly Ser Thr Glu Asp Leu Asn Ser Lys Gly Ser Leu Ser Met
   385                 390                 395                 400
   gac cag gga gat gat aaa agc aat gag ctt gta atg agc tgt cca tat    1248
   Asp Gln Gly Asp Asp Lys Ser Asn Glu Leu Val Met Ser Cys Pro Tyr
                   405                 410                 415
   ttt cgg aat gag ata ggt gga gaa ggg gag agg aaa atc agc ctt tca    1296
   Phe Arg Asn Glu Ile Gly Gly Glu Gly Glu Arg Lys Ile Ser Leu Ser
               420                 425                 430
   aaa tca aat tct ggc tcc ttt agt gga tgt gaa agt gcc tcc ttt gag    1344
   Lys Ser Asn Ser Gly Ser Phe Ser Gly Cys Glu Ser Ala Ser Phe Glu
           435                 440                 445
   tct acc ctt agt tcc cat tgc aca aat gca gga gtg gca gta ctt gaa    1392
   Ser Thr Leu Ser Ser His Cys Thr Asn Ala Gly Val Ala Val Leu Glu
       450                 455                 460
   gtg ccc aag gag aac ttg gtg ttg cac cta gat aga gtg aaa aga tac    1440
   Val Pro Lys Glu Asn Leu Val Leu His Leu Asp Arg Val Lys Arg Tyr
   465                 470                 475                 480
   atc gtg gaa cac gta gat ctg ggt gca tac tat tat aga aaa ttt ttc    1488
   Ile Val Glu His Val Asp Leu Gly Ala Tyr Tyr Tyr Arg Lys Phe Phe
                   485                 490                 495
   tac cag aag gaa cac tgg aac tat ttt ggg gct gat gag aat ctt ggt    1536
   Tyr Gln Lys Glu His Trp Asn Tyr Phe Gly Ala Asp Glu Asn Leu Gly
               500                 505                 510
   cca gtg gct gtg agc att cga agg gaa aaa cca gat gaa atg aaa gaa    1584
   Pro Val Ala Val Ser Ile Arg Arg Glu Lys Pro Asp Glu Met Lys Glu
           515                 520                 525
   aat gga tct ccg tac aac tac cga ata att ttt aga act agt gag ctc    1632
   Asn Gly Ser Pro Tyr Asn Tyr Arg Ile Ile Phe Arg Thr Ser Glu Leu
       530                 535                 540
   atg aca ctg aga ggt tcg gtc ctg gag gac gcc att ccg tcg aca gcc    1680
   Met Thr Leu Arg Gly Ser Val Leu Glu Asp Ala Ile Pro Ser Thr Ala
   545                 550                 555                 560
   aag cac tcg aca gcc aga ggc ctg cct ctc aaa gaa gtg ctg gag cac    1728
   Lys His Ser Thr Ala Arg Gly Leu Pro Leu Lys Glu Val Leu Glu His
                   565                 570                 575
   gtg gtt cct gag ctc aat gtc cag tgc ctg cgg ttg gcc ttc aac aca    1776
   Val Val Pro Glu Leu Asn Val Gln Cys Leu Arg Leu Ala Phe Asn Thr
               580                 585                 590
   ccc aag gtc aca gag cag ctc atg aaa ctg gat gaa caa ggg ctg aac    1824
   Pro Lys Val Thr Glu Gln Leu Met Lys Leu Asp Glu Gln Gly Leu Asn
           595                 600                 605
   tac cag cag aaa gta ggc atc atg tac tgc aaa gct gga cag agc act    1872
   Tyr Gln Gln Lys Val Gly Ile Met Tyr Cys Lys Ala Gly Gln Ser Thr
       610                 615                 620
   gaa gaa gag atg tac aac aat gag tca gct ggc cca gcc ttt gaa gaa    1920
   Glu Glu Glu Met Tyr Asn Asn Glu Ser Ala Gly Pro Ala Phe Glu Glu
   625                 630                 635                 640
   ttc ctt caa cta ttg gga gag cga gtt cgg ctc aaa gga ttt gag aag    1968
   Phe Leu Gln Leu Leu Gly Glu Arg Val Arg Leu Lys Gly Phe Glu Lys
                   645                 650                 655
   tat cga gca cag ctt gat acc aaa act gac tcc act gga acc cat tct    2016
   Tyr Arg Ala Gln Leu Asp Thr Lys Thr Asp Ser Thr Gly Thr His Ser
               660                 665                 670
   ctg tac aca aca tac aaa gat tat gaa att atg ttc cat gtt tct acc    2064
   Leu Tyr Thr Thr Tyr Lys Asp Tyr Glu Ile Met Phe His Val Ser Thr
           675                 680                 685
   atg ctg cca tac aca ccc aac aac aaa caa cag ctc ctg agg aag cgg     2112
   Met Leu Pro Tyr Thr Pro Asn Asn Lys Gln Gln Leu Leu Arg Lys Arg
       690                 695                 700
   cac att gga aat gat atc gta aca att gtt ttc caa gag cct gga gca     2160
   His Ile Gly Asn Asp Ile Val Thr Ile Val Phe Gln Glu Pro Gly Ala
   705                 710                 715                 720
   cag cca ttc agc cca aaa aac atc cga tcc cac ttc cag cac gtt ttc     2208
   Gln Pro Phe Ser Pro Lys Asn Ile Arg Ser His Phe Gln His Val Phe
                   725                 730                 735
   gtc atc gtc agg gtg cac aat ccg tgc tct gac agt gtc tgt tat agt     2256
   Val Ile Val Arg Val His Asn Pro Cys Ser Asp Ser Val Cys Tyr Ser
               740                 745                 750
   gtg gct gtt acc agg tcc aga gat gtg cct tcc ttt ggg cct ccc att     2304
   Val Ala Val Thr Arg Ser Arg Asp Val Pro Ser Phe Gly Pro Pro Ile
           755                 760                 765
   cct aaa ggg gtc act ttc cct aag tca aat gtg ttc agg gac ttc ctt     2352
   Pro Lys Gly Val Thr Phe Pro Lys Ser Asn Val Phe Arg Asp Phe Leu
       770                 775                 780
   ttg gcg aaa gtg att aat gca gaa aat gct gct cat aaa tcg gag aag     2400
   Leu Ala Lys Val Ile Asn Ala Glu Asn Ala Ala His Lys Ser Glu Lys
   785                 790                 795                 800
   ttt cgg gcc atg gca act cgg acc cgc cag gaa tac ctg aaa gat ctg     2448
   Phe Arg Ala Met Ala Thr Arg Thr Arg Gln Glu Tyr Leu Lys Asp Leu
                   805                 810                 815
   gca gaa aag aat gtc acc aac acc cct atc gac cct tct ggc aag ttt     2496
   Ala Glu Lys Asn Val Thr Asn Thr Pro Ile Asp Pro Ser Gly Lys Phe
               820                 825                 830
   ccg ttc atc tct ctg gct tcc aag aag aag gaa aag tct aag cca tat     2544
   Pro Phe Ile Ser Leu Ala Ser Lys Lys Lys Glu Lys Ser Lys Pro Tyr
           835                 840                 845
   cca gga gcc gag ctc agc agc atg ggg gcc att gta tgg gca gtc cgg     2592
   Pro Gly Ala Glu Leu Ser Ser Met Gly Ala Ile Val Trp Ala Val Arg
       850                 855                 860
   gct gaa gac tac aac aag gcc atg gaa cta gac tgc ctt tta ggg atc     2640
   Ala Glu Asp Tyr Asn Lys Ala Met Glu Leu Asp Cys Leu Leu Gly Ile
   865                 870                 875                 880
   tcc aat gag ttc att gtg ctc att gaa cag gaa aca aag agc gtg gtc     2688
   Ser Asn Glu Phe Ile Val Leu Ile Glu Gln Glu Thr Lys Ser Val Val
                   885                 890                 895
   ttc aat tgt tcc tgt aga gat gtg ata ggg tgg act tca act gac acc     2736
   Phe Asn Cys Ser Cys Arg Asp Val Ile Gly Trp Thr Ser Thr Asp Thr
               900                 905                 910
   agc ctc aaa atc ttc tat gaa cga gga gaa tgt gtt tca gtg ggt agt     2784
   Ser Leu Lys Ile Phe Tyr Glu Arg Gly Glu Cys Val Ser Val Gly Ser
           915                 920                 925
   ttt att aac att gag gag atc aaa gag att gtc aaa agg ttg cag ttt    2832
   Phe Ile Asn Ile Glu Glu Ile Lys Glu Ile Val Lys Arg Leu Gln Phe
       930                 935                 940
   gtt tca aaa ggc tgt gaa tcg gtg gag atg act ctg cga aga aat ggg    2880
   Val Ser Lys Gly Cys Glu Ser Val Glu Met Thr Leu Arg Arg Asn Gly
   945                 950                 955                 960
   cta gga cag ctt ggc ttc cat gtc aac tat gag ggc att gtg gcg gat    2928
   Leu Gly Gln Leu Gly Phe His Val Asn Tyr Glu Gly Ile Val Ala Asp
                   965                 970                 975
   gtg gag ccc tac ggt tat gcc tgg cag gca ggg ctg agg cag ggc agt    2976
   Val Glu Pro Tyr Gly Tyr Ala Trp Gln Ala Gly Leu Arg Gln Gly Ser
               980                 985                 990
   cgc ctg gtg gag atc tgc aag gtg gcg gta gcc act ctg agc cat gag    3024
   Arg Leu Val Glu Ile Cys Lys Val Ala Val Ala Thr Leu Ser His Glu
           995                 1000                1005
   cag atg atc gac ctc ctg aga aca tct gtc acg gtg aag gtt gtc atc    3072
   Gln Met Ile Asp Leu Leu Arg Thr Ser Val Thr Val Lys Val Val Ile
       1010                1015                1020
   att ccc ccg cat gat gac tgc acc ccg cgg agg agt tgc tct gaa acc    3120
   Ile Pro Pro His Asp Asp Cys Thr Pro Arg Arg Ser Cys Ser Glu Thr
   1025                1030                1035                1040
   tac cgc atg cca gtg atg gag tac aaa atg aat gaa ggt gtt tca tac    3168
   Tyr Arg Met Pro Val Met Glu Tyr Lys Met Asn Glu Gly Val Ser Tyr
                   1045                1050                1055
   gaa ttc aag ttt ccc ttc cga aat aat aac aag tgg cag agg aac gcc    3216
   Glu Phe Lys Phe Pro Phe Arg Asn Asn Asn Lys Trp Gln Arg Asn Ala
               1060                1065                1070
   agc aag ggg cct cat tca cct caa gtc ccg tcc cag gtg cag agt ccc    3264
   Ser Lys Gly Pro His Ser Pro Gln Val Pro Ser Gln Val Gln Ser Pro
           1075                1080                1085
   atg acc tcg cgg ctg aat gct gga aaa gga gat ggg aag atg cct cct    3312
   Met Thr Ser Arg Leu Asn Ala Gly Lys Gly Asp Gly Lys Met Pro Pro
       1090                1095                1100
   cca gaa aga gcc gcc aac atc cct cga agc atc tcc agt gac ggg cgc    3360
   Pro Glu Arg Ala Ala Asn Ile Pro Arg Ser Ile Ser Ser Asp Gly Arg
   1105                1110                1115                1120
   cca cta gag agg cgg ctg tct cct ggt tcg gac atc tat gtg acg gtc    3408
   Pro Leu Glu Arg Arg Leu Ser Pro Gly Ser Asp Ile Tyr Val Thr Val
                   1125                1130                1135
   tca tcc atg gct tta gca aga tcc cag tgt cgg aac tct cct agc aac    3456
   Ser Ser Met Ala Leu Ala Arg Ser Gln Cys Arg Asn Ser Pro Ser Asn
               1140                1145                1150
   ttg tct tca tcc agt gat act ggt tct gtg ggg ggc act tac agg cag    3504
   Leu Ser Ser Ser Ser Asp Thr Gly Ser Val Gly Gly Thr Tyr Arg Gln
           1155                1160                1165
   aag tcc atg ccc gaa ggg ttt gga gtg agc cgt aga tcc cca gcc tcc    3552
   Lys Ser Met Pro Glu Gly Phe Gly Val Ser Arg Arg Ser Pro Ala Ser
       1170                1175                1180
   att gac agg cag aac acc cag tca gat att ggt ggc agc gga aaa tcc    3600
   Ile Asp Arg Gln Asn Thr Gln Ser Asp Ile Gly Gly Ser Gly Lys Ser
   1185                1190                1195                1200
   acg cct agc tgg caa aga agt gag gat agc att gct gac cag atg gag    3648
   Thr Pro Ser Trp Gln Arg Ser Glu Asp Ser Ile Ala Asp Gln Met Glu
                   1205                1210                1215
   cca aca tgc cat ctc cca gca gta tca aag gta ctg cca gct ttc cga    3696
   Pro Thr Cys His Leu Pro Ala Val Ser Lys Val Leu Pro Ala Phe Arg
               1220                1225                1230
   gag agc ccc agt ggg aga tta atg cgg cag gat cca gtg gtt cat ttg    3744
   Glu Ser Pro Ser Gly Arg Leu Met Arg Gln Asp Pro Val Val His Leu
           1235                1240                1245
   tct cca aac aaa caa ggg cat tct gat agc cac tac tcg agc cac tcc    3792
   Ser Pro Asn Lys Gln Gly His Ser Asp Ser His Tyr Ser Ser His Ser
       1250                1255                1260
   agt agc aat act ctc tcc agc aat gcg tca agt gcc cat agt gat gag    3840
   Ser Ser Asn Thr Leu Ser Ser Asn Ala Ser Ser Ala His Ser Asp Glu
   1265                1270                1275                1280
   aag tgg tac gat ggg gac cgc aca gaa tcc gaa ctc aac agc tat aac    3888
   Lys Trp Tyr Asp Gly Asp Arg Thr Glu Ser Glu Leu Asn Ser Tyr Asn
                   1285                1290                1295
   tat ctg caa ggc acc tct gct gac agt ggc att gac acc acc tct tat    3936
   Tyr Leu Gln Gly Thr Ser Ala Asp Ser Gly Ile Asp Thr Thr Ser Tyr
               1300                1305                1310
   ggc ccc agc cac ggc agc aca gcc tcg ctg ggg gct gcc aca tcg tca    3984
   Gly Pro Ser His Gly Ser Thr Ala Ser Leu Gly Ala Ala Thr Ser Ser
           1315                1320                1325
   cct cgc tca ggg cca ggc aag gag aaa gtg gca ccc cta tgg cac agc    4032
   Pro Arg Ser Gly Pro Gly Lys Glu Lys Val Ala Pro Leu Trp His Ser
       1330                1335                1340
   tcc agt gaa gta atc tcc atg gca gat cgg act ttg gag aca gag agc    4080
   Ser Ser Glu Val Ile Ser Met Ala Asp Arg Thr Leu Glu Thr Glu Ser
   1345                1350                1355                1360
   cac ggc ctg gac cgg aaa aca gag tct tcc ctg agc tta gac ata cac    4128
   His Gly Leu Asp Arg Lys Thr Glu Ser Ser Leu Ser Leu Asp Ile His
                   1365                1370                1375
   agc aag agc caa gcc ggc tcg acc cct ctg aca agg gag aac agc acc    4176
   Ser Lys Ser Gln Ala Gly Ser Thr Pro Leu Thr Arg Glu Asn Ser Thr
               1380                1385                1390
   ttc agt ata aac gat gct gct tcc cac aca agt acc atg agc tcc cga    4224
   Phe Ser Ile Asn Asp Ala Ala Ser His Thr Ser Thr Met Ser Ser Arg
           1395                1400                1405
   cac tct gcc agc cca gtg gtt ttc acc agt gcc cgg agt tca cct aaa    4272
   His Ser Ala Ser Pro Val Val Phe Thr Ser Ala Arg Ser Ser Pro Lys
       1410                1415                1420
   gaa gag ctt cat cca gct gcc ccc tca cag ctc gca cca tcc ttc tcc    4320
   Glu Glu Leu His Pro Ala Ala Pro Ser Gln Leu Ala Pro Ser Phe Ser
   1425                1430                1435                1440
   tcc tct tcc tcc tcc tcc tct ggt cct agg agt ttt tac cct cgc cag    4368
   Ser Ser Ser Ser Ser Ser Ser Gly Pro Arg Ser Phe Tyr Pro Arg Gln
                   1445                1450                1455
   ggc gct act agc aag tac ctg att gga tgg aaa aaa ccc gaa gga acc    4416
   Gly Ala Thr Ser Lys Tyr Leu Ile Gly Trp Lys Lys Pro Glu Gly Thr
               1460                1465                1470
   ata aac tcc gtg gga ttt atg gac acg aga aag cgt cat cag agc gat    4464
   Ile Asn Ser Val Gly Phe Met Asp Thr Arg Lys Arg His Gln Ser Asp
           1475                1480                1485
   ggc aat gaa ata gcc cac acc agg ctg cgt gcc tca acc aga gac ctc    4512
   Gly Asn Glu Ile Ala His Thr Arg Leu Arg Ala Ser Thr Arg Asp Leu
       1490                1495                1500
   cgg gca tct cct aag cca acc tcc aag tcc acc att gaa gaa gat cta    4560
   Arg Ala Ser Pro Lys Pro Thr Ser Lys Ser Thr Ile Glu Glu Asp Leu
   1505                1510                1515                1520
   aag aaa cta att gat ctt gaa agc cca act cct gaa tca cag aag agt    4608
   Lys Lys Leu Ile Asp Leu Glu Ser Pro Thr Pro Glu Ser Gln Lys Ser
                   1525                1530                1535
   ttt aag ttc cac gca ctc tcc tct cct cag tct cct ttc ccc agc acc    4656
   Phe Lys Phe His Ala Leu Ser Ser Pro Gln Ser Pro Phe Pro Ser Thr
               1540                1545                1550
   ccc acc tca cgg cgg gcc ttg cac aga aca ctg tcg gac gag agc att    4704
   Pro Thr Ser Arg Arg Ala Leu His Arg Thr Leu Ser Asp Glu Ser Ile
           1555                1560                1565
   tac aat agc cag agg gag cac ttt ttc acc tcc agg gcg tca ctt ctg    4752
   Tyr Asn Ser Gln Arg Glu His Phe Phe Thr Ser Arg Ala Ser Leu Leu
       1570                1575                1580
   gac caa gcc ctg ccc aac gac gtc ctc ttc agt agc acg tac cct tct    4800
   Asp Gln Ala Leu Pro Asn Asp Val Leu Phe Ser Ser Thr Tyr Pro Ser
   1585                1590                1595                1600
   ctc ccc aag tcg ctc ccg ttg agg agg cct tct tac acc tta gga atg    4848
   Leu Pro Lys Ser Leu Pro Leu Arg Arg Pro Ser Tyr Thr Leu Gly Met
                   1605                1610                1615
   aaa tcg ctg cat gga gag ttc tca gcc tcg gac agc tcc ctc act gac    4896
   Lys Ser Leu His Gly Glu Phe Ser Ala Ser Asp Ser Ser Leu Thr Asp
               1620                1625                1630
   atc cag gag acc cgc agg cag cct atg ccc gac cct ggc ctg atg ccc    4944
   Ile Gln Glu Thr Arg Arg Gln Pro Met Pro Asp Pro Gly Leu Met Pro
           1635                1640                1645
   ctg cct gac act gct gca gac ttg gat tgg tcc aac ctg gta gat gct    4992
   Leu Pro Asp Thr Ala Ala Asp Leu Asp Trp Ser Asn Leu Val Asp Ala
       1650                1655                1660
   gcc aaa gcc tat gag gtc cag aga gcc tca ttt ttt gct gct agt gat    5040
   Ala Lys Ala Tyr Glu Val Gln Arg Ala Ser Phe Phe Ala Ala Ser Asp
   1665                1670                1675                1680
   gaa aac cat cgc ccc ttg agt gct gca tcc aac agt gat cag ctg gag    5088
   Glu Asn His Arg Pro Leu Ser Ala Ala Ser Asn Ser Asp Gln Leu Glu
                   1685                1690                1695
   gac cag gct ctg gcc cag atg aag cct tac agc agc agt aaa gac tcc    5136
   Asp Gln Ala Leu Ala Gln Met Lys Pro Tyr Ser Ser Ser Lys Asp Ser
               1700                1705                1710
   tct ccc act ctg gct tct aaa gtg gac cag ctg gaa ggt atg ctg aag    5184
   Ser Pro Thr Leu Ala Ser Lys Val Asp Gln Leu Glu Gly Met Leu Lys
           1715                1720                1725
   atg ctt cgg gaa gat ttg aag aag gaa aaa gaa gac aaa gct cac ctt    5232
   Met Leu Arg Glu Asp Leu Lys Lys Glu Lys Glu Asp Lys Ala His Leu
       1730                1735                1740
   cag gcg gag gtg cag cac ctg cga gag gac aac ctg agg cta cag gag    5280
   Gln Ala Glu Val Gln His Leu Arg Glu Asp Asn Leu Arg Leu Gln Glu
   1745                1750                1755                1760
   gag tcc cag aac gcc tcg gac aag ctg aag aag ttc aca gaa tgg gtc    5328
   Glu Ser Gln Asn Ala Ser Asp Lys Leu Lys Lys Phe Thr Glu Trp Val
                   1765                1770                1775
   ttc aac acc ata gac atg agc                                        5349
   Phe Asn Thr Ile Asp Met Ser
               1780
<210> 7
   <211> 14
   <212> DNA
   <213> Homo sapiens
<400> 7
   gacggaacag cttc 14
<210> 8
   <211> 13
   <212> DNA
   <213> Homo sapiens
<400> 8
   tgcgtctggt tct 13
32
4

## Claims

1. A pharmaceutical composition comprising a polynucleotide encoding a TGAP7 protein or a biologically active fragment thereof comprising a nucleic acid molecule selected from the group consisting of
(i) DNA sequences comprising a nucleotide sequence encoding the amino acid sequence depicted in SEQ ID NO: 2 or 4;
(ii) DNA sequences comprising the nucleotide sequence depicted in SEQ ID NO: 1 or 3;
(iii) DNA sequences comprising a nucleotide sequence encoding a fragment or derivative of the protein encoded by the DNA sequence of (i) or (ii);
(iv) DNA sequences the complementary strand of which hybridizes with and which is at least 70% identical to the polynucleotide as defined in any one of (i) to (iii); and
(v) DNA sequences the nucleotide sequence of which is degenerate to the nucleotide sequence of a DNA sequence of any one of (i) to (iv).

2. A pharmaceutical composition comprising a nucleic acid molecule of at least 15 nucleotides in length hybridizing specifically with a polynucleotide of claim 1 or with a complementary strand thereof under hybridization conditions of 50%formamide/6×SSC/0.1%SDS/100µg/ml ssDNA, in which temperatures for hybridization are above 37°C and temperatures for washing in 0.1×SSC/0.1%SDS are above 55°C.

3. A pharmaceutical composition comprising a vector comprising the polynucleotide as defined in claim 1 or the nucleic acid molecule of claim 2.

4. A pharmaceutical composition comprising a vector as defined in claim 3, wherein said polynucleotide or nucleic acid molecule is operably linked to regulatory sequences allowing for the transcription and optionally expression of said nucleic acid molecule.

5. A pharmaceutical composition comprising a host cell containing a polynucleotide as defined in claim 1 or the nucleic acid molecule of claim 2 or a vector as defined in claim 3 or 4.

6. A pharmaceutical composition comprising a TGAP7 protein or (a) biologically active fragment(s) thereof encoded by the polynucleotide as defined in claim 1.

7. A pharmaceutical composition comprising an antibody specifically recognizing the protein as defined in claim 6.

8. A pharmaceutical composition comprising an antisense construct capable of inhibiting the expression of a polynucleotide as defined in claim 1.

9. The pharmaceutical composition of any one of claims 1 to 8 for use in cell, tissue or organ transplantation, for the treatment of autoimmune, allergic or infectious diseases, or for the treatment of tumors.

10. A diagnostic composition comprising a polynucleotide as defined in claim 1 or the nucleic acid molecule of claim 2, a vector as defined in claim 3 or 4, a cell as defined in claim 5, a protein as defined in claim 6, an antibody as defined in claim 7 or an antisense construct as defined in claim 8; and, optionally, suitable means for detection.

11. An *in vitro* method of diagnosing a pathological condition or a susceptibility to a pathological condition in a subject related to a disorder in the immune system comprising:
(a) determining the presence or absence of a mutation in the polynucleotide as defined in claim 1; and
(b) diagnosing a pathological condition or a susceptibility to a pathological condition based on the presence or absence of said mutation.

12. An *in vitro* method of diagnosing a pathological condition or a susceptibility to a pathological condition in a subject related to a disorder in the immune system comprising:
(a) determining the presence or amount of expression of the protein as defined in claim 6 in a biological sample; and
(b) diagnosing a pathological condition or a susceptibility to a pathological condition based on the presence or amount of expression of the protein.

13. An in vitro method for identifying a binding partner to a TGAP7 protein comprising:
(a) contacting a protein as defined in claim 6 with a compound to be screened; and
(b) determining whether the compound effects an activity of the protein.

14. A method for identifying leukocyte activating or co-stimulating compounds or for identifying inhibitors of leukocyte activation and stimulation comprising
(a) culturing leukocytes, lymphocytes or monocytes in the presence of the protein as defined in claim 6, the antibody as defined in claim 7, the cell as defined in claim 5 and/or the antisense construct as defined in claim 8; and, optionally, in the presence of a component capable of providing a detectable signal in response to leukocyte proliferation, with a compound to be screened under conditions permitting interaction of the compound with the (poly)peptide, antibody or cell(s); and
(b) detecting the presence or absence of a signal generated from the interaction of the compound with the cells.

15. A method for determining the status of an immune response comprising analyzing the expression of the polynucleotide as defined in claim 1 or the protein as defined in claim 6, wherein an increased level of expression is indicative for leukocyte activation.

16. Use of the polynucleotide as defined in claim 1 or the nucleic acid molecule of claim 2, the vector as defined in claim 3 or 4, the protein as defined in claim 6, the cell as defined in claim 5, the antisense construct of claim 8, the antibody as defined in claim 7, or a compound capable of suppressing TGAP7 activity for the preparation of a composition for diagnosing or the treatment of acute and chronic diseases, involving T cell activation and Th1 and Th2 immune response, for the treatment of acute and chronic rejection of allo-and xeno organ transplants and bone marrow transplantation, for the treatment of rheumatoid arthritis, lupus erythematodes, multiple sklerosis, encephalitis, vasculitis, diabetes mellitus, pancreatitis, gastritis, thyroiditis, for the treatment of maligne disorders of T, B or NK cells, for the treatment of asthma, lepromatosis, *Helicobacter pylori* associated gastritis or for the treatment of skin tumors, adrenal tumors or lung tumors, wound healing, growth disorders, inflammatory and/or infectious diseases.

17. Use of a polynucleotide as defined in claim 1 or the antibody as defined in claim 7 for the preparation of a diagnostic composition for the detection of leukocyte activation.

18. Use of claim 17, wherein said leukocyte is a B cell, T cell, NK cell and/or monocyte.

## Patentansprüche

1. Pharmazeutische Zusammensetzung, umfassend ein Polynukleotid, das ein TGAP7-Protein oder ein biologisch aktives Fragment davon codiert, umfassend ein Nukleinsäuremolekül, ausgewählt aus der Gruppe, bestehend aus:
(i) DNA-Sequenzen, umfassend eine Nukleotidsequenz, die die in SEQ ID NR: 2 oder 4 gezeigte Aminosäuresequenz codiert.
(ii) DNA-Sequenzen, umfassend die in SEQ ID NR: 1 oder 3 gezeigte Nukleotidsequenz;
(iii) DNA-Sequenzen, umfassend eine Nukleotidsequenz, die ein Fragment oder Derivat des Proteins codiert, das von der DNA-Sequenz von (i) oder (ii) codiert wird;
(iv) DNA-Sequenzen, deren komplementärer Strang mit dem Polynukleotid nach einem der Punkte (i) bis (iii) hybridisiert und der zu diesem zu mindestens 70% identisch ist; und
(v) DNA-Sequenzen, deren Nukleotidsequenz zur Nukleotidsequenz einer DNA-Sequenz nach einem der Punkte (i) bis (iv) identisch ist,

2. Pharmazeutische Zusammensetzung, umfassend ein Nukleinsäuremolekül mit mindestens 15 Nukleotiden Länge, das spezifisch mit einem Polynukleotid nach Anspruch 1 oder einem komplementären Strang davon unter Hybridisierungsbedingungen von 50% Formamid/6x SSC/0,1% SDS/100 µg/ml ssDNA hybridisiert, worin die Hybridisierungstemperaturen über 37°C sind und die Waschtemperaturen in 0,1x SSC/0,1 % SDS über 55°C sind.

3. Pharmazeutische Zusammensetzung, umfassend einen Vektor, der das Polynukleotid nach Anspruch 1 oder das Nukleinsäuremolekül nach Anspruch 2 umfasst.

4. Pharmazeutische Zusammensetzung, umfassend einen Vektor nach Anspruch 3, wobei das Polynukleotid oder das Nukleinsäuremolekül funktionsfähig an regulatorische Sequenzen gebunden ist, die die Transkription und gegebenenfalls die Expression des Nukleinsäuremoleküls ermöglichen.

5. Pharmazeutische Zusammensetzung, umfassend eine Wirtszelle, die ein Polynukleotid nach Anspruch 1 oder das Nukleinsäuremolekül nach Anspruch 2 oder einen Vektor nach Anspruch 3 oder 4 enthält.

6. Pharmazeutische Zusammensetzung, umfassend ein TGAP7-Protein oder ein oder mehrere biologisch aktive Fragment(e) davon, das bzw. die vom Polynukleotid nach Anspruch 1 codiert wird bzw. werden.

7. Pharmazeutische Zusammensetzung, umfassend einen Antikörper, der das Protein nach Anspruch 6 spezifisch erkennt.

8. Pharmazeutische Zusammensetzung; umfassend ein Antisense-Konstrukt, das die Expression eines Polynukleotids nach Anspruch 1 hemmen kann.

9. Pharmazeutische Zusammensetzung nach einem der Ansprüche 1 bis 8 zur Verwendung bei Zell-, Gewebe- oder Organtransplantation, zur Behandlung von Autoimmun-, Allergie- oder Infektions-Krankheiten oder zur Behandlung von Tumoren

10. Diagnose-Zusammensetzung, umfassend ein Polynukleotid nach Anspruch 1 oder das Nukleinsäuremolekül nach Anspruch 2, einen Vektor nach Anspruch 3 oder 4, eine Zelle nach Anspruch 5, ein Protein nach Anspruch 6, einen Antikörper nach Anspruch 7, oder ein Antisense-Konstrukt nach Anspruch 8; und gegebenenfalls geeignete Nachweismittel.

11. In-vitro-Verfahren zum Diagnostizieren eines pathologischen Zustands oder einer Anfälligkeit gegenüber einem pathologischen Zustand in einem Individuum, in Bezug auf eine Störung des Immunsystems, umfassend:
(a) Bestimmen der Anwesenheit oder Abwesenheit einer Mutation in dem Polynukleotid nach Anspruch 1; und
(b) Diagnostizieren eines pathologischen Zustands oder einer Anfälligkeit gegenüber einem pathologischen Zustand auf der Basis der Anwesenheit oder Abwesenheit der Mutation.

12. In-vitro-Verfahren zum Diagnostizieren eines pathologischen Zustands oder einer Anfälligkeit gegenüber einem pathologischen Zustand in einem Individuum, in Bezug auf eine Störung des Immunsystems, umfassend:
(a) Bestimmen der Anwesenheit oder des Ausmaßes der Expression des Proteins nach Anspruch 6 in einer biologischen Probe; und
(b) Diagnostizieren eines pathologischen Zustands oder einer Anfälligkeit gegenüber einem pathologischen Zustand auf der Basis der Anwesenheit oder des Ausmaßes der Expression des Proteins.

13. In-vitro-Verfahren zum Identifizieren eines Bindungspartnei-s an ein TGAP7-Protein, umfassend:
(a) Zusammenbringen eines Proteins nach Anspruch 6 mit einer zu überprüfenden Verbindung, und
(b) Bestimmen, ob die Verbindung eine Aktivität des Proteins beeinflusst.

14. Verfahren zum Identifizieren von Leukozyten-aktivierenden oder - costimulierenden Verbindungen oder zum Identifizieren von Inhibitoren der Leukozyten-Aktivierung und -Stimulation, umfassend:
(a) Anzucht der Leukozyten, Lymphozyten oder Monozyten in der Anwesenheit des Proteins nach Anspruch 6, des Antikörpers nach Anspruch 7, der Zelle nach Anspruch 5 und/oder des Antisense-Konstrukts nach Anspruch 8; und gegebenenfalls in Anwesenheit einer Komponente, die ein nachweisbares Signal in Reaktion auf die Leukozyten-Proliferation liefern kann, mit einer Verbindung, die unter Bedingungen überprüft werden soll, die die Wechselwirkung der Verbindung mit dem (Poly)peptid, Antikörper oder mit der oder den Zelle(n) ermöglichen; und
(b) Nachweisen der Anwesenheit oder Abwesenheit eines Signals, das aus der Wechselwirkung der Verbindung mit den Zellen erzeugt wird.

15. Verfahren zur Bestimmung des Status einer Immunreaktion, umfassend das Analysieren der Expression des Polynukleotids nach Anspruch 1 oder des Proteins nach Anspruch 6, wobei ein erhöhtes Expressionsausmaß die Leukozytenaktivierung anzeigt.

16. Verwendung des Polynukleotids nach Anspruch 1 oder des Nukleinsäuremoleküls nach Anspruch 2, des Vektors nach Anspruch 3 oder 4, des Proteins nach Anspruch 6, der Zelle nach Anspruch 5, des Antisense-Konstrukts nach Anspruch 8, des Antikörpers nach Anspruch 7 oder einer Verbindung, die die TGAP7-Aktivität unterdrücken kann, zur Herstellung einer Zusammensetzung zum Diagnostizieren oder zur Behandlung akuter und chronischer Krankheiten, die die T-Zell-Aktivierung und die Th1- und Th2-Immunreaktion beinhalten, zur Behandlung einer akuten und chronischen Abstoßung von Allo- und Xenoorgan-Transplantaten und Knochenmarkstransplantaten, zur Behandlung von rheumatoider Arthritis, Lupus erythematodes, multipler Sklerose, Enzephalitis, Vaskulitis, Diabetes mellitus, Pankreatitis, Gastritis, Thyreoiditis, zur Behandlung maligner Störungen von T-, B- oder NK-Zellen, zur Behandlung von Asthma, Lepra, *Helicobacter pylori*assoziierter. Gastritis oder zur Behandlung von Hauttumoren, Nebennierentumoren oder Lungentumoren, Wundheilung, Wachstumsstörungen, von Entzündungs- und/oder Infektionskrankheiten.

17. Verwendung eines Polynukleotids nach Anspruch 1, oder des Antikörpers nach Anspruch 7 zur Herstellung einer Diagnose-Zusammensetzung zum Nachweisen der Leukozyten-Aktivierung.

18. Verwendung nach Anspruch 17, wobei die Leukozyte eine B-Zelle, T-Zelle, NK-Zelle und/oder ein Monozyt ist.

## Revendications

1. Composition pharmaceutique comprenant un polynucléotide codant pour une protéine TGAP7 ou un fragment biologiquement actif de celle-ci comprenant une molécule d'acide nucléique choisi dans le groupe constitué de
(i) séquences d'ADN comprenant une séquence nucléotidique codant pour la séquence d'acide aminé décrite dans la SEQ ID NO : 2 ou 4 ;
(ii) séquences d'ADN comprenant la séquence nucléotidique décrite dans la SEQ ID NO: 1 ou 3 ;
(iii) séquences d'ADN comprenant une séquence nucléotidique codant pour un fragment ou un dérivé de la protéine codée par la séquence d'ADN de (i) ou (ii) ;
(iv) séquences d'ADN dont le brin complémentaire s'hybride et qui est au moins identique à 70% au polynucléotide tel que défini dans l'un quelconque des séquences (i) à (iii) ; et
(v) séquences d'ADN dont la séquence nucléotidique est dégénérée en la séquence nucléotidique d'une séquence d'ADN d'un quelconque des séquences (i) à (iv).

2. Composition pharmaceutique comprenant une molécule d'acide nucléique d'au moins 15 nucléotides de longueur s'hybridant spécifiquement avec un polynucléotide selon la revendication 1 ou avec son brin complémentaire dans des conditions d'hybridation de 50% de formamide/6xSSC/0,1% de SDS/100 µg/ml d'ADN simple brin, dans lesquelles les températures d'hybridation sont supérieures à 37°C et les températures de lavage dans 0,1 xSSC/0,1 % de SDS sont supérieures à 55°C.

3. Composition pharmaceutique comprenant un vecteur contenant le polynucléotide tel que défini dans la revendication 1 ou la molécule d'acide nucléique selon la revendication 2.

4. Composition pharmaceutique comprenant un vecteur tel que défini dans la revendication 3, dans laquelle ledit polynucléotide ou ladite molécule d'acide nucléique est lié de manière fonctionnelle aux séquences de régulation permettant ou autorisant la transcription et éventuellement l'expression de ladite molécule d'acide nucléique.

5. Composition pharmaceutique comprenant une cellule hôte contenant un polynucléotide tel que défini dans la revendication 1 ou la molécule d'acide nucléique selon la revendication 2 ou un vecteur tel que défini dans la revendication 3 ou 4.

6. Composition pharmaceutique comprenant une protéine TGAP7 ou (a) son ou ses fragment(s) biologiquement actif(s) codés par le polynucléotide tel que défini dans la revendication 1.

7. Composition pharmaceutique comprenant un anticorps reconnaissant spécifiquement la protéine telle que définie dans la revendication 6.

8. Composition pharmaceutique comprenant une structure antisens capable d'inhiber l'expression d'un polynucléotide tel que défini dans la revendication 1.

9. Composition pharmaceutique selon l'une quelconque des revendications 1 à 8 pour l'utilisation dans une transplantation d'une cellule, d'un tissu ou d'organes, pour le traitement de maladies auto-immunes, allergiques ou infectieuses, ou pour le traitement de tumeurs.

10. Composition de diagnostic comprenant un polynucléotide tel que défini dans la revendication 1 ou la molécule d'acide nucléique de la revendication 2, un vecteur tel que défini dans la revendication 3 ou 4, une cellule telle que définie dans la revendication 5, une protéine telle que définie dans la revendication 6, un anticorps tel que défini dans la revendication 7 ou une structure antisens telle que définie dans la revendication 8 ; et, éventuellement, des moyens adéquats de détection.

11. Procédé *in vitro* de diagnostic d'un état pathologique ou d'une prédisposition à un état pathologique chez un sujet associé à un trouble du système immunitaire consistant à:
(a) déterminer la présence ou l'absence d'une mutation dans le polynucléotide tel que défini dans la revendication 1 ; et
(b) diagnostiquer un état pathologique ou une prédisposition à un état pathologique basé sur la présence ou l'absence de ladite mutation.

12. Procédé *in vitro* de diagnostic d'un état pathologique ou d'une prédisposition à un état pathologique chez un sujet associé à un trouble du système immunitaire consistant à :
(a) déterminer la présence ou la quantité d'expression d'une protéine telle que définie dans la revendication 6 dans un échantillon biologique ; et
(b) diagnostiquer un état pathologique ou une prédisposition à un état pathologique basé sur la présence ou la quantité d'expression de la protéine.

13. Procédé d'identification *in vitro* d'un partenaire de liaison à une protéine TGAP7 consistant à:
(a) mettre en contact une protéine telle que définie dans la revendication 6 avec un composé à cribler ; et
(b) déterminer si le composé produit une activité de la protéine.

14. Procédé d'identification des composés activant ou co-stimulant des leucocytes ou d'identification des inhibiteurs de l'activation et de la stimulation de leucocytes consistant à :
(a) mettre en culture des leucocytes, des lymphocytes ou des monocytes en présence de la protéine telle que définie dans la revendication 6, de l'anticorps tel que défini dans la revendication 7, de la cellule telle que définie dans la revendication 5 et/ou de la structure antisens telle que définie dans la revendication 8 ; et, éventuellement, en présence d'un composant capable de fournir un signal détectable en réponse à la prolifération de leucocytes, avec un composé à cribler dans des conditions permettant l'interaction du composé avec le (poly)peptide, l'anticorps ou la ou les cellule(s) ; et
(b) détecter la présence ou l'absence d'un signal généré par l'interaction du composé avec les cellules.

15. Procédé destiné à déterminer le statut d'une réponse immunitaire comprenant l'analyse de l'expression du polynucléotide telle que définie dans la revendication 1 ou de la protéine telle que définie dans la revendication 6, dans lequel un niveau accru de l'expression est révélateur de l'activation de leucocytes.

16. Utilisation du polynucléotide tel que défini dans la revendication 1, ou de la molécule d'acide nucléique de la revendication 2, du vecteur tel que défini dans la revendication 3 ou 4, de la protéine telle que définie dans la revendication 6, de la cellule telle que définie dans la revendication 5, de la structure antisens de la revendication 8, de l'anticorps tel que défini dans la revendication 7, ou d'un composé capable de supprimer l'activité de TGAP7 pour la préparation d'une composition de diagnostic ou de traitement des maladies aiguës ou chroniques, impliquant l'activation de cellules T et la réponse immunitaire Th1 et Th2, pour le traitement du rejet aigu ou chronique d' allo et de xéno-transplantation d'organes et de transplantation de moelle osseuse, pour le traitement de l'arthrite rhumatoïde, du lupus d'érythémateux, de la sclérose multiple, d'encéphalite, de la vascularite, du diabète sucré, de la pancréatite, de la gastrite, de la thyroïdite, pour le traitement de troubles malins des cellules T, B ou NK, pour le traitement de l'asthme, de la lépromatose, d'une gastrite associée. à l' *Helicobacter pylori* ou pour le traitement de tumeurs de la peau, de tumeurs surrénaliennes ou de tumeurs du poumon, de la cicatrisation, des troubles de la croissance, des maladies inflammatoires et/ou infectieuses.

17. Utilisation d'un polynucléotide tel que défini dans la revendication 1 ou de l'anticorps tel que défini dans la revendication 7 pour la préparation d'une composition de diagnostic pour la détection de l'activation de leucocytes.

18. Utilisation selon la revendication 17, dans laquelle ledit leucocyte est une cellule B, une cellule T, une cellule NK et/ou un monocyte.
